# EUROPEAN PATENT SPECIFICATION

(11) **EP 3 390 442 B1**
(45) Date of publication and mention of the grant of the patent: **08.11.2023**
(21) Application number: 16875755.7
(22) Date of filing: 16.12.2016
(51) Int. Cl.: C07K 16/18, C12N 1/15, C12N 1/19, C12N 1/21, C12N 5/10, C12N 15/09, C12P 21/08, A61P 7/00

(54) **ANTI-C5 ANTIBODIES AND METHODS OF USE**
ANTI-C5-ANTIKÖRPER UND VERFAHREN ZUR VERWENDUNG
ANTICORPS ANTI-C5 ET LEURS PROCÉDÉS D'UTILISATION

(30) Priority: 18.12.2015 JP 2015247069
(43) Date of publication of application: 24.10.2018
(73) Proprietor: Chugai Seiyaku Kabushiki Kaisha, Kita-ku Tokyo 115-8543 (JP)
(72) Inventor: SAMPEI, Zenjiro, Synapse 138623 (SG)
(74) Representative: Vossius & Partner Patentanwälte Rechtsanwälte mbB
(86) International application number: PCT/JP2016/087481
(87) International publication number: WO 2017/104779

(56) References cited:
- WO-A1-2013/138680
- WO-A1-2014/028354
- WO-A1-2014/119969
- WO-A1-2016/098356
- WO-A1-2016/098356
- WO-A2-2008/113834
- WO-A2-2008/113834
- WO-A2-2011/111007
- WO-A2-2011/111007
- WO-A2-2013/152001
- WO-A2-2015/127134
- US-A1- 2011 111 406
- US-A1- 2015 239 966
- US-B1- 9 079 949
- IGAWA T ET AL: "pH-dependent antigen-binding antibodies as a novel therapeutic modality", BIOCHIMICA ET BIOPHYSICA ACTA (BBA) - PROTEINS & PROTEOMICS, ELSEVIER, NETHERLANDS, vol. 1844, no. 11, 12 August 2014 (2014-08-12), pages 1943-1950, XP029050320, ISSN: 1570-9639, DOI: 10.1016/J.BBAPAP.2014.08.003
- MOLLNES T.E. ET AL.: 'Identification of a Human C5 beta-Chain Epitope Exposed in the Native Complement Component but Concealed in the SC5b-9 Complex, Scand.' J.IMMUNOL vol. 28, 29 June 1988, pages 307 - 312, XP055206790

## Description

### [Technical Field]

The present invention relates to anti-C5 antibodies and methods of using the same. The invention is as defined in the claims.

### [Background Art]

The complement system plays a central role in the clearance of immune complexes and in immune responses to infectious agents, foreign antigens, virus-infected cells and tumor cells. There are about 25-30 complement proteins, which are found as a complex collection of plasma proteins and membrane cofactors. Complement components achieve their immune defensive functions by interacting in a series of intricate enzymatic cleavages and membrane binding events. The resulting complement cascades lead to the production of products with opsonic, immunoregulatory, and lytic functions.

Currently, it is widely accepted that the complement system can be activated through three distinct pathways: the classical pathway, the lectin pathway, and the alternative pathway. These pathways share many components, and while they differ in their initial steps, they converge and share the same terminal complement components (C5 through C9) responsible for the activation and destruction of target cells.

The classical pathway is normally activated by the formation of antigen-antibody complexes. Independently, the first step in activation of the lectin pathway is the binding of specific lectins such as mannan-binding lectin (MBL), H-ficolin, M-ficolin, L-ficolin and C-type lectin CL-11. In contrast, the alternative pathway spontaneously undergoes a low level of turnover activation, which can be readily amplified on foreign or other abnormal surfaces (bacteria, yeast, virally infected cells, or damaged tissue). These pathways converge at a point where complement component C3 is cleaved by an active protease to yield C3a and C3b.

C3a is an anaphylatoxin. C3b binds to bacterial and other cells, as well as to certain viruses and immune complexes, and tags them for removal from the circulation (the role known as opsonin). C3b also forms a complex with other components to form C5 convertase, which cleaves C5 into C5a and C5b.

C5 is a 190 kDa protein found in normal serum at approximately 80 micro g/ml (0.4 micro M). C5 is glycosylated with about 1.5-3% of its mass attributed to carbohydrate. Mature C5 is a heterodimer of 115 kDa alpha chain that is disulfide linked to 75 kDa beta chain. C5 is synthesized as a single chain precursor protein (pro-C5 precursor) of 1676 amino acids (see, e.g., PTL 1 and PTL 2). The pro-C5 precursor is cleaved to yield the beta chain as an amino terminal fragment and the alpha chain as a carboxyl terminal fragment. The alpha chain and the beta chain polypeptide fragments are connected to each other via a disulfide bond and constitute the mature C5 protein.

Mature C5 is cleaved into the C5a and C5b fragments during activation of the complement pathways. C5a is cleaved from the alpha chain of C5 by C5 convertase as an amino terminal fragment comprising the first 74 amino acids of the alpha chain. The remaining portion of mature C5 is fragment C5b, which contains the rest of the alpha chain disulfide bonded to the beta chain. Approximately 20% of the 11 kDa mass of C5a is attributed to carbohydrate.

C5a is another anaphylatoxin. C5b combines with C6, C7, C8 and C9 to form the membrane attack complex (MAC, C5b-9, terminal complement complex (TCC)) at the surface of the target cell. When sufficient numbers of MACs are inserted into target cell membranes, MAC pores are formed to mediate rapid osmotic lysis of the target cells.

As mentioned above, C3a and C5a are anaphylatoxins. They can trigger mast cell degranulation, which releases histamine and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract granulocytes such as neutrophils, eosinophils, basophils and monocytes to the site of complement activation.

The activity of C5a is regulated by the plasma enzyme carboxypeptidase N that removes the carboxy-terminal arginine from C5a forming C5a-des-Arg derivative. C5a-des-Arg exhibits only 1% of the anaphylactic activity and polymorphonuclear chemotactic activity of unmodified C5a.

While a properly functioning complement system provides a robust defense against infecting microbes, inappropriate regulation or activation of complement has been implicated in the pathogenesis of a variety of disorders including, e.g., rheumatoid arthritis (RA); lupus nephritis; ischemia-reperfusion injury; paroxysmal nocturnal hemoglobinuria (PNH); atypical hemolytic uremic syndrome (aHUS); dense deposit disease (DDD); macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass and hemodialysis (see, e.g., NPL 1). Therefore, inhibition of excessive or uncontrolled activations of the complement cascade can provide clinical benefits to patients with such disorders.

Paroxysmal nocturnal hemoglobinuria (PNH) is an uncommon blood disorder, wherein red blood cells are compromised and are thus destroyed more rapidly than normal red blood cells. PNH results from the clonal expansion of hematopoietic stem cells with somatic mutations in the PIG-A (phosphatidylinositol glycan class A) gene which is located on the X chromosome. Mutations in PIG-A lead to an early block in the synthesis of glycosylphosphatidylinositol (GPI), a molecule which is required for the anchor of many proteins to cell surfaces. Consequently, PNH blood cells are deficient in GPI-anchored proteins, which include complement-regulatory proteins CD55 and CD59. Under normal circumstances, these complement-regulatory proteins block the formation of MAC on cell surfaces, thereby preventing erythrocyte lysis. The absence of the GPI-anchored proteins causes complement-mediated hemolysis in PNH.

PNH is characterized by hemolytic anemia (a decreased number of red blood cells), hemoglobinuria (the presence of hemoglobin in urine, particularly evident after sleeping), and hemoglobinemia (the presence of hemoglobin in the bloodstream). PNH-afflicted individuals are known to have paroxysms, which are defined here as incidences of dark-colored urine. Hemolytic anemia is due to intravascular destruction of red blood cells by complement components. Other known symptoms include dysphasia, fatigue, erectile dysfunction, thrombosis and recurrent abdominal pain.

Eculizumab is a humanized monoclonal antibody directed against the complement protein C5, and the first therapy approved for the treatment of paroxysmal nocturnal hemoglobinuria (PNH) and atypical hemolytic uremic syndrome (aHUS) (see, e.g., NPL 2). Eculizumab inhibits the cleavage of C5 into C5a and C5b by C5 convertase, which prevents the generation of the terminal complement complex C5b-9. Both C5a and C5b-9 cause the terminal complement-mediated events that are characteristic of PNH and aHUS (see also, PTL 3, PTL 4, PTL 5, and PTL 6).

Several reports have described anti-C5 antibodies. For example, PTL 7 described an anti-C5 antibody which binds to the alpha chain of C5 but does not bind to C5a, and blocks the activation of C5, while PTL 8 described an anti-C5 monoclonal antibody which inhibits C5a formation. On the other hand, PTL 9 described an anti-C5 antibody which recognizes the proteolytic site for C5 convertase on the alpha chain of C5, and inhibits the conversion of C5 to C5a and C5b. PTL 10 described an anti-C5 antibody which has an affinity constant of at least 1 ×10⁷ M⁻¹.

Antibodies (IgGs) bind to neonatal Fc receptor (FcRn), and have long plasma retention times. The binding of IgGs to FcRn is typically observed under acidic conditions (e.g., pH 6.0), and it is rarely observed under neutral conditions (e.g., pH 7.4). Typically, IgGs are nonspecifically incorporated into cells via endocytosis, and return to the cell surfaces by binding to endosomal FcRn under the acidic conditions in the endosomes. Then, IgGs dissociate from FcRn under the neutral conditions in plasma. IgGs that have failed to bind to FcRn are degraded in lysosomes. When the FcRn binding ability of an IgG under acidic conditions is eliminated by introducing mutations into its Fc region, the IgG is not recycled from the endosomes into the plasma, leading to marked impairment of the plasma retention of the IgG. To improve the plasma retention of IgGs, a method that enhances their FcRn binding under acidic conditions has been reported. When the FcRn binding of an IgG under acidic conditions is improved by introducing an amino acid substitution into its Fc region, the IgG is more efficiently recycled from the endosomes to the plasma, and thereby shows improved plasma retention. Meanwhile, it has also been reported that an IgG with enhanced FcRn binding under neutral conditions does not dissociate from FcRn under the neutral conditions in plasma even when it returns to the cell surface via its binding to FcRn under the acidic conditions in the endosomes, and consequently its plasma retention remains unaltered, or rather, is worsened (see, e.g., NPL 3; NPL 4; NPL 5).

Recently, antibodies that bind to antigens in a pH-dependent manner have been reported (see, e.g., PTL 11 and PTL 12). These antibodies strongly bind to antigens under the plasma neutral conditions and dissociate from the antigens under the endosomal acidic conditions. After dissociating from the antigens, the antibodies become capable once again of binding to antigens when recycled to the plasma via FcRn. Thus, a single antibody molecule can repeatedly bind to multiple antigen molecules. In general, the plasma retention of an antigen is much shorter than that of an antibody that has the above-mentioned FcRn-mediated recycling mechanism. Therefore, when an antigen is bound to an antibody, the antigen normally shows prolonged plasma retention, resulting in an increase of the plasma concentration of the antigen. On the other hand, it has been reported that the above-described antibodies, which bind to antigens in a pH-dependent manner, eliminate antigens from plasma more rapidly than typical antibodies because they dissociate from the antigens within the endosomes during the FcRn-mediated recycling process. PTL 13 also described computer modeling analysis showing that an antibody with pH-dependent binding directed against C5 could extend antigen knockdown. Anti-C5 antibodies for inhibiting terminal complement and C5a anaphylatoxin-mediated inflammation and treating complement-associated disorders are described in PTL 14.

### [Citation List]

### [Patent Literature]

[PTL 1]
   U.S. Patent No. 6,355,245
[PTL 2]
   U.S. Patent No. 7,432,356
[PTL 3]
   WO 2005/074607
[PTL 4]
   WO 2007/106585
[PTL 5]
   WO 2008/069889
[PTL 6]
   WO 2010/054403
[PTL 7]
   WO 95/29697
[PTL 8]
   WO 02/30985
[PTL 9]
   WO 2004/007553
[PTL 10]
   WO 2010/015608
[PTL 11]
   WO 2009/125825
[PTL 12]
   WO 2011/122011
[PTL 13]
   WO 2011/111007
[PTL 14]
   U.S. Patent No. 9,079,949

### [Non-Patent Literature]

[NPL 1]
   Holers et al., Immunol. Rev. 223:300-316 (2008)
[NPL 2]
   Dmytrijuk et al., The Oncologist 13(9):993-1000 (2008)
[NPL 3]
   Yeung et al., J Immunol. 182(12): 7663-7671 (2009)
[NPL 4]
   Datta-Mannan et al., J Biol. Chem. 282(3): 1709-1717 (2007)
[NPL 5]
   Dall'Acqua et al., J. Immunol. 169(9):5171-5180 (2002)

### [Summary of Invention]

### [Technical Problem]

An objective of the invention is to provide anti-C5 antibodies and methods of using the same.

### [Solution to Problem]

The invention provides anti-C5 antibodies comprising a VH sequence of SEQ ID NO: 106 and a VL sequence of SEQ ID NO: 111 and methods of using the same. The invention is set out in the appended set of claims.

In some embodiments, an isolated anti-C5 antibody of the present disclosure binds to an epitope within the beta chain of C5. In some embodiments, an isolated anti-C5 antibody of the present disclosure binds to an epitope within the MG1-MG2 domain of the beta chain of C5. In some embodiments, an isolated anti-C5 antibody of the present disclosure binds to an epitope within a fragment consisting of amino acids 33-124 of the beta chain (SEQ ID NO: 40) of C5. In some embodiments, an isolated anti-C5 antibody of the present disclosure binds to an epitope within the beta chain (SEQ ID NO: 40) of C5 which comprises at least one fragment selected from the group consisting of amino acids 47-57, 70-76, and 107-110. In some embodiments, an isolated anti-C5 antibody of the present disclosure binds to an epitope within a fragment of the beta chain (SEQ ID NO: 40) of C5 which comprises at least one amino acid residue selected from the group consisting of Glu48, Asp51, His70, His72, Lys109, and His110 of SEQ ID NO: 40. In further embodiments, the antibody binds to C5 with a higher affinity at neutral pH than at acidic pH. In further embodiments, the antibody binds to C5 with a higher affinity at pH7.4 than at pH5.8. In another embodiment, an isolated anti-C5 antibody of the present disclosure binds to the same epitope as an antibody described in Table 2. In further embodiments, the antibody binds to the same epitope as an antibody described in Table 2 with a higher affinity at pH7.4 than at pH5.8. In a further embodiment, the anti-C5 antibody of the present disclosure binds to the same epitope as an antibody described in Tables 7 or 8. In further embodiments, the antibody binds to the same epitope as an antibody described in Tables 7 or 8 with a higher affinity at pH7.4 than at pH5.8.

In certain embodiments, an anti-C5 antibody of the present disclosure competes for binding C5 with an antibody comprising a VH and VL pair selected from: (a) a VH of SEQ ID NO: 1 and a VL of SEQ ID NO:11; (b) a VH of SEQ ID NO: 5 and a VL of SEQ ID NO:15; (c) a VH of SEQ ID NO:4 and a VL of SEQ ID NO:14; (d) a VH of SEQ ID NO: 6 and a VL of SEQ ID NO: 16; (e) a VH of SEQ ID NO:2 and a VL of SEQ ID NO: 12; (f) a VH of SEQ ID NO: 3 and a VL of SEQ ID NO: 13; (g) a VH of SEQ ID NO:9 and a VL of SEQ ID NO: 19; (h) a VH of SEQ ID NO:7 and a VL of SEQ ID NO: 17; (i) aVH of SEQ ID NO:8 and a VL of SEQ ID NO:18; and (j) a VH of SEQ ID NO:10 and a VL of SEQ ID NO:20. In further embodiments, the anti-C5 antibody binds to C5 with a higher affinity at neutral pH than at acidic pH. In further embodiments, the anti-C5 antibody binds to C5 with a higher affinity at pH7.4 than at pH5.8.

In some embodiments, an isolated anti-C5 antibody of the present disclosure has a characteristic selected from the group consisting of: (a) the antibody contacts amino acids D51 and K109 of C5 (SEQ ID NO:39); (b) the affinity of the antibody for C5 (SEQ ID NO:39) is greater than the affinity of the antibody for a C5 mutant consisting of an E48A substitution of SEQ ID NO:39; or (c) the antibody binds to a C5 protein consisting of the amino acid sequence of SEQ ID NO:39 at pH7.4, but does not bind to a C5 protein consisting of the amino acid sequence of SEQ ID NO:39 with a H72Y substitution at pH7.4. In further embodiments, the antibody binds to C5 with a higher affinity at neutral pH than at acidic pH. In further embodiments, the antibody binds to C5 with a higher affinity at pH7.4 than at pH5.8.

In some embodiments, an isolated anti-C5 antibody of the present disclosure inhibits activation of C5. In some embodiments, an isolated anti-C5 antibody of the present disclosure inhibits activation of C5 variant R885H. In some embodiments, an isolated anti-C5 antibody of the present disclosure is a monoclonal antibody. In some embodiments, an isolated anti-C5 antibody of the present disclosure is a human, humanized, or chimeric antibody. In some embodiments, an isolated anti-C5 antibody of the present disclosure is an antibody fragment that binds to C5. In some embodiments, an isolated anti-C5 antibody of the present disclosure is a full length IgG1 antibody.

In some embodiments, an isolated anti-C5 antibody of the present invention comprises a heavy chain variable domain framework FR1 comprising the amino acid sequence of any one of SEQ ID NO: 133; FR2 comprising the amino acid sequence of any one of SEQ ID NO: 135; FR3 comprising the amino acid sequence of any one of SEQ ID NO: 138; and FR4 comprising the amino acid sequence of any one of SEQ ID NO: 140. In some embodiments, an isolated anti-C5 antibody of the present invention comprises a light chain variable domain framework FR1 comprising the amino acid sequence of any one of SEQ ID NO: 143; FR2 comprising the amino acid sequence of any one of SEQ ID NO: 145; FR3 comprising the amino acid sequence of any one of SEQ ID NO: 147; and FR4 comprising the amino acid sequence of SEQ ID NO: 148.

An isolated anti-C5 antibody of the present invention comprises a VH sequence of SEQ ID NO: 106 and a VL sequence of SEQ ID NO: 111.

The invention provides an antibody comprising a VH sequence of SEQ ID NO: 106 and a VL sequence of SEQ ID NO: 111.

The invention also provides isolated nucleic acids encoding the anti-C5 antibody of the present invention. The invention also provides host cells comprising a nucleic acid of the present invention. The invention also provides a method of producing an antibody comprising culturing a host cell of the present invention so that the antibody is produced. The invention also provides an anti-C5 antibody as obtainable by the method of producing an antibody of the present invention.

The disclosure further provides a method of producing an anti-C5 antibody. In some embodiments, the method comprises immunizing an animal against a polypeptide which comprises the MG1-MG2 domain (SEQ ID NO: 43) of the beta chain of C5. In some embodiments, the method comprises immunizing an animal against a polypeptide which comprises the region corresponding to amino acids at positions 33 to 124 of the beta chain (SEQ ID NO: 40) of C5. In some embodiments, the method comprises immunizing an animal against a polypeptide which comprises at least one fragment selected from amino acids 47-57, 70-76, and 107-110 of the beta chain (SEQ ID NO: 40) of C5. In some embodiments, the method comprises immunizing an animal against a polypeptide which comprises a fragment of the beta chain (SEQ ID NO: 40) of C5, which comprises at least one amino acid selected from Glu48, Asp51, His70, His72, Lys109, and His110.

The invention also provides a pharmaceutical formulation comprising an anti-C5 antibody of the present invention and a pharmaceutically acceptable carrier.

Anti-C5 antibodies of the present invention may be for use as a medicament. Anti-C5 antibodies of the present invention may be for use in a method of treating a complement-mediated disease or condition which involves excessive or uncontrolled activation of C5. Anti-C5 antibodies of the present invention may be for use in a method of enhancing the clearance of C5 from plasma.

Anti-C5 antibodies of the present invention may be used in the manufacture of a medicament. In some embodiments, the medicament is for use in a method of treatment of a complement-mediated disease or condition which involves excessive or uncontrolled activation of C5. In some embodiments, the medicament is for use in a method of enhancing the clearance of C5 from plasma.

The invention also provides an anti-C5 antibodies for use in a method of treating an individual having a complement-mediated disease or condition which involves excessive or uncontrolled activation of C5. In some embodiments, the anti-C5 antibody of the present invention is to be administered to the individual in an effective amount. The disclosure also provides an anti-C5 antibodies for use in a method of enhancing the clearance of C5 from plasma in an individual. In some embodiments, the anti-C5 antibody of the present disclosure is to be administered to the individual in an effective amount to enhance the clearance of C5 from plasma.

### [Brief Description of Drawings]

[Fig. 1]

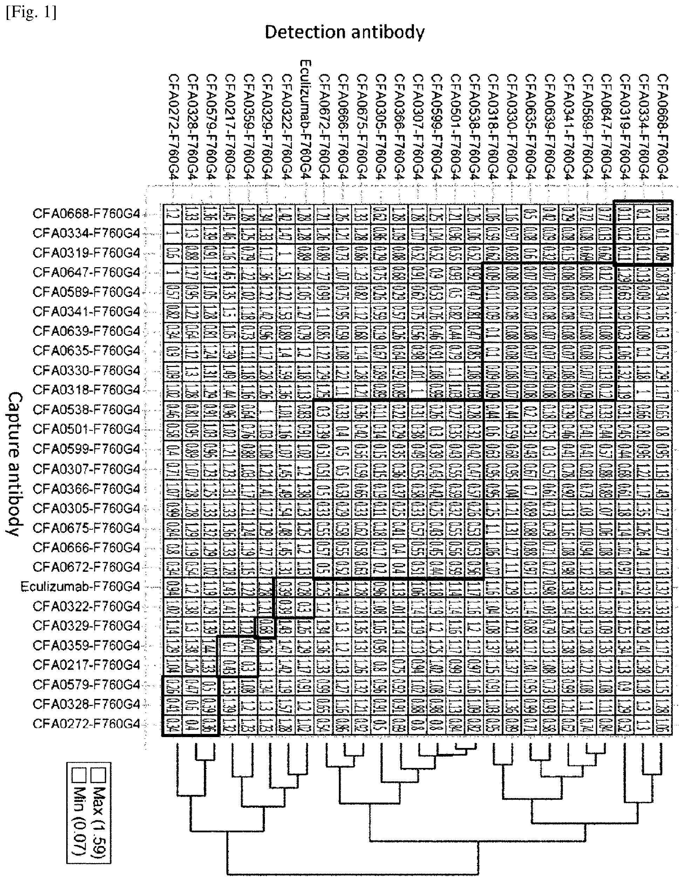

   Figure 1 illustrates epitope binning of anti-C5 antibodies, as described in Example 2.2. Antibodies grouped into the same epitope bin are boxed with a thick line.
[Fig. 2A]

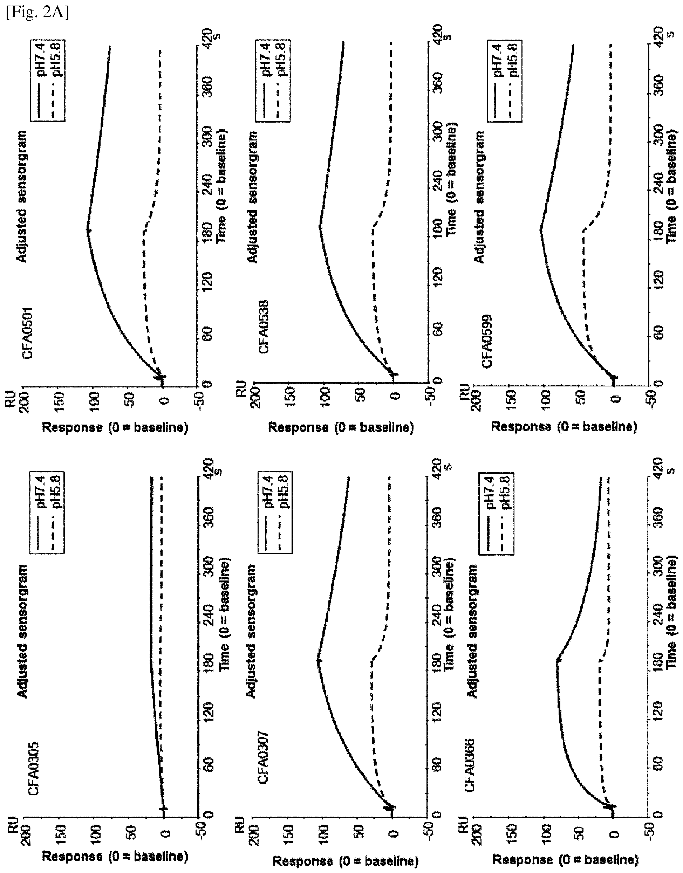

   Figure 2A illustrates BIACORE (registered trademark) sensorgrams of anti-C5 antibodies at pH7.4 (solid line) and pH5.8 (dashed line) to assess pH-dependency, as described in Example 3.2. CFA0305, CFA0307, CFA0366, CFA0501, CFA0538, and CFA0599 are antibodies grouped into epitope C, as described in Example 2.2.
[Fig. 2B]

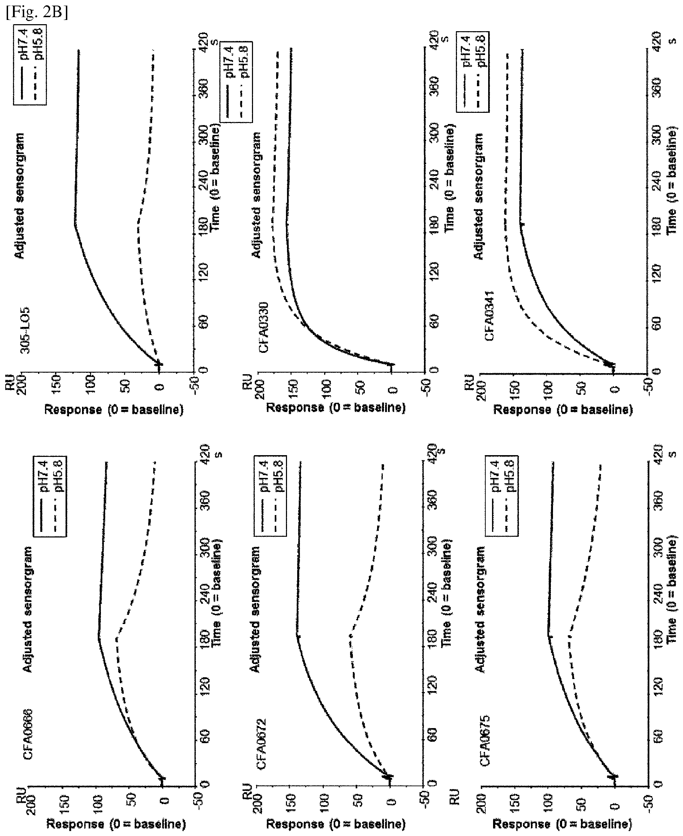

   Figure 2B illustrates BIACORE (registered trademark) sensorgrams of anti-C5 antibodies at pH7.4 (solid line) and pH5.8 (dashed line) to assess pH-dependency, as described in Example 3.2. CFA0666, CFA0672, and CFA0675 are antibodies grouped into epitope C, and CFA0330 and CFA0341 are antibodies grouped into epitope B, as described in Example 2.2. 305LO5 is a humanized antibody of CFA0305, as described in Example 2.3.
[Fig. 3]

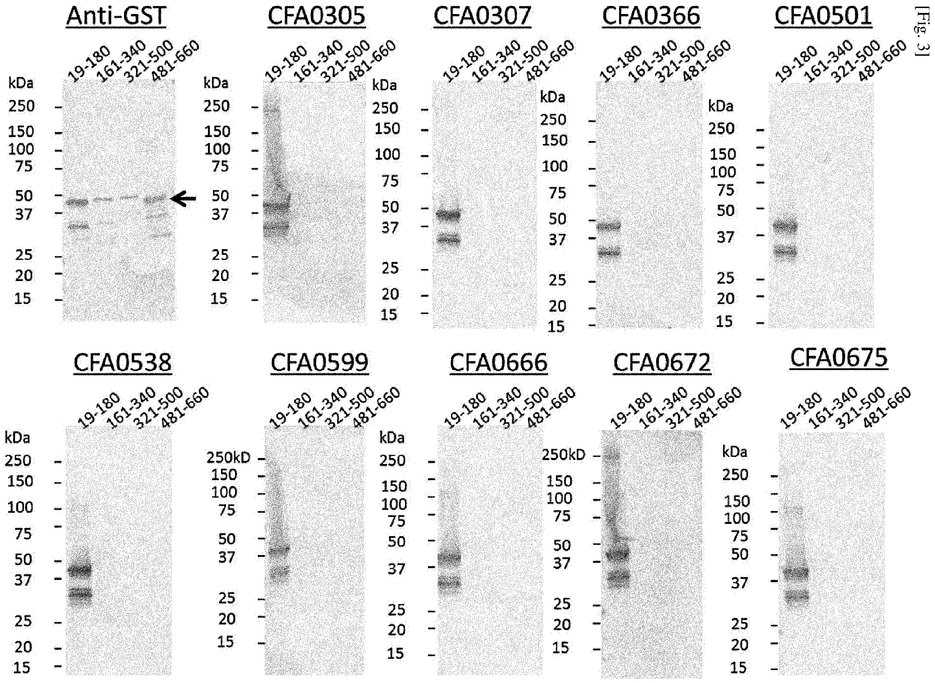

   Figure 3 illustrates Western Blot analysis against C5 beta-chain-derived fragments (amino acids 19-180, 161-340, 321-500, and 481-660 of SEQ ID NO:40) fused to GST-tag, as described in Example 4.1. CFA0305, CFA0307, CFA0366, CFA0501, CFA0538, CFA0599, CFA0666, CFA0672, and CFA0675 are antibodies grouped into epitope C. Anti-GST antibody is a positive control. The position of the GST-fused C5 fragments (46-49 kDa) is marked with an arrow.
[Fig. 4]

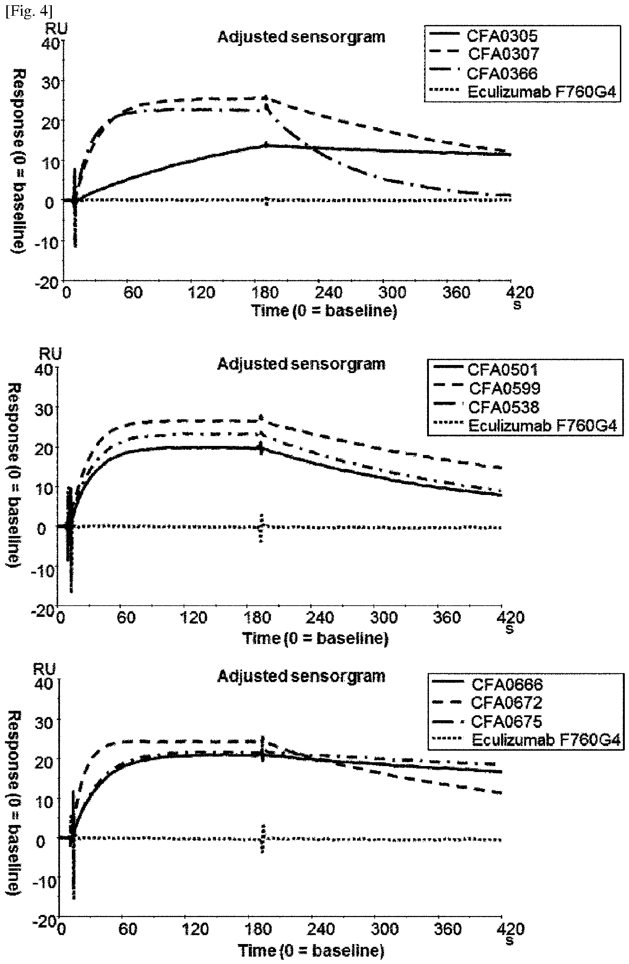

   Figure 4 illustrates BIACORE (registered trademark) sensorgrams of anti-C5 antibodies towards MG1-MG2 domain of C5 beta-chain, as described in Example 4.3. The upper panel shows the results of CFA0305 (solid line), CFA0307 (dashed line), CFA0366 (dash-dot line), and eculizumab (dotted line). The middle panel shows the results of CFA0501 (solid line), CFA0599 (dashed line), CFA0538 (dash-dot line), and eculizumab (dotted line). The lower panel shows the results of CFA0666 (solid line), CFA0672 (dashed line), CFA0675 (dash-dot line), and eculizumab (dotted line). CFA0305, CFA0307, CFA0366, CFA0501, CFA0538, CFA0599, CFA0666, CFA0672, and CFA0675 are antibodies grouped into epitope C. Eculizumab is a control anti-C5 antibody.
[Fig. 5A]

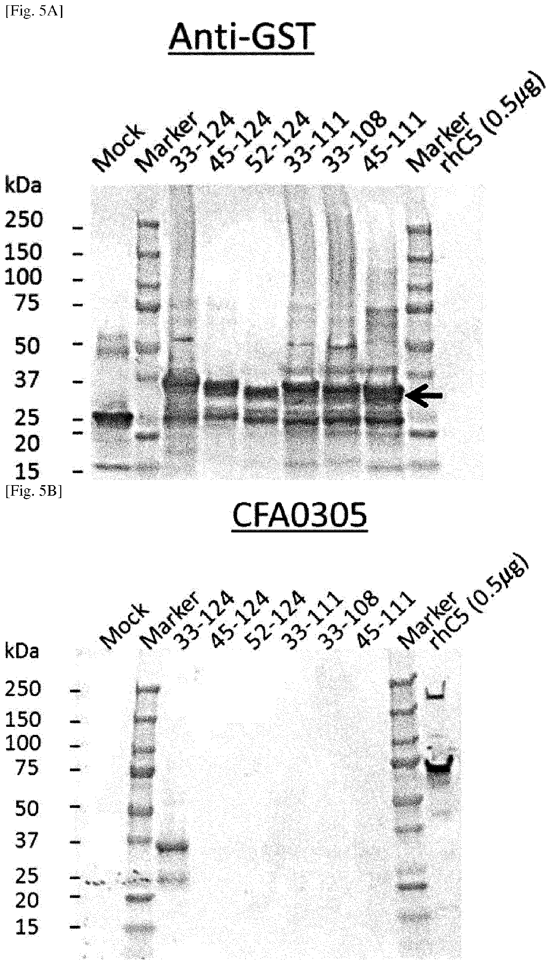

   Figure 5A illustrates Western Blot analysis against MG1-MG2 domain-derived peptide fragments (amino acids 33-124, 45-124, 52-124, 33-111, 33-108, and 45-111 of SEQ ID NO:40) fused to GST-tag, as described in Example 4.4. Anti-GST antibody is used as an antibody for reaction. The position of the GST-fused C5 fragments (35-37kDa) is marked with an arrow.
[Fig. 5B]
   Figure 5B illustrates Western Blot analysis against MG1-MG2 domain-derived peptide fragments (amino acids 33-124, 45-124, 52-124, 33-111, 33-108, and 45-111 of SEQ ID NO:40) fused to GST-tag, as described in Example 4.4. CFA0305 is used as an antibody for reaction.
[Fig. 5C]

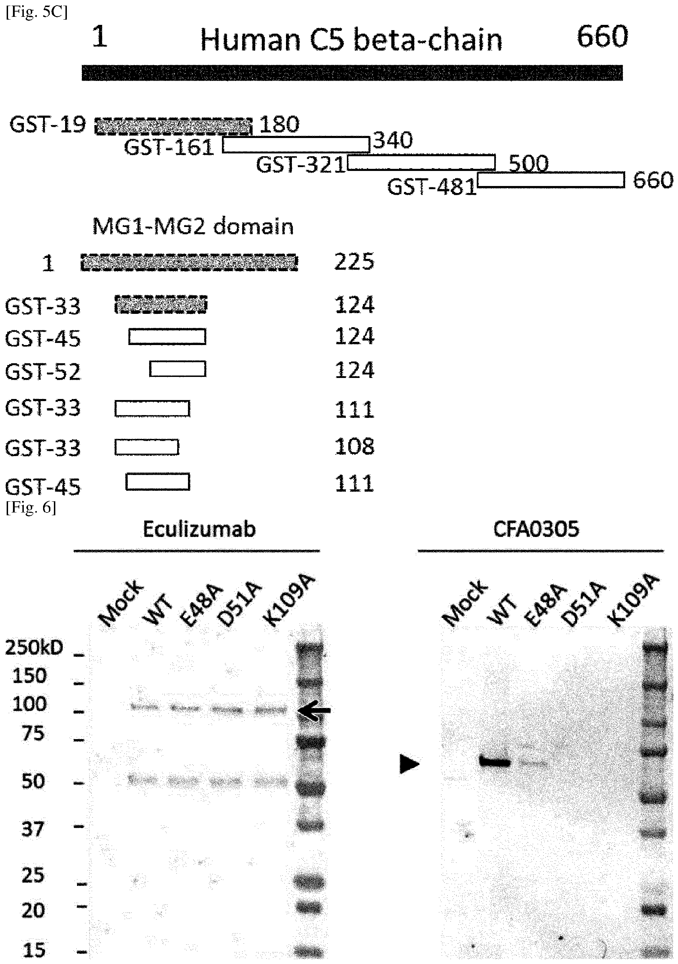

   Figure 5C summarizes binding reactions of anti-C5 antibodies to C5 beta-chain-derived fragments, as described in Example 4.4. The fragments to which the anti-C5 antibodies grouped into epitope C (CFA0305, CFA0307, CFA0366, CFA0501, CFA0538, CFA0599, CFA0666, CFA0672, and CFA0675) bind are shown in gray, and the fragments to which they don't bind are shown in white.
[Fig. 6]
   Figure 6 illustrates Western Blot analysis against C5 point mutants in which E48, D51, and K109 of the beta-chain is substituted with alanine (E48A, D51A, and K109A, respectively), as described in Example 4.5. In the left panel, eculizumab (anti-C5 antibody, alpha-chain binder) is used as an antibody for reaction and the position of the alpha-chain of C5 (approx. 113kDa) is marked with an arrow. In the right panel, CFA0305 (grouped into epitope C, beta-chain binder) is used as an antibody for reaction and the position of the beta-chain of C5 (approx. 74kDa) is marked with an arrowhead.
[Fig. 7]

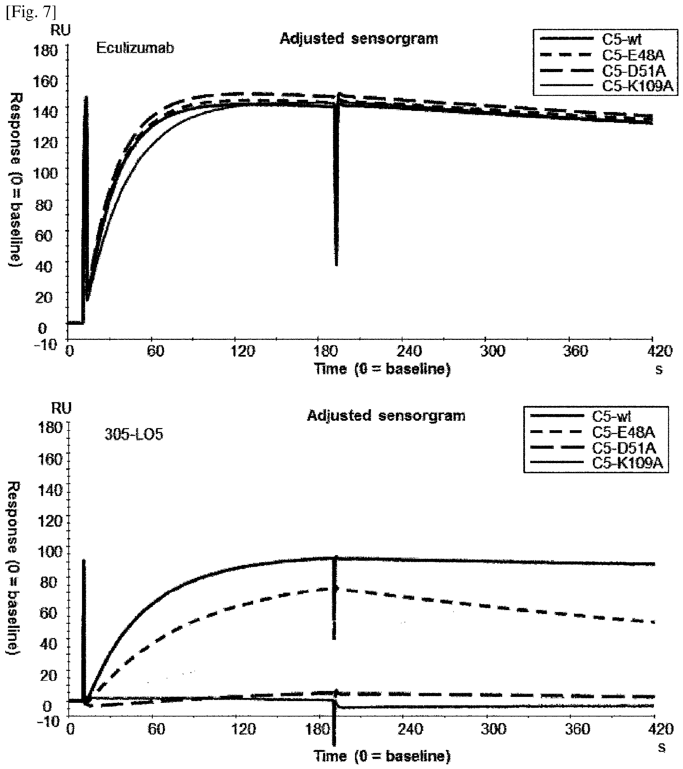

   Figure 7 presents BIACORE (registered trademark) sensorgrams showing the interaction of eculizumab-F760G4 (upper panel) or 305LO5 (lower panel) with C5 mutants, as described in Example 4.6. Sensorgrams were obtained by injection of C5-wt (thick solid curve), C5-E48A (short-dashed curve), C5-D51A (long-dashed curve), and C5-K109A (thin solid curve), respectively, over sensor surface immobilized with eculizumab-F760G4 or 305LO5. Eculizumab is a control anti-C5 antibody. 305LO5 is a humanized antibody of CFA0305 (grouped into epitope C), as described in Example 2.3.
[Fig. 8]

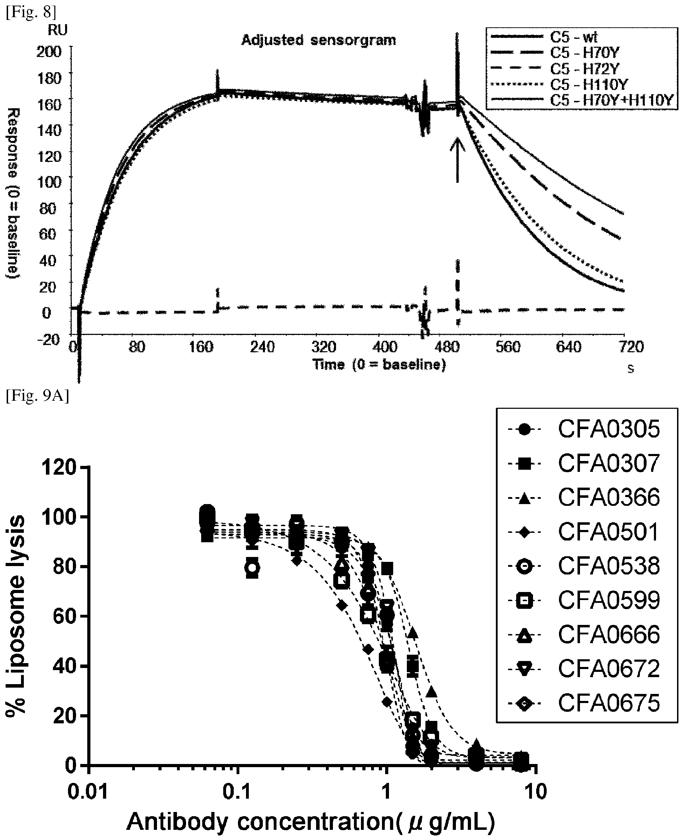

   Figure 8 presents BIACORE (registered trademark) sensorgrams showing the interaction of 305LO5 with C5 His mutants to assess pH-dependency, as described in Example 4.7. Sensorgrams were obtained by injection of C5-wt (thick solid curve), C5-H70Y (long-dashed curve), C5-H72Y (short-dashed curve), C5-H110Y (dotted curve), and C5-H70Y+H110Y (thin solid curve), respectively, over sensor surface immobilized with 305LO5. The antibody/antigen complexes were allowed to dissociate at pH7.4, followed by additional dissociation at pH5.8 (pointed by an arrow) to assess the pH-dependent interactions.
[Fig. 9A]
   Figure 9A illustrates inhibition of complement-activated liposome lysis by anti-C5 antibodies, as described in Example 5.1. The results of CFA0305, CFA0307, CFA0366, CFA0501, CFA0538, CFA0599, CFA0666, CFA0672, and CFA0675 grouped into epitope C, as described in Example 2.2, are shown.
[Fig. 9B]

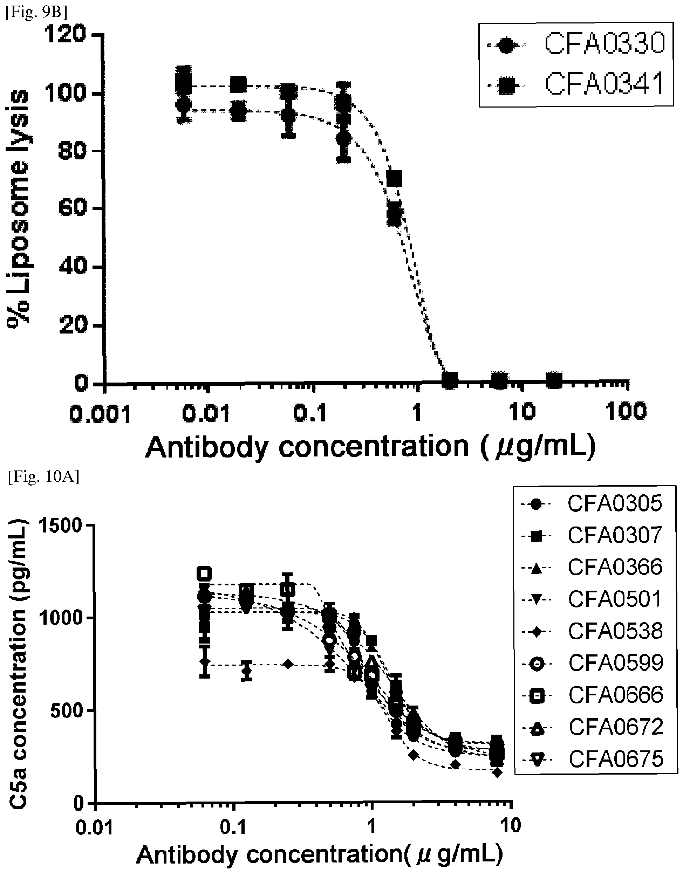

   Figure 9B illustrates inhibition of complement-activated liposome lysis by anti-C5 antibodies, as described in Example 5.1. The results of antibodies CFA0330 and CFA0341 grouped into epitope B, as described in Example 2.2, are shown.
[Fig. 10A]
   Figure 10A illustrates inhibition of C5a generation by anti-C5 antibodies, as described in Example 5.2. C5a concentrations were quantified in the supernatants obtained during the liposome lysis assay described in Figure 9A.
[Fig. 10B]

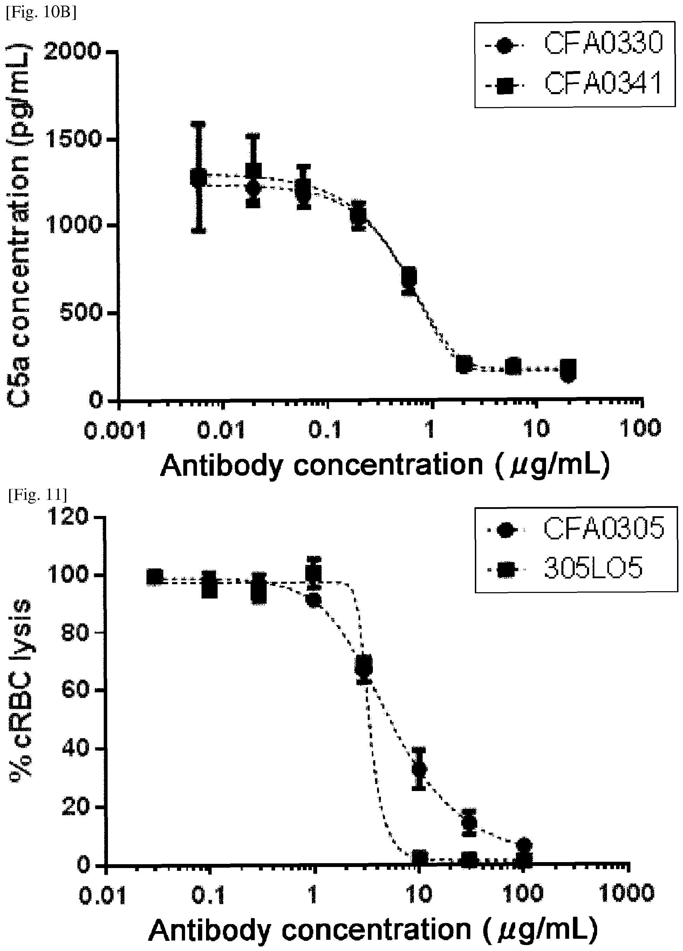

   Figure 10B illustrates inhibition of C5a generation by anti-C5 antibodies, as described in Example 5.2. C5a concentrations were quantified in the supernatants obtained during the liposome lysis assay described in Figure 9B.
[Fig. 11]
   Figure 11 illustrates inhibition of complement-activated hemolysis by anti-C5 antibodies, as described in Example 5.3. Complements were activated via the classical pathway.
[Fig. 12]

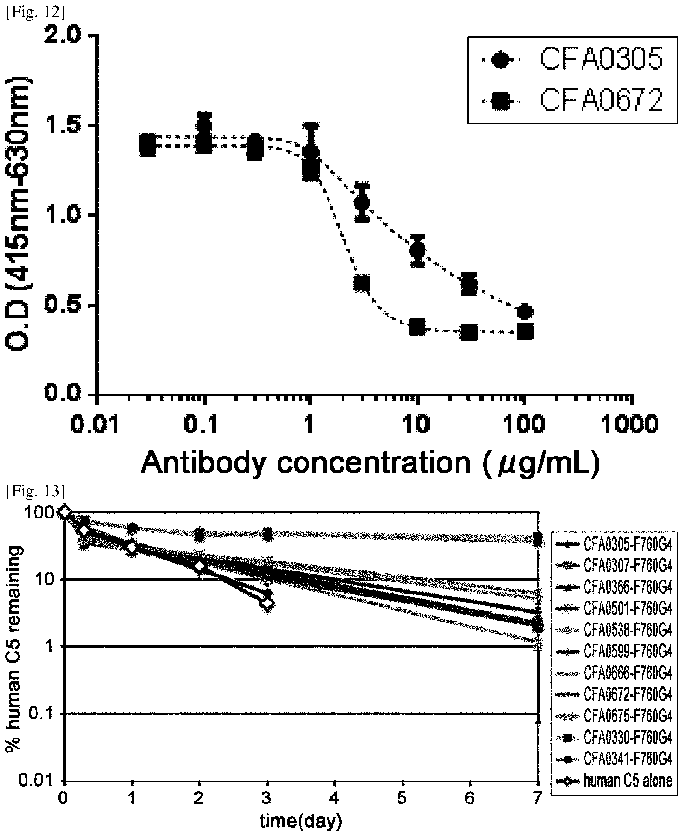

   Figure 12 illustrates inhibition of complement-activated hemolysis by anti-C5 antibodies, as described in Example 5.4. Complements were activated via the alternative pathway.
[Fig. 13]
   Figure 13 illustrates the time course of plasma human C5 concentration after intravenous administration of human C5 alone or human C5 and an anti-human C5 antibody in mice assessing C5 clearance, as described in Example 6.2. CFA0305, CFA0307, CFA0366, CFA0501, CFA0538, CFA0599, CFA0666, CFA0672, and CFA0675 are antibodies grouped into epitope C and CFA0330 and CFA0341 are antibodies grouped into epitope B, as described in Example 2.2.
[Fig. 14]

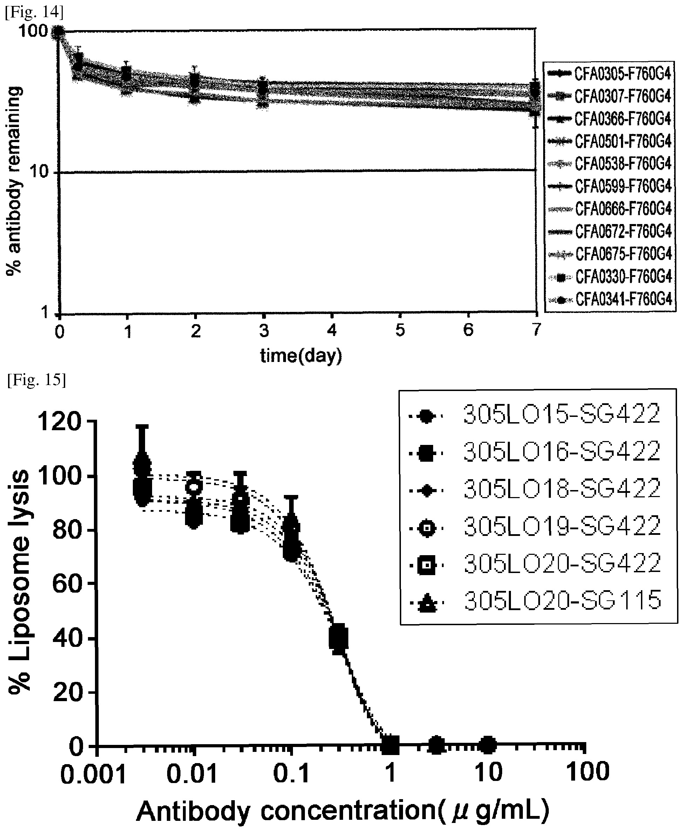

   Figure 14 illustrates the time course of plasma anti-human C5 antibody concentration after intravenous administration of human C5 and an anti-human C5 antibody in mice assessing antibody pharmacokinetics, as described in Example 6.3. CFA0305, CFA0307, CFA0366, CFA0501, CFA0538, CFA0599, CFA0666, CFA0672, and CFA0675 are antibodies grouped into epitope C and CFA0330 and CFA0341 are antibodies grouped into epitope B, as described in Example 2.2.
[Fig. 15]
   Figure 15 illustrates inhibition of complement-activated liposome lysis by anti-C5 antibodies, as described in Example 9.1. The results of antibodies 305LO15-SG422, 305LO16-SG422, 305LO18-SG422, 305LO19-SG422, 305LO20-SG422, and 305L020-SG115 are shown.
[Fig. 16]

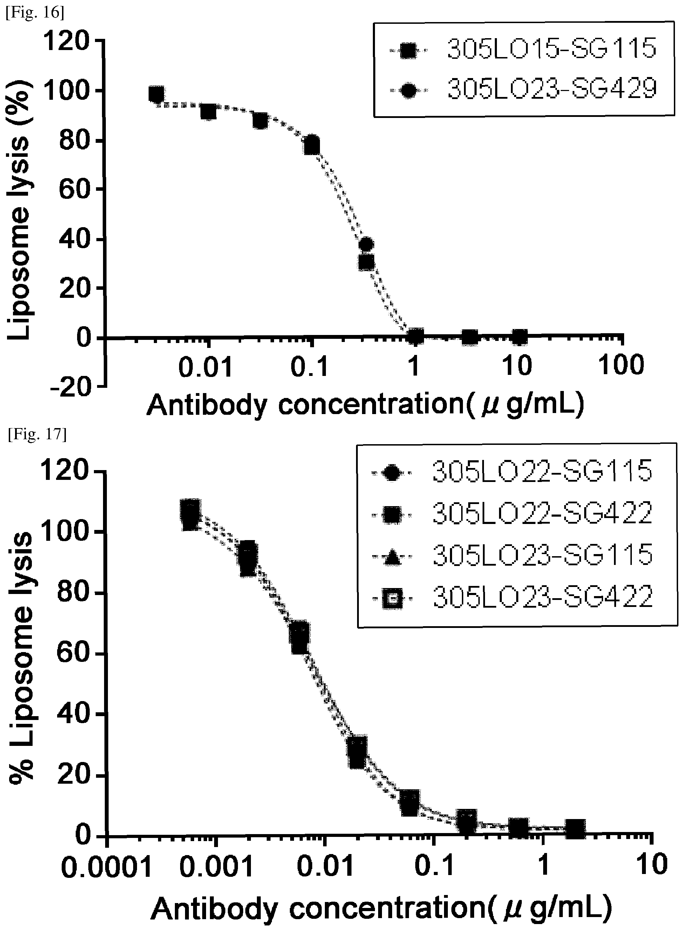

   Figure 16 illustrates inhibition of complement-activated liposome lysis by anti-C5 antibodies, as described in Example 9.1. The results of antibodies 305LO15-SG115 and 305LO23-SG429 are shown.
[Fig. 17]
   Figure 17 illustrates inhibition of complement-activated liposome lysis by anti-C5 antibodies, as described in Example 9.1. The results of antibodies 305LO22-SG115, 305LO22-SG422, 305LO23-SG115, and 305LO23-SG422 are shown.
[Fig. 18]

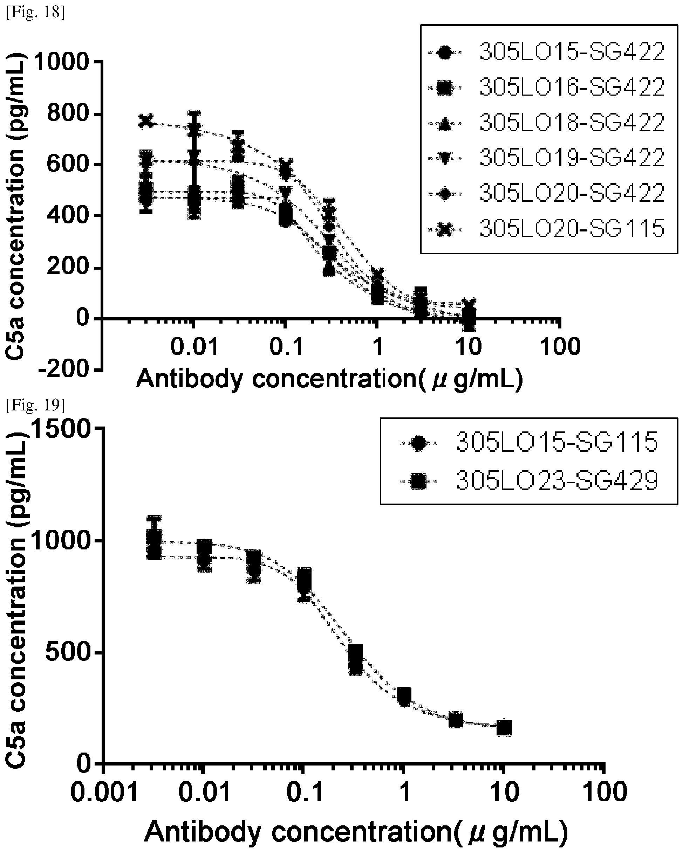

   Figure 18 illustrates inhibition of C5a generation by anti-C5 antibodies, as described in Example 9.2. C5a concentrations were quantified in the supernatants obtained during the liposome lysis assay described in Figure 15.
[Fig. 19]
   Figure 19 illustrates inhibition of C5a generation by anti-C5 antibodies, as described in Example 9.2. C5a concentrations were quantified in the supernatants obtained during the liposome lysis assay described in Figure 16.
[Fig. 20]

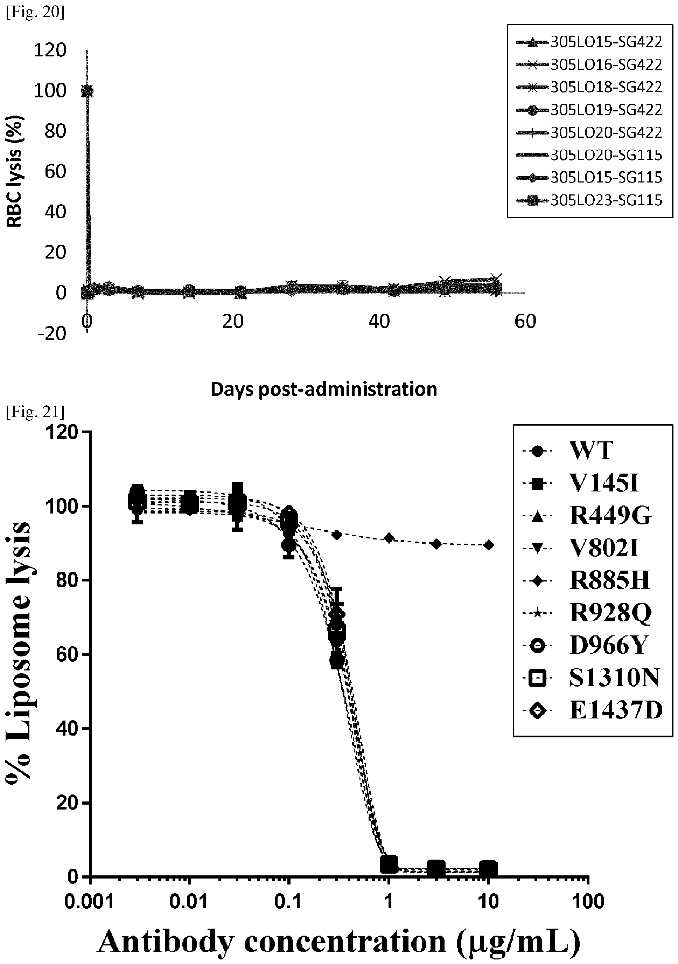

   Figure 20 illustrates inhibition of complement activity in monkey plasma by anti-C5 antibodies, as described in Example 9.3. Anti-C5 antibodies were administered into cynomolgus monkeys, and complement activities in plasma of the monkeys were measured in hemolysis assay.
[Fig. 21]
   Figure 21 illustrates inhibition of biological activity of wild type C5 (WT) and C5 variants (V145I, R449G, V802I, R885H, R928Q, D966Y, S1310N, and E1437D) by an anti-C5 antibody (eculizumab), as described in Example 9.4.
[Fig. 22]

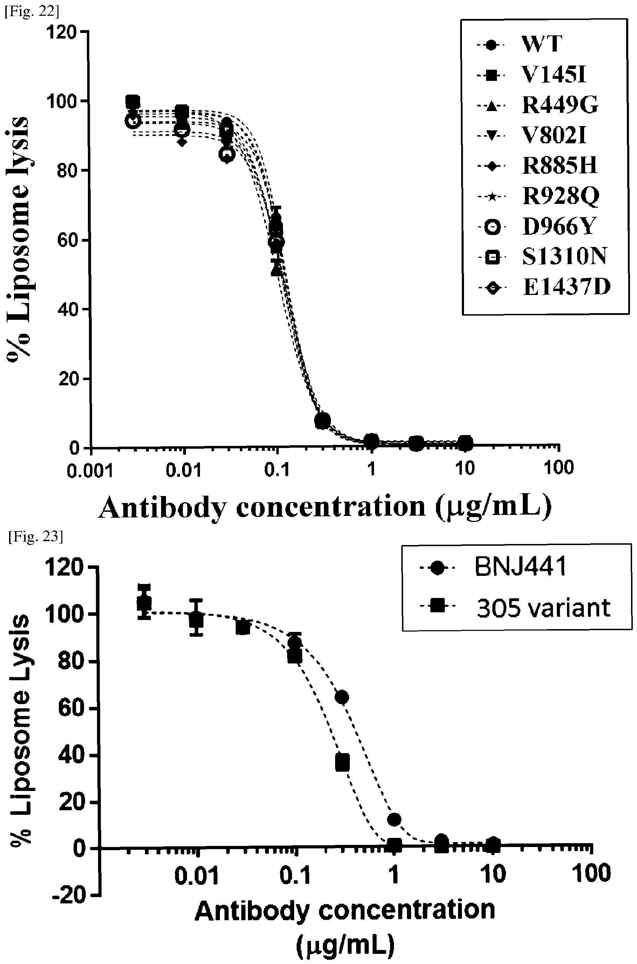

   Figure 22 illustrates inhibition of biological activity of wild type C5 (WT) and C5 variants (V145I, R449G, V802I, R885H, R928Q, D966Y, S1310N, and E1437D) by anti-C5 antibody (a 305 variant), as described in Example 9.4.
[Fig. 23]
   Figure 23 illustrates inhibition of complement-activated liposome lysis by anti-C5 antibodies (BNJ441 and a 305 variant), as described in Example 9.5.
[Fig. 24]

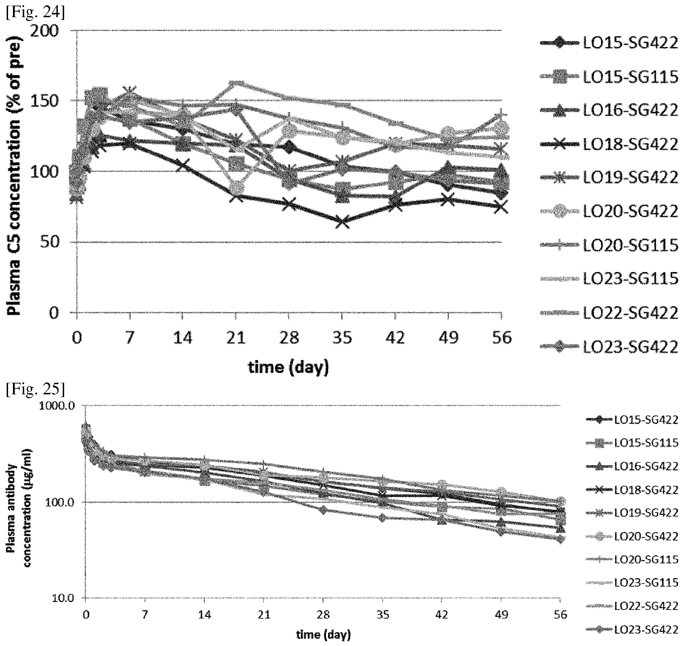

   Figure 24 illustrates the time course of plasma cynomolgus C5 concentration after intravenous administration of an anti-human C5 antibody in cynomolgus monkeys assessing C5 clearance, as described in Example 10.2.
[Fig. 25]
   Figure 25 illustrates the time course of plasma anti-human C5 antibody concentration after intravenous administration of an anti-human C5 antibody in cynomolgus monkeys assessing antibody pharmacokinetics, as described in Example 10.3.
[Fig. 26]

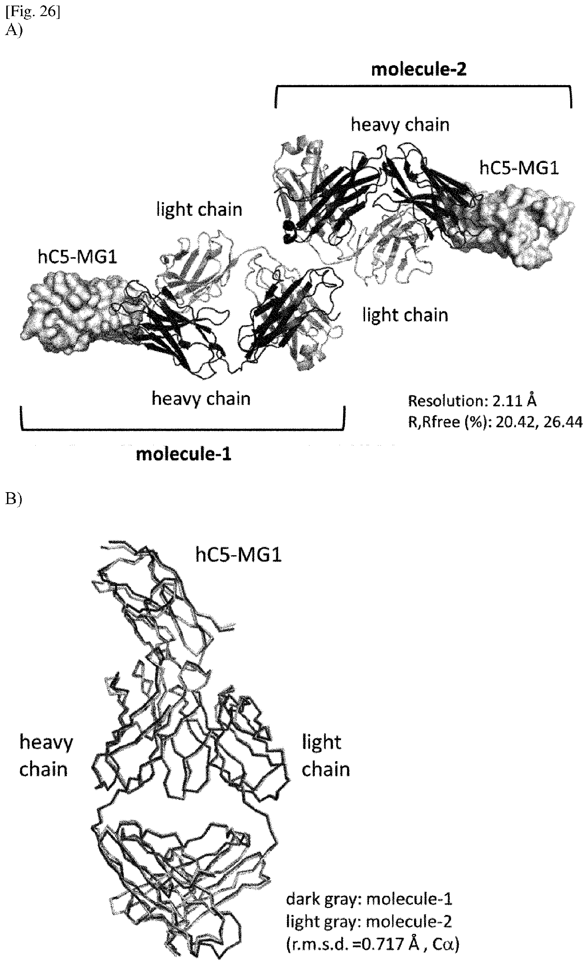

   Figures 26A and 26B illustrate the crystal structure of the 305 Fab bound to the human C5 (hC5)-MG1 domain, as described in Example 11.6. Figure 26A illustrates an asymmetric unit. MG1 is shown in surface representation and the 305 Fab is shown as ribbons (dark gray: heavy chain, light gray: light chain). Figure 26B illustrates molecules 1 and 2 superimposed (dark gray: molecule 1, light gray: molecule 2).
[Fig. 27A]

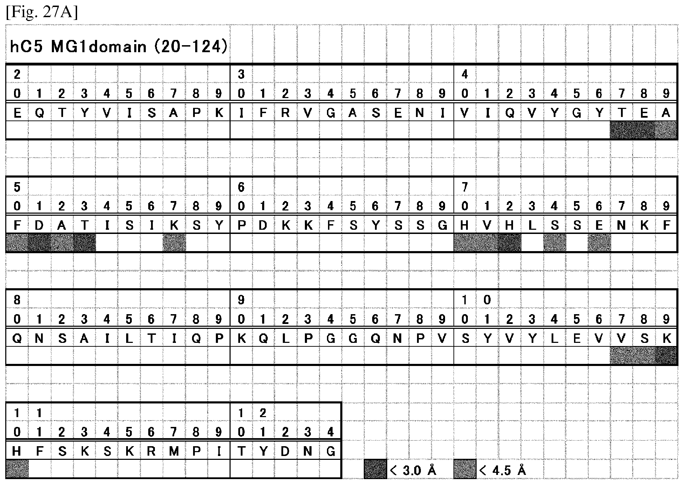

   Figure 27A illustrates the epitope of the 305 Fab contact region on the MG1 domain, as described in Example 11.6. Figure 27A illustrates epitope mapping in the MG1 amino acid sequence (dark gray: closer than 3.0 Angstrom(s), light gray: closer than 4.5 Angstrom(s)).
[Fig. 27B]

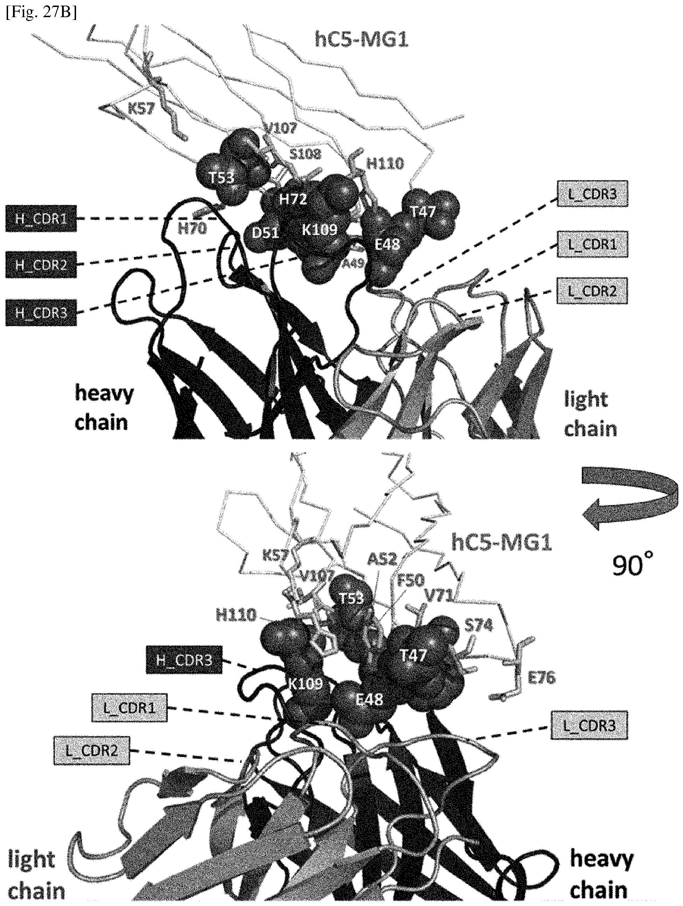

   Figure 27B illustrates the epitope of the 305 Fab contact region on the MG1 domain, as described in Example 11.6. Figure 27B illustrates epitope mapping in the crystal structure (dark gray spheres: closer than 3.0 Angstrom(s), light gray sticks: closer than 4.5 Angstrom(s)).
[Fig. 28A]

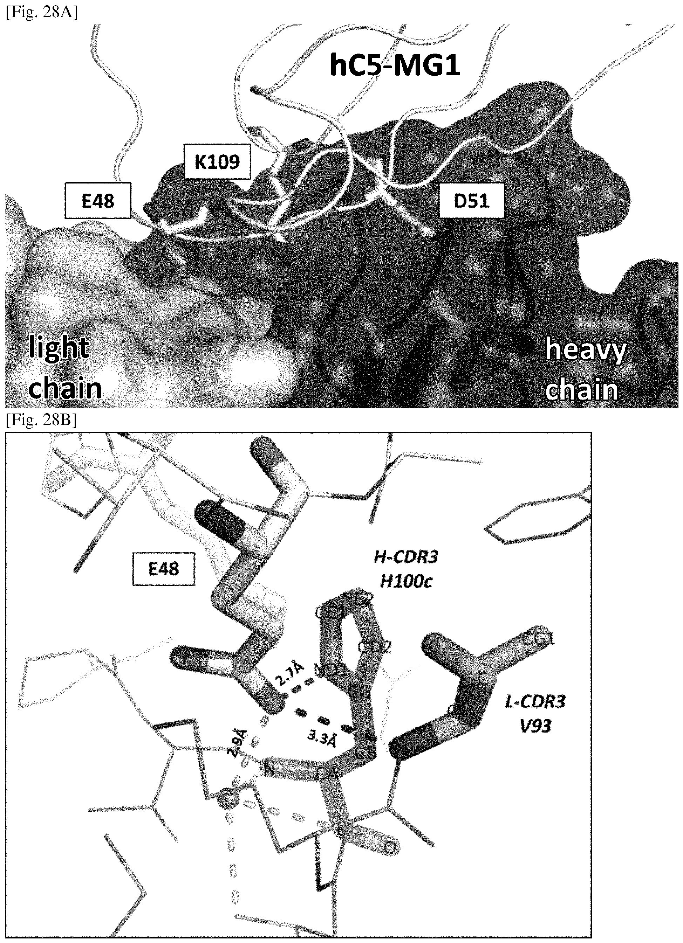

   Figure 28A illustrates a close-up view of the interactions E48, D51, and K109 (stick representation) with the 305 Fab (surface representation), as described in Example 11.7.
[Fig. 28B]
   Figure 28B illustrates interactions between E48 and its environment (dark gray dotted line: hydrogen bond with the Fab, light gray dotted line: water-mediated hydrogen bond), as described in Example 11.7.
[Fig. 28C]

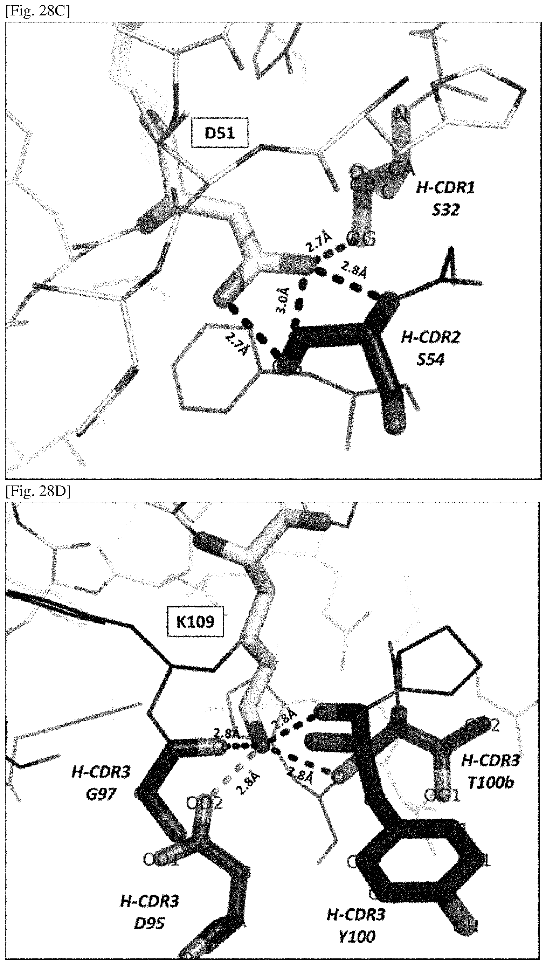

   Figure 28C illustrates interactions between D51 and its environment (dark gray dotted line: hydrogen bond with the Fab), as described in Example 11.7.
[Fig. 28D]
   Figure 28D illustrates interactions between K109 and its environment (dark gray dotted line: hydrogen bond with the Fab, light gray dotted line: salt bridge with H-CDR3_D95), as described in Example 11.7.
[Fig. 29A]

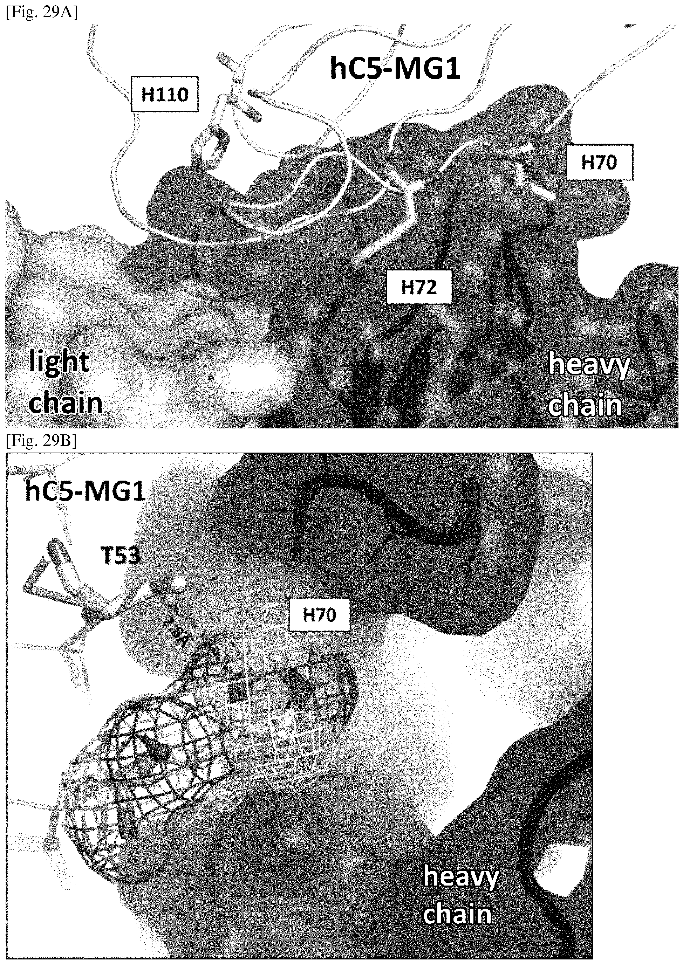

   Figure 29A illustrates a close-up view of the interactions of H70, H72, and H110 (stick representation) with the 305 Fab (surface representation), as described in Example 11.8, in the same orientation as Figure 28A.
[Fig. 29B]
   Figure 29B illustrates interactions between H70 and its environment, as described in Example 11.8. This histidine residue is indicated in stick and mesh representation. The hydrogen bond is indicated by dotted line.
[Fig. 29C]

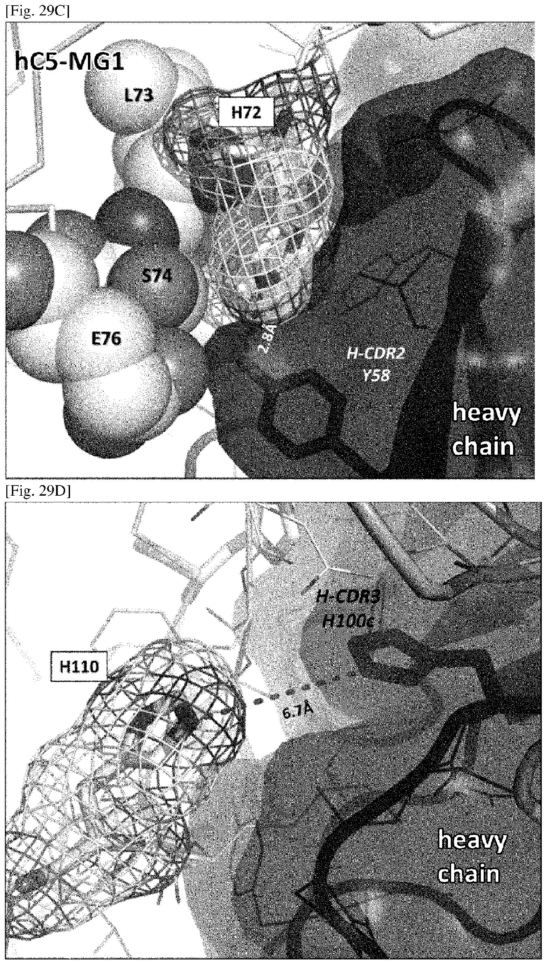

   Figure 29C illustrates interactions between H72 and its environment, as described in Example 11.8. This histidine residue is indicated in stick and mesh representation. The hydrogen bond is indicated by dotted line.
[Fig. 29D]
   Figure 29D illustrates interactions between H110 and its environment, as described in Example 11.8. This histidine residue is indicated in stick and mesh representation. The distance between H110 and H-CDR3_H100c is shown by dotted line.

### [Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### I. DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

For purposes of interpreting this specification, the following definitions will apply and whenever appropriate, terms used in the singular will also include the plural and vice versa. It is to be understood that the terminology used herein is for the purpose of describing particular embodiments only, and is not intended to be limiting.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-C5 antibody" and "an antibody that binds to C5" refer to an antibody that is capable of binding C5 with sufficient affinity such that the antibody is useful as a diagnostic and/or therapeutic agent in targeting C5. In one embodiment, the extent of binding of an anti-C5 antibody to an unrelated, non-C5 protein is less than about 10% of the binding of the antibody to C5 as measured, e.g., by a radioimmunoassay (RIA). In certain embodiments, an antibody that binds to C5 has a dissociation constant (Kd) of 1 micro M or less, 100 nM or less, 10 nM or less, 1 nM or less, 0.1 nM or less, 0.01 nM or less, or 0.001 nM or less (e.g., 10⁻⁸ M or less, e.g., from 10⁻⁸ M to 10⁻¹³ M, e.g., from 10⁻⁹ M to 10⁻¹³ M). In certain embodiments, an anti-C5 antibody binds to an epitope of C5 that is conserved among C5 from different species.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multispecific antibodies formed from antibody fragments.

An "antibody that binds to the same epitope" as a reference antibody refers to an antibody that blocks binding of the reference antibody to its antigen in a competition assay, and/or conversely, the reference antibody blocks binding of the antibody to its antigen in a competition assay. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgGs, IgG₄, IgA₁, and IgA₂. The heavy chain constant domains that correspond to the different classes of immunoglobulins are called alpha, delta, epsilon, gamma, and mu, respectively.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "epitope" includes any determinant capable of being bound by an antibody. An epitope is a region of an antigen that is bound by an antibody that targets that antigen, and includes specific amino acids that directly contact the antibody. Epitope determinants can include chemically active surface groupings of molecules such as amino acids, sugar side chains, phosphoryl or sulfonyl groups, and can have specific three dimensional structural characteristics, and/or specific charge characteristics. Generally, antibodies specific for a particular target antigen will preferentially recognize an epitope on the target antigen in a complex mixture of proteins and/or macromolecules.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include: (a) hypervariable loops occurring at amino acid residues 26-32 (L1), 50-52 (L2), 91-96 (L3), 26-32 (H1), 53-55 (H2), and 96-101 (H3) (Chothia, J. Mol. Biol. 196:901-917 (1987));(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, NIH, Bethesda, MD (1991));(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262:732-745 (1996)); and(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC) methods. For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-C5 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phagedisplay methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (kappa) and lambda (lambda), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared. The ALIGN-2 sequence comparison computer program was authored by Genentech, Inc., and the source code has been filed with user documentation in the US Copyright Office, Washington D.C., 20559, where it is registered under US Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y, where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

The term "C5", as used herein, encompasses any native C5 from any vertebrate source, including mammals such as primates (e.g., humans and monkeys) and rodents (e.g., mice and rats). Unless otherwise indicated, the term "C5" refers to a human C5 protein having the amino acid sequence shown in SEQ ID NO: 39 and containing the beta chain sequence shown in SEQ ID NO: 40. The term encompasses "full-length," unprocessed C5 as well as any form of C5 that results from processing in the cell. The term also encompasses naturally occurring variants of C5, e.g., splice variants or allelic variants. The amino acid sequence of an exemplary human C5 is shown in SEQ ID NO: 39 ("wild-type" or "WT" C5). The amino acid sequence of an exemplary beta chain of human C5 is shown in SEQ ID NO: 40. The amino acid sequences of exemplary MG1, MG2 and MG1-MG2 domains of the beta chain of human C5 are shown in SEQ ID NO: 41, 42, and 43, respectively. The amino acid sequences of exemplary cynomolgus monkey and murine C5 are shown in SEQ ID NO: 44 and 105, respectively. Amino acid residues 1-19 of SEQ ID NOs: 39, 40, 43, 44, and 105 correspond to a signal sequence that is removed during processing in the cell and is thus missing from the corresponding exemplary amino acid sequence.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are for use in a method of delaying development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### II. COMPOSITIONS AND METHODS

In one aspect, the invention is based, in part, on anti-C5 antibodies comprising a VH sequence of SEQ ID NO: 106 and a VL sequence of SEQ ID NO: 111 and methods of producing them. Antibodies of the invention are useful, e.g., for use in a method of diagnosis or treatment of a complement-mediated disease or condition which involves excessive or uncontrolled activation of C5.

### A. Exemplary Anti-C5 Antibodies

The disclosure provides isolated antibodies that bind to C5. In certain embodiments, an anti-C5 antibody of the present disclosure binds to an epitope within the beta chain of C5. In certain embodiments, the anti-C5 antibody binds to an epitope within the MG1-MG2 domain of the beta chain of C5. In certain embodiments, the anti-C5 antibody binds to an epitope within a fragment consisting of amino acids 19-180 of the beta chain of C5. In certain embodiments, the anti-C5 antibody binds to an epitope within the MG1 domain (amino acids 20-124 of SEQ ID NO: 40 (SEQ ID NO: 41)) of the beta chain of C5. In certain embodiments, the anti-C5 antibody binds to an epitope within a fragment consisting of amino acids 33-124 of the beta chain of C5 (SEQ ID NO: 40). In another embodiment, the antibody does not bind to a fragment shorter than the fragment consisting of amino acids 33-124 of the beta chain of C5, e.g., a fragment consisting of amino acids 45-124, 52-124, 33-111, 33-108, or 45-111 of the beta chain of C5 (SEQ ID NO: 40).

The disclosure provides anti-C5 antibodies that exhibit pH-dependent binding characteristics. As used herein, the expression "pH-dependent binding" means that the antibody exhibits "reduced binding to C5 at acidic pH as compared to its binding at neutral pH" (for purposes of the present disclosure, both expressions may be used interchangeably). For example, antibodies "with pH-dependent binding characteristics" include antibodies that bind to C5 with higher affinity at neutral pH than at acidic pH. The antibodies of the present disclosure bind to C5 with at least 2, 3, 5, 10, 15, 20, 25, 30, 35, 40, 45, 50, 55, 60, 65, 70, 75, 80, 85, 90, 95, 100, 200, 400, 1000, 10000, or more times higher affinity at neutral pH than at acidic pH. In some embodiments, the antibodies bind to C5 with higher affinity at pH7.4 than at pH5.8. In further embodiments, the antibodies of the present disclosure bind to C5 with at least 2, 3, 5, 10, 15, 20, 25, 30, 35, 40, 45, 50, 55, 60, 65, 70, 75, 80, 85, 90, 95, 100, 200, 400, 1000, 10000, or more times higher affinity at pH7.4 than at pH5.8.

The "affinity" of an antibody for C5, for purposes of the present disclosure, is expressed in terms of the KD of the antibody. The KD of an antibody refers to the equilibrium dissociation constant of an antibody-antigen interaction. The greater the KD value is for an antibody binding to its antigen, the weaker its binding affinity is for that particular antigen. Accordingly, as used herein, the expression "higher affinity at neutral pH than at acidic pH" (or the equivalent expression "pH-dependent binding") means that the KD for the antibody binding to C5 at acidic pH is greater than the KD for the antibody binding to C5 at neutral pH. For example, in the context of the present disclosure, an antibody is considered to bind to C5 with a higher affinity at neutral pH than at acidic pH if the KD of the antibody binding to C5 at acidic pH is at least 2 times greater than the KD of the antibody binding to C5 at neutral pH. Thus, the present disclosure includes antibodies that bind to C5 at acidic pH with a KD that is at least 2, 3, 5, 10, 15, 20, 25, 30, 35, 40, 45, 50, 55, 60, 65, 70, 75, 80, 85, 90, 95, 100, 200, 400, 1000, 10000, or more times greater than the KD of the antibody binding to C5 at neutral pH. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater.

An antibody is considered to bind to C5 with a higher affinity at neutral pH than at acidic pH if the KD of the antibody binding to C5 at pH5.8 is at least 2 times greater than the KD of the antibody binding to C5 at pH7.4. In some embodiments the disclosed antibodies bind to C5 at pH5.8 with a KD that is at least 3, 5, 10, 15, 20, 25, 30, 35, 40, 45, 50, 55, 60, 65, 70, 75, 80, 85, 90, 95, 100, 200, 400, 1000, 10000, or more times greater than the KD of the antibody binding to C5 at pH7.4. In another embodiment, the KD value of the antibody at pH7.4 can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at pH5.8 can be 10⁻⁹ M, 10⁻⁸ M, 10⁻⁷ M, 10⁻⁶ M, or greater.

The binding properties of an antibody for a particular antigen may also be expressed in terms of the kd of the antibody. The kd of an antibody refers to the dissociation rate constant of the antibody with respect to a particular antigen and is expressed in terms of reciprocal seconds (i.e., sec⁻¹). An increase in kd value signifies weaker binding of an antibody to its antigen. The present disclosure therefore includes antibodies that bind to C5 with a higher kd value at acidic pH than at neutral pH. The present disclosure includes antibodies that bind to C5 at acidic pH with a kd that is at least 2, 3, 5, 10, 15, 20, 25, 30, 35, 40, 45, 50, 55, 60, 65, 70, 75, 80, 85, 90, 95, 100, 200, 400, 1000, 10000, or more times greater than the kd of the antibody binding to C5 at neutral pH. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻¹ 1/s, or greater. The disclosure also includes antibodies that bind to C5 with a higher kd value at pH5.8 than at pH7.4. The present disclosure includes antibodies that bind to C5 at pH5.8 with a kd that is at least 3, 5, 10, 15, 20, 25, 30, 35, 40, 45, 50, 55, 60, 65, 70, 75, 80, 85, 90, 95, 100, 200, 400, 1000, 10000, or more times greater than the kd of the antibody binding to C5 at pH7.4. In another embodiment, the kd value of the antibody at pH7.4 can be 10⁻² 1/s, 10⁻³ 1/s, 10⁻⁴ 1/s, 10⁻⁵ 1/s, 10⁻⁶ 1/s, or less. In another embodiment, the kd value of the antibody at pH5.8 can be 10⁻³ 1/s, 10⁻² 1/s, 10⁻¹ 1/s, or greater.

In certain instances, a "reduced binding to C5 at acidic pH as compared to its binding at neutral pH" is expressed in terms of the ratio of the KD value of the antibody binding to C5 at acidic pH to the KD value of the antibody binding to C5 at neutral pH (or vice versa). For example, an antibody may be regarded as exhibiting "reduced binding to C5 at acidic pH as compared to its binding at neutral pH", for purposes of the present disclosure, if the antibody exhibits an acidic/neutral KD ratio of 2 or greater. In certain exemplary embodiments, the pH5.8/pH7.4 KD ratio for an antibody of the present disclosure is 2 or greater. In certain exemplary embodiments, the acidic/neutral KD ratio for an antibody of the present disclosure can be 2, 3, 5, 10, 15, 20, 25, 30, 35, 40, 45, 50, 55, 60, 65, 70, 75, 80, 85, 90, 95, 100, 200, 400, 1000, 10000, or greater. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater. In further instances an antibody may be regarded as exhibiting "reduced binding to C5 at acidic pH as compared to its binding at neutral pH", for purposes of the present disclosure, if the antibody exhibits an pH5.8/pH7.4 KD ratio of 2 or greater. In certain exemplary embodiments, the pH5.8/pH7.4 KD ratio for an antibody of the present disclosure can be 3, 5, 10, 15, 20, 25, 30, 35, 40, 45, 50, 55, 60, 65, 70, 75, 80, 85, 90, 95, 100, 200, 400, 1000, 10000, or greater. In another embodiment, the KD value of the antibody at pH7.4 can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at pH5.8 can be 10⁻⁹ M, 10⁻⁸ M, 10⁻⁷ M, 10⁻⁶ M, or greater.

In certain instances, a "reduced binding to C5 at acidic pH as compared to its binding at neutral pH" is expressed in terms of the ratio of the kd value of the antibody binding to C5 at acidic pH to the kd value of the antibody binding to C5 at neutral pH (or vice versa). For example, an antibody may be regarded as exhibiting "reduced binding to C5 at acidic pH as compared to its binding at neutral pH", for purposes of the present disclosure, if the antibody exhibits an acidic/neutral kd ratio of 2 or greater. In certain exemplary embodiments, the pH5.8/pH7.4 kd ratio for an antibody of the present disclosure is 2 or greater. In certain exemplary embodiments, the acidic/neutral kd ratio for an antibody of the present disclosure can be 2, 3, 5, 10, 15, 20, 25, 30, 35, 40, 45, 50, 55, 60, 65, 70, 75, 80, 85, 90, 95, 100, 200, 400, 1000, 10000, or greater. In further exemplary embodiments, the pH 5.8/pH 7.4 kd ratio for an antibody of the present disclosure can be 2, 3, 5, 10, 15, 20, 25, 30, 35, 40, 45, 50, 55, 60, 65, 70, 75, 80, 85, 90, 95, 100, 200, 400, 1000, 10000, or greater. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In a further embodiment, the kd value of the antibody at pH 7.4 can be 10⁻² 1/s, 10⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻¹ 1/s, or greater. In a further embodiment, the kd value of the antibody at pH5.8 can be 10⁻³ 1/s, 10⁻² 1/s, 10⁻¹ 1/s, or greater.

As used herein, the expression "acidic pH" means a pH of 4.0 to 6.5. The expression "acidic pH" includes pH values of any one of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, and 6.5. In particular aspects, the "acidic pH" is 5.8.

As used herein, the expression "neutral pH" means a pH of 6.7 to about 10.0. The expression "neutral pH" includes pH values of any one of 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0. In particular aspects, the "neutral pH" is 7.4.

KD values, and kd values, as expressed herein, may be determined using a surface plasmon resonance-based biosensor to characterize antibody-antigen interactions. (See, e.g., Example 3, herein). KD values, and kd values can be determined at 25 degrees C or 37 degrees C.

In certain embodiments, an anti-C5 antibody of the present disclosure binds to an epitope within the beta chain of C5 which consists of the MG1 domain (SEQ ID NO:41). In certain embodiments, an anti-C5 antibody of the present disclosure binds to an epitope within the beta chain (SEQ ID NO: 40) of C5 which comprises at least one fragment selected from the group consisting of amino acids 47-57, 70-76, and 107-110. In certain embodiments, an anti-C5 antibody of the present disclosure binds to an epitope within a fragment of the beta chain (SEQ ID NO: 40) of C5 which comprises at least one amino acid selected from the group consisting of Thr47, Glu48, Ala49, Phe50, Asp51, Ala52, Thr53, Lys57, His70, Val71, His72, Ser74, Glu76, Val107, Ser108, Lys109, and His110. In certain embodiments, an anti-C5 antibody of the present disclosure binds to an epitope within a fragment of the beta chain (SEQ ID NO: 40) of C5 which comprises at least one amino acid selected from the group consisting of Glu48, Asp51, His70, His72, Lys109, and His110. In certain embodiments, binding of an anti-C5 antibody of the present disclosure to a C5 mutant is reduced compared to its binding to wild type C5, wherein the C5 mutant has at least one amino acid substitution at a position selected from the group consisting of Glu48, Asp51, His72, and Lys109. In another embodiment, pH-dependent binding of an anti-C5 antibody of the present disclosure to a C5 mutant is reduced compared to its pH-dependent binding to wild type C5, wherein the C5 mutant has at least one amino acid substitution at a position selected from the group consisting of His70, His72, and His110. In a further embodiment, an amino acid at a position selected from Glu48, Asp51, and Lys109 is substituted with alanine, and an amino acid at a position selected from His70, His72, and His110 is substituted with tyrosine in the C5 mutant.

In certain embodiments, an anti-C5 antibody of the present disclosure competes for binding C5 with an antibody comprising a VH and VL pair selected from: (a) a VH of SEQ ID NO:1 and a VL of SEQ ID NO:11; (b) a VH of SEQ ID NO: 22 and a VL of SEQ ID NO:26; (c) a VH of SEQ ID NO:21 and a VL of SEQ ID NO:25; (d) a VH of SEQ ID NO: 5 and a VL of SEQ ID NO: 15; (e) a VH of SEQ ID NO:4 and a VL of SEQ ID NO: 14; (f) a VH of SEQ ID NO: 6 and a VL of SEQ ID NO: 16; (g) a VH of SEQ ID NO:2 and a VL of SEQ ID NO: 12; (h) a VH of SEQ ID NO: 3 and a VL of SEQ ID NO: 13; (i) a VH of SEQ ID NO:9 and a VL of SEQ ID NO:19; (j) a VH of SEQ ID NO:7 and a VL of SEQ ID NO: 17; (k) aVH of SEQ ID NO:8 and a VL of SEQ ID NO: 18; (l) a VH of SEQ ID NO: 23 and a VL of SEQ ID NO:27; and (m) a VH of SEQ ID NO: 10 and a VL of SEQ ID NO:20.

In certain embodiments, an anti-C5 antibody of the present disclosure binds C5 and contacts amino acid Asp51 (D51) of SEQ ID NO:39. In additional embodiments, an anti-C5 antibody of the present disclosure binds C5 and contacts amino acid Lys109 (K109) of SEQ ID NO:39. In a further embodiment, an anti-C5 antibody of the present disclosure binds C5 and contacts amino acid Asp51 (D51) and amino acid Lys109 (K109) of SEQ ID NO:39.

In certain embodiments, binding of an anti-C5 antibody of the present disclosure to a C5 mutant is reduced compared to its binding to wild type C5, wherein the C5 mutant has a Glu48Ala (E48A) substitution of SEQ ID NO:39. In another embodiment, pH-dependent binding of an anti-C5 antibody of the present disclosure to a C5 mutant is reduced compared to its pH-dependent binding to wild type C5, wherein the C5 mutant has a Glu48Ala (E48A) substitution of SEQ ID NO:39.

In a further embodiment, an anti-C5 antibody binds to a C5 protein consisting of the amino acid sequence of SEQ ID NO:39, but does not bind to a C5 protein consisting of the amino acid sequence of SEQ ID NO:39 with a H72Y substitution, wherein the C5 protein and the H72Y substituted C5 protein are prepared and screened under the same conditions. In a further embodiment, the anti-C5 antibody binds to a C5 protein consisting of the amino acid sequence of SEQ ID NO:39 at pH7.4, but does not bind to the H72Y substituted C5 protein at pH7.4.

Without being restricted to a particular theory, it can be speculated that the binding of an anti-C5 antibody to C5 is reduced (or almost lost) when an amino acid residue on C5 is substituted with another amino acid, which means that the amino acid residue on C5 is critical for the interactions between the anti-C5 antibody and C5, and that the antibody may recognize an epitope around the amino acid residue on C5.

It has been discovered in the present invention that a group of anti-C5 antibodies that compete with one another or bind to the same epitope can exhibit pH-dependent binding characteristics. Among amino acids, histidine, with a pKa value of approximately 6.0 to 6.5, can have different proton dissociation states between neutral and acidic pH. Therefore, a histidine residue on C5 can contribute to the pH-dependent interactions between an anti-C5 antibody and C5. Without being restricted to a particular theory, it can be speculated that an anti-C5 antibody may recognize a conformational structure around a histidine residue on C5, which is variable depending on pH. That speculation can be consistent with the experimental results described below: that the pH-dependency of an anti-C5 antibody is reduced (or almost lost) when a histidine residue on C5 is substituted with another amino acid (i.e., an anti-C5 antibody with pH-dependent binding characteristics binds to a histidine mutant of C5 with similar affinity to wild type C5 at neutral pH, while the same antibody binds to the histidine mutant of C5 with higher affinity than wild type C5 at acidic pH).

In certain embodiments, an anti-C5 antibody of the present disclosure binds to C5 from more than one species. In further embodiments, the anti-C5 antibody binds to C5 from human and a non-human animal. In further embodiments, the anti-C5 antibody binds to C5 from human and monkey (e.g., cynomolgus, rhesus macaque, marmoset, chimpanzee, or baboon).

In one aspect, the disclosure provides anti-C5 antibodies that inhibit activation of C5. In certain embodiments, anti-C5 antibodies are disclosed which prevent the cleavage of C5 to form C5a and C5b, thus preventing the generation of anaphylatoxic activity associated with C5a, as well as preventing the assembly of the C5b-9 membrane attack complex (MAC) associated with C5b. In certain embodiments, anti-C5 antibodies are provided which block the conversion of C5 into C5a and C5b by C5 convertase. In certain embodiments, anti-C5 antibodies are provided which block access of the C5 convertase to the cleavage site on C5. In certain embodiments, anti-C5 antibodies are disclosed which block hemolytic activity caused by the activation of C5. In further embodiments, anti-C5 antibodies of the present disclosure inhibit the activation of C5 via classical pathway and/or alternative pathway.

In one aspect, the disclosure provides anti-C5 antibodies that inhibit activation of a C5 variant. A C5 variant means a genetic variant of C5 which is due to genetic variation such as a mutation, polymorphism or allelic variation. A genetic variation may comprise a deletion, substitution or insertion of one or more nucleotides. A C5 variant may comprise one or more genetic variations in C5. In certain embodiments, the C5 variant has biological activity similar to wild type C5. Such C5 variant may comprise at least one variation selected from the group consisting of V145I, R449G, V802I, R885H, R928Q, D966Y, S1310N, and E1437D. Herein, R885H, for example, means a genetic variation where arginine at position 885 is substituted by histidine. In certain embodiments, an anti-C5 antibody of the present disclosure inhibits activation of both wild type C5 and at least one C5 variant selected from the group consisting of V145I, R449G, V802I, R885H, R928Q, D966Y, S1310N, and E1437D.

In one aspect of the invention, the antibody comprises a VH sequence of SEQ ID NO:106 and a VL sequence of SEQ ID NO: 111.

In certain embodiments, an anti-C5 antibody of the present invention comprises a VH and VL as in any of the embodiments provided above and a heavy chain constant region comprising the amino acid sequence of SEQ ID NO: 114. In certain embodiments, an anti-C5 antibody of the present invention comprises a VH and VL as in any of the embodiments provided above and a light chain constant region comprising the amino acid sequence SEQ ID NO: 38.

In another aspect, the disclosure provides an antibody that binds to the same epitope as an anti-C5 antibody provided herein. For example, in certain embodiments, an antibody is disclosed that binds to the same epitope as an antibody described in Table 2. As demonstrated by the working examples below, all the anti-C5 antibodies described in Table 2 are grouped into the same epitope bin of C5 and exhibit pH-dependent binding characteristics.

In an additional aspect, the disclosure provides an antibody that binds to the same epitope as an antibody provided herein. In a further aspect, the disclosure provides an antibody that binds to the same epitope as an antibody described in Tables 7 or 8. In certain embodiments, an antibody is disclosed that binds to an epitope within a fragment consisting of amino acids 33-124 of the beta chain of C5 (SEQ ID NO: 40). In certain embodiments, an antibody is disclosed that binds to an epitope within the beta chain of C5 (SEQ ID NO: 40) which comprises at least one fragment selected from the group consisting of amino acids 47-57, 70-76, and 107-110. In certain embodiments, an antibody is disclosed that binds to an epitope within a fragment of the beta chain of C5 (SEQ ID NO: 40) which comprises at least one amino acid selected from the group consisting of Thr47, Glu48, Ala49, Phe50, Asp51, Ala52, Thr53, Lys57, His70, Val71, His72, Ser74, Glu76, Val107, Ser108, Lys109, and His110. In another embodiment, an epitope of an anti-C5 antibody of the present disclosure is a conformational epitope.

In a further aspect of the disclosure, an anti-C5 antibody according to any of the above embodiments is a monoclonal antibody, including a chimeric, humanized or human antibody. In one embodiment, an anti-C5 antibody is an antibody fragment, e.g., a Fv, Fab, Fab', scFv, diabody, or F(ab')₂ fragment. In another embodiment, the antibody is a full length IgG1 antibody.

In a further aspect, an anti-C5 antibody according to any of the above embodiments may incorporate any of the features, singly or in combination, as described in Sections 1-7 below:

### 1. Antibody Affinity

In certain embodiments, an antibody disclosed herein has a dissociation constant (Kd) of 1 micro M or less, 100 nM or less, 10 nM or less, 1 nM or less, 0.1 nM or less, 0.01 nM or less, or 0.001 nM or less (e.g., 10⁻⁸ M or less, e.g., from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 micro g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 micro l/well of scintillant (MICROSCINT-20^{™}; Packard) is added, and the plates are counted on a TOPCOUNT ^{™} gamma counter (Packard) for ten minutes. Concentrations of each Fab that give less than or equal to 20% of maximal binding are chosen for use in competitive binding assays.

According to another embodiment, Kd is measured using a BIACORE (registered trademark) surface plasmon resonance assay. For example, an assay using a BIACORE (registered trademark)-2000 or a BIACORE (registered trademark)-3000 (BIACORE (registered trademark), Inc., Piscataway, NJ) is performed at 25 degrees C with immobilized antigen CM5 chips at ~10 response units (RU). In one embodiment, carboxymethylated dextran biosensor chips (CM5, BIACORE (registered trademark), Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 micro g/ml (~0.2 micro M) before injection at a flow rate of 5 micro l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25 degrees C at a flow rate of approximately 25 micro l/minute. Association rates (kₒₙ) and dissociation rates (k_{off}) are calculated using a simple one-to-one Langmuir binding model (BIACORE (registered trademark) Evaluation Software version 3.2) by simultaneously fitting the association and dissociation sensorgrams. The equilibrium dissociation constant (Kd) is calculated as the ratio k_{off}/kₒₙ. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 degrees C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO^{™} spectrophotometer (ThermoSpectronic) with a stirred cuvette.

### 2. Antibody Fragments

In certain embodiments, an antibody disclosed herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al., Nat. Med. 9:129-134 (2003). For a review of scFv fragments, see, e.g., Pluckthun, in The Pharmacology of Monoclonal Antibodies, vol. 113, Rosenburg and Moore eds., (Springer-Verlag, New York), pp. 269-315 (1994); see also WO 93/16185; and US Patent Nos. 5,571,894 and 5,587,458. For discussion of Fab and F(ab')₂ fragments comprising salvage receptor binding epitope residues and having increased in vivo half-life, see US Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90:6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., US Patent No. 6,248,516 B1).

Antibody fragments can be made by various techniques, including but not limited to proteolytic digestion of an intact antibody as well as production by recombinant host cells (e.g., E. coli or phage), as described herein.

### 3. Chimeric and Humanized Antibodies

In certain embodiments, an antibody disclosed herein is a chimeric antibody. Certain chimeric antibodies are described, e.g., in US Patent No. 4,816,567; and Morrison et al., Proc. Natl. Acad. Sci. USA 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5,821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" approach to FR shuffling).

Human framework regions that may be used for humanization include but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA 89:4285 (1992); and Presta et al., J. Immunol. 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody disclosed herein is a human antibody. Human antibodies can be produced using various techniques known in the art. Human antibodies are described generally in van Dijk and van de Winkel, Curr. Opin. Pharma. 5:368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., US Patent Nos. 6,075,181 and 6,150,584 describing XENOMOUSE^{™} technology; US Patent No. 5,770,429 describing HUMAB (registered trademark) technology; US Patent No. 7,041,870 describing K-M MOUSE (registered trademark) technology, and US Patent Application Publication No. US 2007/0061900, describing VELOCIMOUSE (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor, J. Immunol. 133:3001 (1984); Brodeur et al., Monoclonal Antibody Production Techniques and Applications, pp. 51-63 (Dekker, Inc., New York, 1987); and Boerner et al., J. Immunol. 147:86 (1991).) Human antibodies generated via human B-cell hybridoma technology are also described in Li et al., Proc. Natl. Acad. Sci. USA 103:3557-3562 (2006). Additional methods include those described, for example, in US Patent No. 7,189,826 (describing production of monoclonal human IgM antibodies from hybridoma cell lines) and Ni, Xiandai Mianyixue 26(4):265-268 (2006) (describing human-human hybridomas). Human hybridoma technology (Trioma technology) is also described in Vollmers, Histology and Histopathology 20(3):927-937 (2005) and Vollmers, Methods and Findings in Experimental and Clinical Pharmacology 27(3):185-191 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5, Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al., Methods in Molecular Biology 178:1-37 (O'Brien et al., ed., Human Press, Totowa, NJ, 2001) and further described, e.g., in McCafferty et al., Nature 348:552-554; Clackson et al., Nature 352:624-628 (1991); Marks et al., J. Mol. Biol. 222:581-597 (1992); Marks, Meth.Mol. Biol. 248:161-175 (Lo, ed., Human Press, Totowa, NJ, 2003); Sidhu et al., J. Mol. Biol. 338(2):299-310 (2004); Lee et al., J. Mol. Biol. 340(5):1073-1093 (2004); Fellouse, Proc. Natl. Acad. Sci. USA 101(34):12467-12472 (2004); Lee et al., J. Immunol. Methods 284(1-2):119-132 (2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol. 12:433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12:725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom, J. Mol. Biol. 227:381-388 (1992). Patent publications describing human antibody phage libraries include, for example: US Patent No. 5,750,373, and US Publ.Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody disclosed herein is a multispecific antibody, e.g., a bispecific antibody. Multispecific antibodies are monoclonal antibodies that have binding specificities for at least two different sites. In certain embodiments, one of the binding specificities is for C5 and the other is for any other antigen. In certain embodiments, bispecific antibodies may bind to two different epitopes of C5. Bispecific antibodies may also be used to localize cytotoxic agents to cells which express C5. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305:537 (1983)), WO 93/08829, and Traunecker et al., EMBO J. 10:3655 (1991)), and "knob-in-hole" engineering (see, e.g., US Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science 229:81 (1985)); using leucine zippers to produce bi-specific antibodies (see, e.g., Kostelny et al., J. Immunol. 148(5):1547-1553 (1992)); using "diabody" technology for making bispecific antibody fragments (see, e.g., Hollinger et al., Proc. Natl. Acad. Sci. USA 90:6444-6448 (1993)); and using single-chain Fv (scFv) dimers (see, e.g., Gruber et al., J. Immunol. 152:5368 (1994)); and preparing trispecific antibodies as described, e.g., in Tutt et al., J. Immunol. 147:60 (1991).

Engineered antibodies with three or more functional antigen binding sites, including "Octopus antibodies," are also included herein (see, e.g., US 2006/0025576).

The antibody or fragment herein also includes a "Dual Acting FAb" or "DAF" comprising an antigen binding site that binds to C5 as well as another, different antigen (see, US 2008/0069820, for example).

### 7. Antibody Variants

In certain embodiments, amino acid sequence variants of the antibodies disclosed herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a. Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**[Table 1]**

| **Original Residue** | **Exemplary Substitutions** | **Preferred Substitutions** |
|---|---|---|
| Ala (A) | Val; Leu; Ile | Val |
| Arg (R) | Lys; Gin; Asn | Lys |
| Asn (N) | Gln; His; Asp, Lys; Arg | Gln |
| Asp (D) | Glu; Asn | Glu |
| Cys (C) | Ser; Ala | Ser |
| Gin (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe.

Non-conservative substitutions will entail exchanging a member of one of these classes for another class.

One type of substitutional variant involves substituting one or more hypervariable region residues of a parent antibody (e.g., a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g., binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al., in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotidedirected mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham, Science 244:1081-1085 (1989). In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the Nor C-terminus of the antibody to an enzyme (e.g., for ADEPT) or a polypeptide which increases the serum half-life of the antibody.

### b. Glycosylation variants

In certain embodiments, an antibody disclosed herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al., TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are disclosed having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki et al., J. Mol. Biol. 336:1239-1249 (2004); Yamane-Ohnuki et al., Biotech. Bioeng. 87:614 (2004). Examples of cell lines capable of producing defucosylated antibodies include Lec13 CHO cells deficient in protein fucosylation (Ripka et al., Arch. Biochem. Biophys. 249:533-545 (1986); US 2003/0157108, Presta, L; and WO 2004/056312, Adams et al., especially at Example 11), and knockout cell lines, such as alpha-1,6-fucosyltransferase gene, FUT8, knockout CHO cells (see, e.g., Yamane-Ohnuki et al., Biotech. Bioeng. 87:614 (2004); Kanda et al., Biotechnol. Bioeng. 94(4):680-688 (2006); and WO2003/085107).

Antibodies variants are further disclosed with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c. Fc region variants

In certain embodiments, one or more amino acid modifications may be introduced into the Fc region of an antibody disclosed herein, thereby generating an Fc region variant. The Fc region variant may comprise a human Fc region sequence (e.g., a human IgG1, IgG2, IgG3 or IgG4 Fc region) comprising an amino acid modification (e.g., a substitution) at one or more amino acid positions.

In certain embodiments, the disclosure contemplates an antibody variant that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in US Patent No. 5,500,362 (see, e.g., Hellstrom et al., Proc. Nat'l Acad. Sci. USA 83:7059-7063 (1986)) and Hellstrom et al., Proc. Nat'l Acad. Sci. USA 82:1499-1502 (1985); US Pat. No. 5,821,337 (see Bruggemann et al., J. Exp. Med. 166:1351-1361 (1987)). Alternatively, non-radioactive assays methods may be employed (see, for example, ACT1^{™} non-radioactive cytotoxicity assay for flow cytometry (CellTechnology, Inc. Mountain View, CA); and CytoTox 96 (registered trademark) non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al., Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg et al., Blood 101:1045-1052 (2003); and Cragg et al., Blood 103:2738-2743 (2004)). FcRn binding and in vivo clearance/half life determinations can also be performed using methods known in the art (see, e.g., Petkova et al., Int'l. Immunol. 18(12): 1759-1769 (2006)).

Antibodies with reduced effector function include those with substitution of one or more of Fc region residues 238, 265, 269, 270, 297, 327 and 329 (US Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., US Patent No. 6,737,056; WO 2004/056312, and Shields et al., J. Biol. Chem. 9(2):6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either improved or diminished) C1q binding and/or Complement Dependent Cytotoxicity (CDC), e.g., as described in US Patent No. 6,194,551, WO 1999/51642, and Idusogie et al., J. Immunol. 164:4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, Nature 322:738-40 (1988); US Patent No. 5,648,260; US Patent No. 5,624,821; and WO 1994/29351 concerning other examples of Fc region variants.

### d. Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in US Patent No. 7,521,541.

### e. Antibody Derivatives

In certain embodiments, an antibody disclosed herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102:11600-11605 (2005)). The radiation may be of any wavelength, and includes, but is not limited to, wavelengths that do not harm ordinary cells, but which heat the nonproteinaceous moiety to a temperature at which cells proximal to the antibodynonproteinaceous moiety are killed.

### B. Recombinant Methods and Compositions

Antibodies may be produced using recombinant methods and compositions, e.g., as described in US Patent No. 4,816,567. In one embodiment, isolated nucleic acid encoding an anti-C5 antibody comprising a VH sequence of SEQ ID NO: 106 and a VL sequence of SEQ ID NO: 111 described herein is provided. In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VH sequence of SEQ ID NO: 106 and a an amino acid sequence comprising the VL sequence of SEQ ID NO: 111, or (2) a first vector comprising a nucleic acid that encodes an amino acid sequence comprising the VH sequence of SEQ ID NO: 106and a second vector comprising a nucleic acid that encodes an amino acid sequence comprising the VL sequence of SEQ ID NO: 111. In one embodiment, the host cell is eukaryotic, e.g., a Chinese Hamster Ovary (CHO) cell or lymphoid cell (e.g., Y0, NS0, Sp20 cell). In one embodiment, a method of making an anti-C5 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-C5 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals (usually non-human mammals) are immunized against the antigen, immunogenic conjugates, or derivatives by combining, e.g., 100 micro g or 5 micro g of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies are obtained from a population of substantially homogeneous antibodies, i.e., the individual antibodies comprising the population are identical except for possible naturally occurring mutations and/or post-translational modifications (e.g., isomerizations, amidations) that may be present in minor amounts. Thus, the modifier "monoclonal" indicates the character of the antibody as not being a mixture of discrete antibodies.

For example, the monoclonal antibodies may be made using the hybridoma method first described by Kohler et al. Nature 256(5517):495-497 (1975). In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized in vitro.

The immunizing agent will typically include the antigenic protein or a fusion variant thereof. Generally either peripheral blood lymphocytes (PBLs) are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press (1986), pp. 59-103).

Immortalized cell lines are usually transformed mammalian cells, particularly myeloma cells of rodent, bovine and human origin. Usually, rat or mouse myeloma cell lines are employed. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which are substances that prevent the growth of HGPRT-deficient cells.

Preferred immortalized myeloma cells are those that fuse efficiently, support stable high-level production of antibody by the selected antibody-producing cells, and are sensitive to a medium such as HAT medium. Among these, preferred are murine myeloma lines, such as those derived from MOPC-21 and MPC-11 mouse tumors available from the Salk Institute Cell Distribution Center, San Diego, California USA, and SP-2 cells (and derivatives thereof, e.g., X63-Ag8-653) available from the American Type Culture Collection, Manassas, Virginia USA. Human myeloma and mouse-human heteromyeloma cell lines also have been described for the production of human monoclonal antibodies (Kozbor et al., J Immunol. 133(6):3001-3005 (1984); Brodeur et al., Monoclonal Antibody Production Techniques and Applications, Marcel Dekker, Inc., New York (1987), pp. 51-63).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunosorbent assay (ELISA). Such techniques and assays are known in the art. For example, binding affinity may be determined by the Scatchard analysis of Munson, Anal Biochem. 107(1):220-239 (1980).

After hybridoma cells are identified that produce antibodies of the desired specificity, affinity, and/or activity, the clones may be subcloned by limiting dilution procedures and grown by standard methods (Goding, supra). Suitable culture media for this purpose include, for example, D-MEM or RPMI-1640 medium. In addition, the hybridoma cells may be grown in vivo as tumors in a mammal.

The monoclonal antibodies secreted by the subclones are suitably separated from the culture medium, ascites fluid, or serum by conventional immunoglobulin purification procedures such as, for example, protein A-Sepharose, hydroxyapatite chromatography, gel electrophoresis, dialysis, or affinity chromatography.

Antibodies may be produced by immunizing an appropriate host animal against an antigen. In one embodiment, the antigen is a polypeptide comprising a full-length C5. In one embodiment, the antigen is a polypeptide comprising the beta chain (SEQ ID NO: 40) of C5. In one embodiment, the antigen is a polypeptide comprising the MG1-MG2 domain (SEQ ID NO: 43) of the beta chain of C5. In one embodiment, the antigen is a polypeptide comprising the MG1 domain (SEQ ID NO: 41) of the beta chain of C5. In one embodiment, the antigen is a polypeptide comprising the region corresponding to the amino acids at positions 19 to 180 of the beta chain of C5. In one embodiment, the antigen is a polypeptide comprising the region corresponding to the amino acids at positions 33 to 124 of the beta chain of C5. In one embodiment, the antigen is a polypeptide comprising at least one fragment selected from amino acids 47-57, 70-76, and 107-110 of the beta chain (SEQ ID NO: 40) of C5. In one embodiment, the antigen is a polypeptide comprising a fragment of the beta chain of C5 which comprises at least one amino acid selected from the group consisting of Thr47, Glu48, Ala49, Phe50, Asp51, Ala52, Thr53, Lys57, His70, Val71, His72, Ser74, Glu76, Val107, Ser108, Lys109, and His110. In one embodiment, the antigen is a polypeptide comprising a fragment of the beta chain of C5 which comprises at least one amino acid selected from the group consisting of Glu48, Asp51, His70, His72, Lys109, and His110. Also included in the present invention are antibodies produced by immunizing an animal against the antigen. The antibodies may incorporate any of the features, singly or in combination, as described in "Exemplary Anti-C5 Antibodies" above.

### C. Assays

Anti-C5 antibodies provided herein may be identified, screened for, or characterized for their physical/chemical properties and/or biological activities by various assays known in the art.

### 1. Binding assays and other assays

In one aspect, an antibody of the invention is tested for its antigen binding activity, e.g., by known methods such as ELISA, Western blot, BIACORE (registered trademark), etc.

In another aspect, competition assays may be used to identify an antibody that competes for binding to C5 with an anti-C5 antibody described herein. In certain embodiments, when such a competing antibody is present in excess, it blocks (e.g., reduces) the binding of a reference antibody to C5 by at least 10%, 15%, 20%, 25%, 30%, 35%, 40%, 45%, 50%, 55%, 60%, 65%, 70%, 75%, or more. In some instances, binding is inhibited by at least 80%, 85%, 90%, 95%, or more. In certain embodiments, such a competing antibody binds to the same epitope (e.g., a linear or a conformational epitope) that is bound by an anti-C5 antibody described herein (e.g., an anti-C5 antibody described in Table 2). Detailed exemplary methods for mapping an epitope to which an antibody binds are provided in Morris, "Epitope Mapping Protocols," in Methods in Molecular Biology vol. 66 (Humana Press, Totowa, NJ) (1996).

In an exemplary competition assay, immobilized C5 is incubated in a solution comprising a first labeled (reference) antibody that binds to C5 and a second unlabeled antibody that is being tested for its ability to compete with the first antibody for binding to C5. The second antibody may be present in a hybridoma supernatant. As a control, immobilized C5 is incubated in a solution comprising the first labeled antibody but not the second unlabeled antibody. After incubation under conditions permissive for binding of the first antibody to C5, excess unbound antibody is removed, and the amount of label associated with immobilized C5 is measured. If the amount of label associated with immobilized C5 is substantially reduced in the test sample relative to the control sample, then that indicates that the second antibody is competing with the first antibody for binding to C5. See, Harlow and Lane, Antibodies: A Laboratory Manual ch. 14 (Cold Spring Harbor Laboratory, Cold Spring Harbor, NY) (1988).

In another exemplary competition assay, BIACORE (registered trademark) analysis is used to determine the ability of a test anti-C5 antibody to compete with the binding to C5 by a second (reference) anti-C5 antibody. In a further aspect in which a BIACORE (registered trademark) instrument (for example, the BIACORE (registered trademark) 3000) is operated according to the manufacturer's recommendations, C5 protein is captured on a CM5 BIACORE (registered trademark) chip using a standard technique known in the art to generate a C5-coated surface. Typically 200-800 resonance units of C5 would be coupled to the chip (an amount that gives easily measurable levels of binding but that is readily saturable by the concentrations of test antibody being used). The two antibodies (i.e., the test and reference antibody) to be assessed for their ability to compete with each other are mixed at a 1:1 molar ratio of binding sites in a suitable buffer to create a test mixture. When calculating the concentrations on a binding site basis the molecular weight of an a test or reference antibody is assumed to be the total molecular weight of the corresponding antibody divided by the number of C5-binding sites on the antibody. The concentration of each antibody (i.e., test and reference antibody) in the test mixture should be high enough to readily saturate the binding sites for that antibody on the C5 molecules captured on the BIACORE (registered trademark) chip. The test and reference antibodies in the mixture are at the same molar concentration (on a binding basis), typically between 1.00 and 1.5 micromolar (on a binding site basis). Separate solutions containing the test antibody alone and the reference antibody alone are also prepared. Test antibody and reference antibody in these solutions should be in the same buffer and at the same concentration and conditions as in the test mixture. The test mixture containing the test antibody and reference antibody is passed over the C5-coated BIACORE (registered trademark) chip and the total amount of binding is recorded. The chip is then treated in such a way as to remove the bound test or reference antibody without damaging the chip-bound C5. Typically, this is done by treating the chip with 30 mM HC1 for 60 seconds. The solution of test antibody alone is then passed over the C5-coated surface and the amount of binding recorded. The chip is again treated to remove all of the bound antibody without damaging the chip-bound C5. The solution of reference antibody alone is then passed over the C5-coated surface and the amount of binding recorded. The maximum theoretical binding of the mixture of test antibody and reference antibody is next calculated, and is the sum of the binding of each antibody (i.e. test and reference) when passed over the C5 surface alone. If the actual recorded binding of the mixture is less than this theoretical maximum then test antibody and reference antibody are competing with each other for binding C5. Thus, in general, a competing test anti-C5 antibody is one which will bind to C5 in the above BIACORE (registered trademark) blocking assay such that during the assay and in the presence of the reference anti-C5 antibody the recorded binding is between 80% and 0.1% (e.g., 80% > to 4%) of the maximum theoretical binding, specifically between 75% and 0.1 % (e.g., 75% to 4%) of the maximum theoretical binding, and more specifically between 70% and 0.1% (e.g., 70% to 4%) of maximum theoretical binding (as defined above) of the test antibody and reference antibody in combination.

In certain embodiments, an anti-C5 antibody of the present disclosure competes for binding C5 with an antibody comprising a VH and VL pair selected from antibody CFA0341 and CFA0330. In some embodiments, an anti-C5 antibody competes for binding C5 with an antibody selected from: CFA0538, CFA0501, CFA0599, CFA0307, CFA0366, CFA0675, and CFA0672. In some embodiments, an anti-C5 antibody competes for binding C5 with antibody CFA0329. In some embodiments, an anti-C5 antibody competes for binding C5 with antibody CFA0666.

In certain embodiments, an anti-C5 antibody of the present disclosure competes for binding C5 with an antibody comprising a VH and VL pair of antibody CFA0305 or 305LO5.

In further embodiments, the anti-C5 antibody binds to C5 with a higher affinity at neutral pH than at acidic pH. In certain embodiments, an anti-C5 antibody of the present disclosure competes for binding C5 with an antibody comprising a VH and VL pair selected from: CFA0538, CFA0501, CFA0599, CFA0307, CFA0366, CFA0675, and CFA0672. In some embodiments, an anti-C5 antibody competes for binding C5 with antibody CFA0666. In further embodiments, the anti-C5 antibody binds to C5 with a higher affinity at pH7.4 than at pH5.8.

In further embodiments, the anti-C5 antibody binds to C5 with a higher affinity at neutral pH than at acidic pH. In certain embodiments, an anti-C5 antibody of the present invention competes for binding C5 with an antibody comprising a VH and VL pair of antibody CFA0305 or 305LO5. In further embodiments, the anti-C5 antibody binds to C5 with a higher affinity at pH7.4 than at pH5.8.

In certain embodiments, an anti-C5 antibody of the present disclosure competes for binding C5 with an antibody comprising a VH and VL pair selected from a VH of SEQ ID NO:22 and a VL of SEQ ID NO:26, or a VH of SEQ ID NO:21 and a VL of SEQ ID NO:25. In some embodiments, an anti-C5 antibody competes for binding C5 with an antibody comprising a VH and VL pair selected from: (a) a VH of SEQ ID NO: 5 and a VL of SEQ ID NO: 15; (b) a VH of SEQ ID NO: 4 and a VL of SEQ ID NO: 14; (c) a VH of SEQ ID NO:6 and a VL of SEQ ID NO:16; (d) aVH of SEQ ID NO:2 and a VL of SEQ ID NO:12; (e) a VH of SEQ ID NO: 3 and a VL of SEQ ID NO: 13; (f) a VH of SEQ ID NO: 1 and a VL of SEQ ID NO: 11; (g) a VH of SEQ ID NO:9 and a VL of SEQ ID NO:19; (h) aVH of SEQ ID NO:7 and a VL of SEQ ID NO:17; and (i) aVH of SEQ ID NO:8 and a VL of SEQ ID NO:18. In some embodiments, an anti-C5 antibody competes for binding C5 with antibody comprising a VH of SEQ ID NO:23 and a VL of SEQ ID NO:27. In some embodiments, an anti-C5 antibody competes for binding C5 with antibody comprising a VH of SEQ ID NO:7 and a VL of SEQ ID NO:17.

In certain embodiments, an anti-C5 antibody of the present disclosure competes for binding C5 with an antibody comprising a VH and VL pair selected from: (a) a VH of SEQ ID NO:1 and a VL of SEQ ID NO:11; (b) a VH of SEQ ID NO: 22 and a VL of SEQ ID NO:26; (c) a VH of SEQ ID NO:21 and a VL of SEQ ID NO:25; (d) a VH of SEQ ID NO: 5 and a VL of SEQ ID NO:15; (e) a VH of SEQ ID NO:4 and a VL of SEQ ID NO:14; (f) a VH of SEQ ID NO: 6 and a VL of SEQ ID NO: 16; (g) a VH of SEQ ID NO:2 and a VL of SEQ ID NO:12; (h) a VH of SEQ ID NO: 3 and a VL of SEQ ID NO: 13; (i) a VH of SEQ ID NO:9 and a VL of SEQ ID NO:19; (j) a VH of SEQ ID NO:7 and a VL of SEQ ID NO: 17; (k) aVH of SEQ ID NO:8 and a VL of SEQ ID NO:18; (l) a VH of SEQ ID NO: 23 and a VL of SEQ ID NO:27; and (m) a VH of SEQ ID NO:10 and a VL of SEQ ID NO:20.

In certain embodiments, an anti-C5 antibody of the present disclosure competes for binding C5 with an antibody comprising a VH and VL pair selected from: (a) a VH of SEQ ID NO: 22 and a VL of SEQ ID NO:26; (b) a VH of SEQ ID NO:21 and a VL of SEQ ID NO:25; (c) a VH of SEQ ID NO: 5 and a VL of SEQ ID NO:15; (d) a VH of SEQ ID NON and a VL of SEQ ID NO:14; (e) a VH of SEQ ID NO: 6 and a VL of SEQ ID NO: 16; (f) a VH of SEQ ID NO:2 and a VL of SEQ ID NO:12; (g) a VH of SEQ ID NO: 3 and a VL of SEQ ID NO: 13; (h) a VH of SEQ ID NO:9 and a VL of SEQ ID NO:19; (i) a VH of SEQ ID NO:7 and a VL of SEQ ID NO: 17; (j) aVH of SEQ ID NO:8 and a VL of SEQ ID NO:18; (k) a VH of SEQ ID NO: 23 and a VL of SEQ ID NO:27.

In certain embodiments, an anti-C5 antibody of the present disclosure competes for binding C5 with an antibody comprising a VH and VL pair selected from a VH of SEQ ID NO:1 and a VL of SEQ ID NO:11, or a VH of SEQ ID NO:10 and a VL of SEQ ID NO:20.

In further embodiments, the anti-C5 antibody binds to C5 with a higher affinity at neutral pH than at acidic pH. In certain embodiments, an anti-C5 antibody binds to C5 with a higher affinity at neutral pH than at acidic pH and competes for binding C5 with an antibody comprising a VH and VL pair selected from: (a) a VH of SEQ ID NO:1 and a VL of SEQ ID NO:11; (b) a VH of SEQ ID NO: 5 and a VL of SEQ ID NO:15; (c) a VH of SEQ ID NO:4 and a VL of SEQ ID NO:14; (d) a VH of SEQ ID NO: 6 and a VL of SEQ ID NO: 16; (e) a VH of SEQ ID NO:2 and a VL of SEQ ID NO:12; (f) a VH of SEQ ID NO: 3 and a VL of SEQ ID NO: 13; (g) a VH of SEQ ID NO:9 and a VL of SEQ ID NO:19; (h) a VH of SEQ ID NO:7 and a VL of SEQ ID NO: 17; (i) aVH of SEQ ID NO:8 and a VL of SEQ ID NO:18; and (j) a VH of SEQ ID NO:10 and a VL of SEQ ID NO:20. In further embodiments, the anti-C5 antibody binds to C5 with a higher affinity at pH7.4 than at pH5.8.

In some embodiments, the anti-C5 antibody binds to C5 with a higher affinity at neutral pH than at acidic pH and competes for binding C5 with an antibody comprising a VH and VL pair selected from: (a) a VH of SEQ ID NO: 5 and a VL of SEQ ID NO: 15; (b) a VH of SEQ ID NO: 4 and a VL of SEQ ID NO: 14; (c) a VH of SEQ ID NO:6 and a VL of SEQ ID NO:16; (d) aVH of SEQ ID NO:2 and a VL of SEQ ID NO:12; (e) a VH of SEQ ID NO: 3 and a VL of SEQ ID NO: 13; (f) a VH of SEQ ID NO: 1 and a VL of SEQ ID NO: 11; (g) a VH of SEQ ID NO:9 and a VL of SEQ ID NO:19; (h) aVH of SEQ ID NO: 7 and a VL of SEQ ID NO:17; and (i) aVH of SEQ ID NO:8 and a VL of SEQ ID NO:18. In further embodiments, the anti-C5 antibody binds to C5 with a higher affinity at pH7.4 than at pH5.8.

In some embodiments, the anti-C5 antibody binds to C5 with a higher affinity at neutral pH than at acidic pH and competes for binding C5 with an antibody comprising a VH and VL pair selected from a VH of SEQ ID NO:1 and a VL of SEQ ID NO:11, or a VH of SEQ ID NO:10 and a VL of SEQ ID NO:20. In further embodiments, the anti-C5 antibody binds to C5 with a higher affinity at pH7.4 than at pH5.8.

In certain embodiments, whether an anti-C5 antibody of the present disclosure binds to a certain epitope can be determined as follows: C5 point mutants in which an amino acid (except for alanine) on C5 is substituted with alanine are expressed in 293 cells, and binding of an anti-C5 antibody to the C5 mutants is tested via ELISA, Western blot or BIACORE (registered trademark); wherein a substantial reduction or elimination of binding of the anti-C5 antibody to the C5 mutant relative to its binding to wild type C5 indicates that the anti-C5 antibody binds to an epitope comprising that amino acid on C5. In certain embodiments, the amino acid on C5 to be substituted with alanine is selected from the group consisting of Glu48, Asp51, His70, His72, Lys109, and His110 of the beta chain of C5 (SEQ ID NO:40). In further embodiments, the amino acid on C5 to be substituted with alanine is Asp51 or Lys109 of the beta chain of C5 (SEQ ID NO:40).

In another embodiment, whether an anti-C5 antibody with pH-dependent binding characteristics binds to a certain epitope can be determined as follows: C5 point mutants in which a histidine residue on C5 is substituted with another amino acid (e.g., tyrosine) are expressed in 293 cells, and binding of an anti-C5 antibody to the C5 mutants is tested via ELISA, Western blot or BIACORE (registered trademark); wherein a substantial reduction of binding of the anti-C5 antibody to wild type C5 at acidic pH relative to its binding to the C5 mutant at acidic pH, indicates that the anti-C5 antibody binds to an epitope comprising that histidine residue on C5. In further embodiments, binding of the anti-C5 antibody to wild type C5 at neutral pH is not substantially reduced relative to its binding to the C5 mutant at neutral pH. In certain embodiments, the histidine residue on C5 to be substituted with another amino acid is selected from the group consisting of His70, His72, and His110 of the beta chain of C5 (SEQ ID NO:40). In a further embodiment, the histidine residue His70 is substituted with tyrosine.

### 2. Activity assays

In one aspect, assays are disclosed for identifying anti-C5 antibodies thereof having biological activity. Biological activity may include, e.g., inhibiting the activation of C5, preventing the cleavage of C5 to form C5a and C5b, blocking the access of C5 convertase to the cleavage site on C5, blocking hemolytic activity caused by the activation of C5, etc. Antibodies having such biological activity in vivo and/or in vitro are also provided.

In certain embodiments, an antibody of the disclosure is tested for such biological activity.

In certain embodiments, whether a test antibody inhibits the cleavage of C5 into C5a and C5b, is determined by methods described in, e.g., Isenman et al., J Immunol. 124(1):326-331 (1980). In another embodiment, this is determined by methods for specific detection of cleaved C5a and/or C5b proteins, e.g., ELISAs or Western blots. Where a decreased amount of a cleavage product of C5 (i.e., C5a and/or C5b) is detected in the presence of (or following contact with) the test antibody, the test antibody is identified as an antibody that can inhibit the cleavage of C5. In certain embodiments, the concentration and/or physiologic activity of C5a can be measured by methods, e.g., chemotaxis assays, RIAs, or ELISAs (See, e.g., Ward and Zvaifler J. Clin. Invest. 50(3):606-616 (1971)).

In certain embodiments, whether a test antibody blocks the access of C5 convertase to C5 is determined by methods for the detection of protein interactions between the C5 convertase and C5, e.g., ELISAs or BIACORE (registered trademark). Where the interactions are decreased in the presence of (or following contact with) the test antibody, the test antibody is identified as an antibody that can block the access of C5 convertase to C5.

In certain embodiments, C5 activity can be measured as a function of its cell-lysing ability in a subject's body fluids. The cell-lysing ability, or a reduction thereof, of C5 can be measured by methods well known in the art, for example, a conventional hemolytic assay, such as the hemolysis assay described by Kabat and Mayer (eds), Experimental Immunochemistry, 2nd Edition, 135-240, Springfield, IL, CC Thomas (1961), pages 135-139, or a conventional variation of that assay, such as the chicken erythrocyte hemolysis method as described in, e.g., Hillmen et al., N. Engl. J. Med. 350(6): 552-559 (2004). In certain embodiments, C5 activity, or inhibition thereof, is quantified using a CH50eq assay. The CH50eq assay is a method for measuring the total classical complement activity in serum. This test is a lytic assay, which uses antibodysensitized erythrocytes as the activator of the classical complement pathway, and various dilutions of the test serum to determine the amount required to give 50% lysis (CH50). The percentage of hemolysis can be determined, for example, using a spectrophotometer. The CH50eq assay provides an indirect measure of terminal complement complex (TCC) formation, since the TCC themselves are directly responsible for the hemolysis measured. Inhibition of C5 activation can also be detected and/or measured using the methods set forth and exemplified in the working examples. Using assays of these or other suitable types, candidate antibodies capable of inhibiting the activation of C5 can be screened. In certain embodiments, inhibition of C5 activation includes at least a 5%, 10%, 15%, 20%, 25%, 30%, 35%, or 40% or greater decrease in the C5 activation in an assay as compared to the effect of a negative control under similar conditions. In some embodiments, it refers to inhibition of C5 activation by at least 45%, 50%, 55%, 60%, 65%, 70%, 75%, 80%, 85%, 90%, or 95% or greater.

### D. Immunoconjugates

The disclosure also provides immunoconjugates comprising an anti-C5 antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see, US Patent Nos. 5,208,020, 5,416,064 and European Patent EP 0 425 235 B1); an auristatin such as monomethylauristatin drug moieties DE and DF (MMAE and MMAF) (see, US Patent Nos. 5,635,483 and 5,780,588, and 7,498,298); a dolastatin; a calicheamicin or derivative thereof (see, US Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and US Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example tc99m or I123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); US Patent No. 5,208,020) may be used.

The immunoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SlAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain embodiments, any of the anti-C5 antibodies provided herein is useful for detecting the presence of C5 in a biological sample. The term "detecting" as used herein encompasses quantitative or qualitative detection. In certain embodiments, a biological sample comprises a cell or tissue, such as serum, whole blood, plasma, biopsy sample, tissue sample, cell suspension, saliva, sputum, oral fluid, cerebrospinal fluid, amniotic fluid, ascites fluid, milk, colostrums, mammary gland secretion, lymph, urine, sweat, lacrimal fluid, gastric fluid, synovial fluid, peritoneal fluid, ocular lens fluid and mucus.

In one embodiment, an anti-C5 antibody for use in a method of diagnosis or detection is disclosed. In a further aspect, a method of detecting the presence of C5 in a biological sample is provided. In certain embodiments, the method comprises contacting the biological sample with an anti-C5 antibody as described herein under conditions permissive for binding of the anti-C5 antibody to C5, and detecting whether a complex is formed between the anti-C5 antibody and C5. Such method may be an in vitro method. In one embodiment, an anti-C5 antibody is for use in a method of selecting subjects eligible for therapy with an anti-C5 antibody, e.g., where C5 is a biomarker for selection of patients.

In another embodiment, an anti-C5 antibody of the present invention for use in a method of selecting an individual having a complement-mediated disease or condition which involves excessive or uncontrolled activation of C5 as suitable for a therapy is provided. In certain embodiments, an anti-C5 antibody of the present invention is for use in a method comprising (a) detecting a genetic variation in C5 derived from the individual, and (b) selecting the individual as suitable for the therapy when the genetic variation is detected in C5 derived from the individual. In another embodiment, an anti-C5 antibody of the present invention is for use in a method of selecting a therapy for an individual having a complement-mediated disease or condition which involves excessive or uncontrolled activation of C5 is provided. In certain embodiments, an anti-C5 antibody of the present invention is for use in a method comprising (a) detecting a genetic variation in C5 derived from the individual, and (b) selecting a therapy the individual when the genetic variation is detected in C5 derived from the individual.

In another embodiment, an anti-C5 antibody of the present invention is for use in a method of treating an individual having a complement-mediated disease or condition which involves excessive or uncontrolled activation of C5 is provided. In certain embodiments, an anti-C5 antibody of the present invention is for use in a method comprising (a) detecting a genetic variation in C5 derived from the individual, (b) selecting the individual as suitable for the therapy when the genetic variation is detected in C5 derived from the individual, and (c) providing the anti-C5 antibody to be administered to the individual.

In another embodiment, an anti-C5 antibody of the present invention for use in a method of treating an individual having a complement-mediated disease or condition which involves excessive or uncontrolled activation of C5 is disclosed. In certain embodiments, an anti-C5 antibody of the present invention is for use in a method of treating the individual when the genetic variation is detected in C5 derived from the individual.

In another embodiment, in vitro use of a genetic variation in C5 for selecting an individual having a complement-mediated disease or condition which involves excessive or uncontrolled activation of C5 as suitable for a therapy comprising an anti-C5 antibody of the present invention is provided. In certain embodiments, the individual is selected as being suitable for the therapy when the genetic variation is detected in C5 derived from the individual. In another embodiment, in vitro use of a genetic variation in C5 for selecting a therapy for an individual having a complement-mediated disease or condition which involves excessive or uncontrolled activation of C5 is provided. In certain embodiments, a therapy comprising an anti-C5 antibody of the present invention is selected for the individual when the genetic variation is detected in C5 derived from the individual.

It has been reported that some patients who have genetic variation in C5 show poor response to a therapy comprising an existing anti-C5 antibody (Nishimura et al., N. Engl. J. Med. 370:632-639 (2014)). It is recommended that such a patient be treated with a therapy comprising an anti-C5 antibody of the present invention, because such an antibody has an inhibitory activity on the activation of C5 variants as well as wild type C5, as demonstrated in the working examples below.

Detection of a genetic variation in C5 can be carried out by using a method known in the prior art. Such a method may include sequencing, PCR, RT-PCR, and a hybridization-based method such as southern blot or northern blot, but is not limited thereto. C5 variants may comprise at least one genetic variation. The genetic variation may be selected from a group consisting of V145I, R449G, V802I, R885H, R928Q, D966Y, S1310N, and E1437D. Herein, R885H, for example, means a genetic variation where arginine at position 885 is substituted by histidine. In certain embodiments, a C5 variant has biological activity similar to wild type C5.

Exemplary disorders that may be diagnosed using an antibody of the invention include rheumatoid arthritis (RA); systemic lupus erythematosus (SLE); lupus nephritis; ischemia reperfusion injury (IRI); asthma; paroxysmal nocturnal hemoglobinuria (PNH); hemolytic uremic syndrome (HUS) (e.g., atypical hemolytic uremic syndrome (aHUS)); dense deposit disease (DDD); neuromyelitis optica (NMO); multifocal motor neuropathy (MMN); multiple sclerosis (MS); systemic sclerosis; macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; epidermolysis bullosa; recurrent fetal loss; pre-eclampsia; traumatic brain injury; myasthenia gravis; cold agglutinin disease; Sjogren's syndrome; dermatomyositis; bullous pemphigoid; phototoxic reactions; Shiga toxin E. coli-related hemolytic uremic syndrome; typical or infectious hemolytic uremic syndrome (tHUS); C3 Glomerulonephritis; Antineutrophil cytoplasmic antibody (ANCA)-associated vasculitis; humoral and vascular transplant rejection; acute antibody mediated rejection (AMR); graft dysfunction; myocardial infarction; an allogenic transplant; sepsis; coronary artery disease; hereditary angioedema; dermatomyositis; Graves' disease; atherosclerosis; Alzheimer's disease (AD); Huntington's disease; Creutzfeld-Jacob disease; Parkinson's disease; cancers; wounds; septic shock; spinal cord injury; uveitis; diabetic ocular diseases; retinopathy of prematurity; glomerulonephritis; membranous nephritis; immunoglobulin A nephropathy; adult respiratory distress syndrome (ARDS); chronic obstructive pulmonary disease (COPD); cystic fibrosis; hemolytic anemia; paroxysmal cold hemoglobinuria; anaphylactic shock; allergy; osteoporosis; osteoarthritis; Hashimoto's thyroiditis; type I diabetes; psoriasis; pemphigus; autoimmune hemolytic anemia (AIHA); idiopathic thrombocytopenic purpura (ITP); Goodpasture syndrome; Degos disease; antiphospholipid syndrome (APS); catastrophic APS (CAPS); a cardiovascular disorder; myocarditis; a cerebrovascular disorder; a peripheral vascular disorder; a renovascular disorder; a mesenteric/enteric vascular disorder; vasculitis; Henoch-Schonlein purpura nephritis; Takayasu's disease; dilated cardiomyopathy; diabetic angiopathy; Kawasaki's disease (arteritis); venous gas embolus (VGE), restenosis following stent placement; rotational atherectomy; membraneous nephropathy; Guillain-Barre syndrome (GBS); Fisher syndrome; antigeninduced arthritis; synovial inflammation; viral infections; bacterial infections; fungal infections; and injury resulting from myocardial infarction, cardiopulmonary bypass and hemodialysis.

In certain embodiments, labeled anti-C5 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (US Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### F. Pharmaceutical Formulations

Pharmaceutical formulations of an anti-C5 antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Publ. Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g., films, or microcapsules.

The formulations to be used for in vivo administration are generally sterile. Sterility may be readily accomplished, e.g., by filtration through sterile filtration membranes.

### G. Therapeutic Methods and Compositions

Any of the anti-C5 antibodies provided herein may be for use in therapeutic methods.

In one aspect, an anti-C5 antibody for use as a medicament is disclosed. In further aspects, an anti-C5 antibody for use in a method of treating a complement-mediated disease or condition which involves excessive or uncontrolled activation of C5 is disclosed. In certain embodiments, an anti-C5 antibody for use in a method of treatment is disclosed. In certain embodiments, the disclosure provides an anti-C5 antibody for use in a method of treating an individual having a complement-mediated disease or condition which involves excessive or uncontrolled activation of C5, wherein the anti-C5 antibody is to be administered to the individual in an effective amount. In one such embodiment, the method further comprises providing at least one additional therapeutic agent to be administered to the individual in an effective amount. An "individual" according to any of the above embodiments is preferably a human.

When the antigen is a soluble protein, the binding of an antibody to its antigen can result in an extended half-life of the antigen in plasma (i.e., reduced clearance of the antigen from plasma), since the antibody itself has a longer half-life in plasma and serves as a carrier for the antigen. This is due to the recycling of the antigen-antibody complex by FcRn through the endosomal pathway in cell (Roopenian, Nat. Rev. Immunol. 7(9):715-725 (2007)). However, an antibody with pH-dependent binding characteristics, which binds to its antigen in neutral extracellular environment while releasing it into acidic endosomal compartments following entry into cells, is expected to have superior properties in terms of antigen neutralization and clearance relative to its counterpart that binds in a pH-independent manner (Igawa et al., Nat. Biotech.. 28(11):1203-1207 (2010); Devanaboyina et al., mAbs 5(6):851-859 (2013); WO 2009/125825).

In further embodiments, the disclosure provides an anti-C5 antibody for use in a method of enhancing the clearance of C5 from plasma. In certain embodiments, the disclosure provides an anti-C5 antibody for use in a method of enhancing the clearance of C5 from plasma in an individual, wherein the anti-C5 antibody is to be administered to the individual in an effective amount of the to enhance the clearance of C5 from plasma. In one embodiment, an anti-C5 antibody enhances the clearance of C5 from plasma, compared to a conventional anti-C5 antibody which does not have pH-dependent binding characteristics. An "individual" according to any of the above embodiments is preferably a human.

In further embodiments, the disclosure provides an anti-C5 antibody for use in a method of suppressing the accumulation of C5 in plasma. In certain embodiments, the invention provides an anti-C5 antibody for use in a method of suppressing the accumulation of C5 in plasma in an individual, wherein the anti-C5 antibody is to be administered to the individual in an effective amount to suppress the accumulation of C5 in plasma. In one embodiment, the accumulation of C5 in plasma is the result of the formation of an antigen-antibody complex. In another embodiment, an anti-C5 antibody suppresses the accumulation of C5 in plasma, compared to a conventional anti-C5 antibody which does not have pH-dependent binding characteristics. An "individual" according to any of the above embodiments is preferably a human.

An anti-C5 antibody of the present disclosure may inhibit the activation of C5. In further embodiments, the disclosure provides an anti-C5 antibody for use in a method of inhibiting the activation of C5. In certain embodiments, the invention provides an anti-C5 antibody for use in a method of inhibiting the activation of C5 in an individual, wherein the anti-C5 antibody is to be administered to the individual in an effective amount to inhibit the activation of C5. In one embodiment, the cytotoxicity mediated by C5 is suppressed by inhibiting the activation of C5. An "individual" according to any of the above embodiments is preferably a human.

In a further aspect, the disclosure provides the use of an anti-C5 antibody in the manufacture or preparation of a medicament. In one embodiment, the medicament is for use in a method of treatment of a complement-mediated disease or condition which involves excessive or uncontrolled activation of C5. In a further embodiment, the medicament is for use in a method of treating a complement-mediated disease or condition which involves excessive or uncontrolled activation of C5, wherein the medicament is to be administered to an individual having a complement-mediated disease or condition which involves excessive or uncontrolled activation of C5 in an effective amount. In one such embodiment, the method further comprises providing at least one additional therapeutic agent to be administered to the individual in an effective amount. An "individual" according to any of the above embodiments is preferably a human.

In a further embodiment, the medicament is for use in a method of enhancing the clearance of C5 from plasma. In a further embodiment, the medicament is for use in a method of enhancing the clearance of C5 from plasma in an individual, wherein the medicament is to be administered to the individual in an effective amount to enhance the clearance of C5 from plasma. In one embodiment, an anti-C5 antibody enhances the clearance of C5 from plasma, compared to a conventional anti-C5 antibody which does not have pH-dependent binding characteristics. An "individual" according to any of the above embodiments may be a human.

In a further embodiment, the medicament is for use in a method of suppressing the accumulation of C5 in plasma. In a further embodiment, the medicament is for use in a method of suppressing the accumulation of C5 in plasma in an individual, wherein the medicament is to be administered to the individual in an effective amount to suppress the accumulation of C5 in plasma. In one embodiment, the accumulation of C5 in plasma is a result of the formation of an antigen-antibody complex. In another embodiment, an anti-C5 antibody suppresses the accumulation of C5 in plasma, compared to a conventional anti-C5 antibody which does not have pH-dependent binding characteristics. An "individual" according to any of the above embodiments may be a human.

An anti-C5 antibody of the present disclosure may inhibit the activation of C5. In a further embodiment, the medicament is for use in a method of inhibiting the activation of C5. In a further embodiment, the medicament is for use in a method of inhibiting the activation of C5 in an individual, wherein the medicament is to be administered to the individual in an effective amount to inhibit the activation of C5. In one embodiment, the cytotoxicity mediated by C5 is suppressed by inhibiting the activation of C5. An "individual" according to any of the above embodiments may be a human.

In a further aspect, the disclosure provides pharmaceutical formulations comprising any of the anti-C5 antibodies provided herein, e.g., for use in any of the above therapeutic methods. In one embodiment, a pharmaceutical formulation comprises any of the anti-C5 antibodies provided herein and a pharmaceutically acceptable carrier. In another embodiment, a pharmaceutical formulation comprises any of the anti-C5 antibodies provided herein and at least one additional therapeutic agent.

In a further aspect, the pharmaceutical formulation is for use in a method of treatment of a complement-mediated disease or condition which involves excessive or uncontrolled activation of C5. In a further embodiment, the pharmaceutical formulation is for use in a method of enhancing the clearance of C5 from plasma. In one embodiment, an anti-C5 antibody enhances the clearance of C5 from plasma, compared to a conventional anti-C5 antibody which does not have pH-dependent binding characteristics. In a further embodiment, the pharmaceutical formulation is for use in a method of suppressing the accumulation of C5 in plasma. In one embodiment, the accumulation of C5 in plasma is a result of the formation of an antigen-antibody complex. In another embodiment, an anti-C5 antibody suppresses the accumulation of C5 in plasma, compared to a conventional anti-C5 antibody which does not have pH-dependent binding characteristics. An anti-C5 antibody of the present invention may inhibit the activation of C5. In a further embodiment, the pharmaceutical formulation is for use in a method of inhibiting the activation of C5. In one embodiment, the cytotoxicity mediated by C5 is suppressed by inhibiting the activation of C5. In one embodiment, the pharmaceutical formulation is to be administered to an individual having a complement-mediated disease or condition which involves excessive or uncontrolled activation of C5. An "individual" according to any of the above embodiments is preferably a human.

In one aspect, an individual has wild type C5. In another aspect, an individual has a C5 variant. In certain embodiments, a C5 variant has biological activity similar to wild type C5. Such a C5 variant may comprise at least one variation selected from the group consisting of V145I, R449G, V802I, R885H, R928Q, D966Y, S1310N, and E1437D. Herein, R885H, for example, means a genetic variation where arginine at position 885 is substituted by histidine.

In a further aspect, the disclosure provides methods for preparing a medicament or a pharmaceutical formulation, comprising mixing any of the anti-C5 antibodies provided herein with a pharmaceutically acceptable carrier, e.g., for use in any of the above therapeutic methods. In one embodiment, the methods for preparing a medicament or a pharmaceutical formulation further comprise adding at least one additional therapeutic agent to the medicament or pharmaceutical formulation.

In certain embodiments, the complement-mediated disease or condition which involves excessive or uncontrolled activation of C5 is selected from the group consisting of rheumatoid arthritis (RA); systemic lupus erythematosus (SLE); lupus nephritis; ischemia reperfusion injury (IRI); asthma; paroxysmal nocturnal hemoglobinuria (PNH); hemolytic uremic syndrome (HUS) (e.g., atypical hemolytic uremic syndrome (aHUS)); dense deposit disease (DDD); neuromyelitis optica (NMO); multifocal motor neuropathy (MMN); multiple sclerosis (MS); systemic sclerosis; macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; epidermolysis bullosa; recurrent fetal loss; pre-eclampsia; traumatic brain injury; myasthenia gravis; cold agglutinin disease; Sjogren's syndrome; dermatomyositis; bullous pemphigoid; phototoxic reactions; Shiga toxin E. coli-related hemolytic uremic syndrome; typical or infectious hemolytic uremic syndrome (tHUS); C3 Glomerulonephritis; Antineutrophil cytoplasmic antibody (ANCA)-associated vasculitis; humoral and vascular transplant rejection; acute antibody mediated rejection (AMR); graft dysfunction; myocardial infarction; an allogeneic transplant; sepsis; coronary artery disease; hereditary angioedema; dermatomyositis; Graves' disease; atherosclerosis; Alzheimer's disease (AD); Huntington's disease; Creutzfeld-Jacob disease; Parkinson's disease; cancers; wounds; septic shock; spinal cord injury; uveitis; diabetic ocular diseases; retinopathy of prematurity; glomerulonephritis; membranous nephritis; immunoglobulin A nephropathy; adult respiratory distress syndrome (ARDS); chronic obstructive pulmonary disease (COPD); cystic fibrosis; hemolytic anemia; paroxysmal cold hemoglobinuria; anaphylactic shock; allergy; osteoporosis; osteoarthritis; Hashimoto's thyroiditis; type I diabetes; psoriasis; pemphigus; autoimmune hemolytic anemia (AIHA); idiopathic thrombocytopenic purpura (ITP); Goodpasture syndrome; Degos disease; antiphospholipid syndrome (APS); catastrophic APS (CAPS); a cardiovascular disorder; myocarditis; a cerebrovascular disorder; a peripheral vascular disorder; a renovascular disorder; a mesenteric/enteric vascular disorder; vasculitis; Henoch-Schonlein purpura nephritis; Takayasu's disease; dilated cardiomyopathy; diabetic angiopathy; Kawasaki's disease (arteritis); venous gas embolus (VGE), restenosis following stent placement; rotational atherectomy; membranous nephropathy; Guillain-Barre syndrome (GBS); Fisher syndrome; antigeninduced arthritis; synovial inflammation; viral infections; bacterial infections; fungal infections; and injury resulting from myocardial infarction, cardiopulmonary bypass and hemodialysis.

In certain embodiments, the complement-mediated disease or condition is an ocular disease condition. In further embodiments, the ocular condition is macular degeneration. In further embodiments the macular degeneration is AMD. In further embodiments, the AMD is the dry form of AMD.

In certain embodiments, the complement-mediated disease or condition is PNH.

In certain embodiments, the complement-mediated disease or condition is a myocardial infarction.

In certain embodiments, the complement-mediated disease or condition is RA.

In certain embodiments, the complement-mediated disease or condition is osteoporosis or osteoarthritis.

In certain embodiments, the complement-mediated disease or condition is inflammation.

In certain embodiments, the complement-mediated disease or condition is cancer.

Antibodies of the invention can be for use in a method of therapy either alone or in combination with other agents. For instance, an antibody of the invention is optionally to be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-C5 antibody and administration of an additional therapeutic agent occur within about one month, or within about one, two or three weeks, or within about one, two, three, four, five, or six days, of each other.

An antibody of the invention (and any additional therapeutic agent) is to be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and are to be administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For use in a method of prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is to be administered for preventive or therapeutic purposes, previous therapy, the patient's clinical history and response to the antibody, and the discretion of the attending physician. The antibody is to be suitably administered to the patient at one time or over a series of treatments. Depending on the type and severity of the disease, about 1 micro g/kg to 15 mg/kg (e.g., 0.1mg/kg-10mg/kg) of antibody can be an initial candidate dosage for administration to the patient, whether, for example, by one or more separate administrations, or by continuous infusion. One typical daily dosage might range from about 1 micro g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g., every week or every three weeks (e.g., such that the patient receives from about two to about twenty, or e.g., about six doses of the antibody). An initial higher loading dose, followed by one or more lower doses may be administered. The progress of this therapy is easily monitored by conventional techniques and assays.

It is understood that any of the above formulations or therapeutic methods may be carried out using an immunoconjugate of the invention in place of or in addition to an anti-C5 antibody.

### H. Articles of Manufacture

In another aspect of the disclosure, an article of manufacture containing materials for use in a method of treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for use in a method of treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is for use in a method of treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the disclosure may further comprise a package insert indicating that the compositions are for use in a method of treating a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the disclosure in place of or in addition to an anti-C5 antibody.

### [Examples]

The following are examples of methods and compositions of the invention. It is understood that various other embodiments may be practiced, given the general description provided above.

### [EXAMPLE 1]

### Preparation of C5

1.1. Expression and purification of recombinant human and cynomolgus monkey C5 Recombinant human C5 (NCBI GenBank accession number: NP_001726.2, SEQ ID NO: 39) was expressed transiently using FreeStyle293-F cell line (Thermo Fisher, Carlsbad, CA, USA). Conditioned media expressing human C5 was diluted with equal volume of milliQ water, then applied to a Q-sepharose FF or Q-sepharose HP anion exchange column (GE healthcare, Uppsala, Sweden), followed by elution with a NaCl gradient. Fractions containing human C5 were pooled, then salt concentration and pH was adjusted to 80mM NaCl and pH6.4, respectively. The resulting sample was applied to a SP-sepharose HP cation exchange column (GE healthcare, Uppsala, Sweden) and eluted with a NaCl gradient. Fractions containing human C5 were pooled and subjected to CHT ceramic Hydroxyapatite column (Bio-Rad Laboratories, Hercules, CA, USA). Human C5 eluate was then applied to a Superdex 200 gel filtration column (GE healthcare, Uppsala, Sweden). Fractions containing human C5 were pooled and stored at -150 degrees C.

Expression and purification of recombinant cynomolgus monkey C5 (NCBI GenBank accession number: XP_005580972, SEQ ID NO: 44) was performed the same way as the human counterpart.

### 1.2. Purification of cynomolgus monkey C5 (cynoC5) from plasma

Plasma sample from cynomolgus monkey was applied to SSL7-agarose (Invivogen, San Diego, CA, USA) followed by elution with 100mM NaAcetate, pH3.5. Fractions containing cynoC5 were immediately neutralized and subjected to a Protein A HP column (GE healthcare, Uppsala, Sweden) in tandem to a Peptide M agarose (Invivogen, San Diego, CA, USA). The flow through fraction was then applied to a Superdex 200 gel filtration column (GE healthcare, Uppsala, Sweden). Fractions containing cynoC5 were pooled and stored at -80 degrees C.

### [EXAMPLE 2]

### Generation of anti-C5 antibodies

### 2.1. Antibody screening

Anti-C5 antibodies were prepared, selected and assayed as follows:
Twelve to sixteen week old NZW rabbits were immunized intradermally with human C5 and/or monkey C5 (50-100 micro g/dose/rabbit). This dose was repeated 4-5 times over a 2 month period. One week after the final immunization, the spleen and blood were collected from the immunized rabbits. Antigen-specific B-cells were stained with labelled antigen, sorted with FCM cell sorter (FACS aria III, BD), and plated in 96-well plates at one cell/well density together with 25,000 cells/well of EL4 cells (European Collection of Cell Cultures) and activated rabbit T-cell conditioned medium diluted 20 times, and were cultured for 7-12 days. EL4 cells were treated with mitomycin C (Sigma, Cat No. M4287) for 2 hours and washed 3 times in advance. The activated rabbit T-cell conditioned medium was prepared by culturing rabbit thymocytes in RPMI-1640 containing Phytohemagglutinin-M (Roche, Cat No. 1 1082132-001), phorbol 12-myristate 13-acetate (Sigma, Cat No. P1585) and 2% FBS. After cultivation, B-cell culture supernatants were collected for further analysis and pellets were cryopreserved.

An ELISA assay was used to test the specificity of antibodies in a B-cell culture supernatant. Streptavidin (GeneScript, Cat No. Z02043) was coated onto a 384-well MAXISorp (Nunc, Cat No. 164688) at 50nM in PBS for 1 hour at room temperature. Plates were then blocked with Blocking One (Nacalai Tesque, Cat No. 03953-95) diluted 5 times. Human or monkey C5 was labelled with NHS-PEG4-Biotin (PIERCE, Cat No. 21329) and was added to the blocked ELISA plates, incubated for 1 hour and washed. B-cell culture supernatants were added to the ELISA plates, incubated for 1 hour and washed. Binding was detected by goat anti-rabbit IgG-Horseradish peroxidase (BETHYL, Cat No. A120-111P) followed by the addition of ABTS (KPL, Cat No. 50-66-06).

An ELISA assay was used to evaluate pH-dependent binding of antibodies against C5. Goat anti-rabbit IgG-Fc (BETHYL, Cat No. A120-111A) diluted to 1 micro g/ml with PBS(-) was added to a 384-well MAXISorp (Nunc, Cat No. 164688), incubated for 1 hour at room temperature, and blocked with Blocking One (Nacalai Tesque, Cat No. 03953-95) diluted 5 times. After incubation, plates were washed and B-cell culture supernatants were added. Plates were incubated for 1 hour, washed, and 500pM of biotinylated human or monkey C5 was added and incubated for 1 hour. After incubation, plates were washed and incubated with either pH7.4 MES buffer (20 mM MES, 150 mM NaCl and 1.2 mM CaCl₂) or pH5.8 MES buffer (20 mM MES, 150 mM NaCl and 1 mM EDTA) for 1 hour at room temperature. After incubation, binding of biotinylated C5 was detected by Streptavidin-Horseradish peroxidase conjugate (Thermo Scientific, Cat No. 21132) followed by the addition of ABTS (KPL, Cat No. 50-66-06).

Octet RED384 system (Pall Life Sciences) was used to evaluate affinity and pH-dependent binding of antibodies against C5. Antibodies secreted in the B-cell culture supernatant were loaded onto a Protein A biosensor tip (Pall Life Sciences) and dipped into 50 nM of human or monkey C5 in pH7.4 MES buffer to analyze association kinetics. Dissociation kinetics was analyzed in both pH7.4 MES buffer and pH5.8 MES buffer.

A total of 41,439 B-cell lines were screened for affinity and pH-dependent binding to human or monkey C5 and 677 lines were selected and designated CFA0001-0677. RNA of the selected lines was purified from cryopreserved cell pellets using ZR-96 Quick-RNA kits (ZYMO RESEARCH, Cat No. R1053). DNA encoding antibody heavy chain variable regions in the selected lines was amplified by reverse transcription PCR and recombined with DNA encoding F760G4 (SEQ ID NO: 33) or F939G4 (SEQ ID NO: 34) heavy chain constant region. DNA encoding antibody light chain variable regions was amplified by reverse transcription PCR and recombined with DNA encoding k0MTC light chain constant region (SEQ ID NO: 36). Separately, the heavy and light chain genes of an existing humanized anti-C5 antibody, eculizumab (EcuH-G2G4, SEQ ID NO: 29 and EcuL-k0, SEQ ID NO: 30), were synthesized. DNA encoding VH (EcuH, SEQ ID NO: 31) was fused in-frame to DNA encoding a modified human IgG4 CH (F760G4, SEQ ID NO: 33), and DNA encoding VL (EcuL, SEQ ID NO: 32) was fused in-frame to DNA encoding a k0 light chain constant region (SEQ ID NO: 37). Each of the fused coding sequences was also cloned into an expression vector. The antibodies were expressed in FreeStyle^{™} 293-F Cells (Invitrogen) and purified from culture supernatant to evaluate functional activity. Neutralizing activities of the antibodies were evaluated by testing inhibition of complement activity using a liposome lysis assay as described in Example 5.1.

### 2.2. Epitope binning by sandwich ELISA

Anti-C5 antibodies with high affinity, pH dependency or neutralizing activity were selected for further analysis. A sandwich ELISA assay was used to group the selected antibodies into different epitope bins binding to the same or overlapping epitopes of the C5 protein. Unlabelled capture antibodies were diluted to 1 micro g/ml with PBS (-) and added to 384-well MAXISorp plates (Nunc, Cat No. 164688). Plates were incubated for 1 hour at room temperature and blocked with Blocking One (Nacalai Tesque, Cat No. 03953-95) diluted 5 times. Plates were incubated for 1 hour, washed, and 2 nM of human C5 was added and incubated for 1 hour. After incubation, plates were washed and labelled detection antibodies (1 micro g/mL, biotinylated by NHS-PEG4-Biotin) were added. After 1 hour incubation, binding of biotinylated antibody was detected by Streptavidin-Horseradish peroxidase conjugate (Thermo Scientific, Cat No. 21132) followed by the addition of ABTS (KPL, Cat No. 50-66-06).

All anti-C5 antibodies were used as both a capture antibody and a detection antibody, and paired comprehensively. As shown in Figure 1, mutually competitive antibodies were grouped into 7 epitope bins: CFA0668, CFA0334 and CFA0319 were grouped into epitope A, CFA0647, CFA0589, CFA0341, CFA0639, CFA0635, CFA0330 and CFA0318 were grouped into epitope B, CFA0538, CFA0501, CFA0599, CFA0307, CFA0366, CFA0305, CFA0675, CFA0666 and CFA0672 were grouped into epitope C, eculizumab and CFA0322 were grouped into epitope D, CFA0329 was grouped into epitope E, CFA0359 and CFA0217 were grouped into epitope F, and CFA0579, CFA0328 and CFA0272 were grouped into epitope G. Figure 1 shows epitope binning of some of the anti-C5 chimeric antibodies. The sequences of the VH and VL anti-C5 antibodies grouped into epitope C are listed in Table 2.

**[Table 2]**

| **Anti-C5 antibodies grouped into epitope C** | | | | | | | | |
|---|---|---|---|---|---|---|---|---|
| | **SEQ ID NO:** | | | | | | | |
| **Antibody** | **VH** | **VL** | **HVR-H1** | **HVR-H2** | **HVR-H3** | **HVR-L1** | **HVR-L2** | **HVR-L3** |
| CPA0305 | 1 | 11 | 45 | 55 | 65 | 75 | 85 | 95 |
| CFA0307 | 2 | 12 | 46 | 56 | 66 | 76 | 86 | 96 |
| CFA0366 | 3 | 13 | 47 | 57 | 67 | 77 | 87 | 97 |
| CFA0501 | 4 | 14 | 48 | 58 | 68 | 78 | 88 | 98 |
| CFA0538 | 5 | 15 | 49 | 59 | 69 | 79 | 89 | 99 |
| CFA0599 | 6 | 16 | 50 | 60 | 70 | 80 | 90 | 100 |
| CFA0666 | 7 | 17 | 51 | 61 | 71 | 81 | 91 | 101 |
| CFA0672 | 8 | 18 | 52 | 62 | 72 | 82 | 92 | 102 |
| CFA0675 | 9 | 19 | 53 | 63 | 73 | 83 | 93 | 103 |

### 2.3. Humanization and optimization

Humanization of the variable region of some of the anti-C5 antibodies was performed in order to reduce the potential immunogenicity of the antibodies. Complementarity-determining regions (CDRs) of the anti-C5 rabbit antibody were grafted onto homologous human antibody frameworks (FRs) using a conventional CDR grafting approach (Nature 321:522-525 (1986)). The genes encoding the humanized VH and VL were synthesized and combined with a modified human IgG4 CH (SG402, SEQ ID NO: 35) and a human CL (SK1, SEQ ID NO: 38), respectively, and each of the combined sequences was cloned into an expression vector.

A number of mutations and mutation combinations were examined to identify mutations and mutation combinations that improved the binding properties of some of the lead antibodies. Multiple mutations were then introduced to the humanized variable regions to enhance the binding affinity to C5 at a neutral pH or to reduce the binding affinity to C5 at an acidic pH. One of the optimized variants, 305LO5 (VH, SEQ ID NO: 10; VL, SEQ ID NO: 20; HVR-H1, SEQ ID NO: 54; HVR-H2, SEQ ID NO: 64; HVR-H3, SEQ ID NO: 74; HVR-L1, SEQ ID NO: 84; HVR-L2, SEQ ID NO: 94; and HVR-L3, SEQ ID NO: 104), was hence generated from CFA0305.

Antibodies were expressed in HEK293 cells co-transfected with a mixture of heavy and light chain expression vectors and were purified by protein A.

### [EXAMPLE 3]

### Binding characterization of anti-C5 antibodies

### 3.1. Expression and purification of recombinant antibodies

Recombinant antibodies were expressed transiently using FreeStyle293-F cell line (Thermo Fisher, Carlsbad, CA, USA). Purification from the conditioned media expressing antibodies was performed using a conventional method using protein A. Gel filtration was further conducted if needed.

### 3.2. Assessment of pH dependency

The kinetic parameters of anti-C5 antibodies against recombinant human C5 were assessed at pH7.4 and pH5.8, at 37 degrees C using BIACORE (registered trademark) T200 instrument (GE Healthcare). ProA/G (Pierce) was immobilized onto a CM4 sensorchip using amine coupling kit (GE Healthcare) according to the recommended settings by GE Healthcare. Antibodies and analytes were diluted into the respective running buffers, ACES pH7.4 and pH5.8 (20 mM ACES, 150 mM NaCl, 1.2 mM CaCl₂, 0.05% Tween 20, 0.005% NaN₃). Each antibody was captured onto the sensor surface by ProA/G. Antibody capture levels were typically 60-90 resonance units (RU). Then, recombinant human C5 was injected at concentrations of 10 and 20 nM or 20 and 40 nM followed by dissociation. The surface was regenerated using 25 mM NaOH. Kinetic parameters at both pH conditions were determined by fitting the sensorgrams with 1: 1 binding model using BIACORE (registered trademark) T200 Evaluation software, version 2.0 (GE Healthcare). The sensorgrams of all antibodies are shown in Figures 2A and 2B. The association rate (ka), dissociation rate (kd), and binding affinity (KD) of the antibodies are listed in Table 3. All antibodies except CFA0330 (VH, SEQ ID NO: 21 and VL, SEQ ID NO: 25) and CFA0341 (VH, SEQ ID NO: 22 and VL, SEQ ID NO: 26) showed a relatively faster dissociation rate at pH 5.8 than pH7.4.

**Table 3**

| **Kinetic parameters of anti-C5 antibodies under pH7.4 and pH5.8 conditions** | | | | | | |
|---|---|---|---|---|---|---|
| Antibody Name | pH7.4 | | | pH5.8 | | |
| | ka | kd | KD | ka | kd | KD |
| CFA0305 | 3.82E+04 | 5.89E-04 | 1.54E-08 | 4.27E+04 | 1.83E-02 | 4.30E-07 |
| CFA0307 | 3.24E+05 | 2.63E-03 | 8.13E-09 | 2.04E+05 | 3.34E-02 | 1.64E-07 |
| CFA0366 | 1.04E+06 | 9.34E-03 | 8.99E-09 | 9.35E+05 | 7.03E-02 | 7.52E-08 |
| CFA0501 | 4.74E+05 | 1.69E-03 | 3.56E-09 | 1.50E+05 | 2.62E-02 | 1.74E-07 |
| CFA0538 | 4.73E+05 | 1.85E-03 | 3.91E-09 | 1.22E+05 | 3.01E-02 | 2.46E-07 |
| CFA0599 | 4.74E+05 | 2.81E-03 | 5.93E-09 | 4.54E+05 | 3.73E-02 | 8.21E-08 |
| CFA0666 | 3.65E+05 | 6.26E-04 | 1.71E-09 | 2.82E+05 | 9.39E-03 | 3.33E-08 |
| CFA0672 | 5.23E+05 | 1.83E-04 | 3.51E-10 | 7.11E+04 | 9.78E-03 | 1.38E-07 |
| CFA0675 | 3.83E+05 | 4.12E-04 | 1.08E-09 | 3.89E+05 | 6.61E-03 | 1.70E-08 |
| 305-LO5 | 4.48E+05 | 2.11E-04 | 4.71E-10 | 2.03E+06 | 2.85E-02 | 1.40E-08 |
| CFA0330 | 1.66E+06 | 2.02E-04 | 1.22E-10 | 1.22E+06 | 2.24E-04 | 1.84E-10 |
| CFA0341 | 6.28E+05 | 9.77E-05 | 1.55E-10 | 1.24E+06 | 7.39E-05 | 5.95E-11 |

### 3.3. Cross reactivity check

To observe the cross-reactivity of anti-C5 antibodies against human C5 (hC5) and cynomolgus monkey C5 (cynoC5), BIACORE (registered trademark) kinetics analysis was performed. The assay setting was the same as described in Example 3.2, Recombinant cynoC5 was injected at concentrations of 2, 10, and 50 nM. Kinetic parameters were determined by the same data fitting as described in Example 3.2. Binding kinetics and affinity at pH7.4 are listed in Table 4. The kinetic parameters against hC5 presented in Table 4 are the results of Example 3.2. All anti-C5 antibodies except CFA0672 showed comparable KD toward hC5 and cynoC5. KD of CFA0672 toward cynoC5 was 8 times weaker than toward hC5.

**[Table 4]**

| **Binding kinetics and affinity of anti-C5 antibodies against hC5 and cynoC5 at pH7.4** | | | | | | |
|---|---|---|---|---|---|---|
| Antibody Name | affinity against hC5 | | | affinity against cynoC5 | | |
| | ka | kd | KD | ka | kd | KD |
| CFA0305 | 3.82E+04 | 5.89E-04 | 1.54E-08 | 1.21E+04 | 6.70E-04 | 5.54E-09 |
| CFA0307 | 3.24E+05 | 2.63E-03 | 8.13E-09 | 2.90E+05 | 2.23E-03 | 7.68E-09 |
| CFA0366 | 1.04E+06 | 9.34E-03 | 8.99E-09 | 5.04E+05 | 9.04E-03 | 1.79E-08 |
| CFA0501 | 4.74E+05 | 1.69E-03 | 3.56E-09 | 2.66E+05 | 1.56E-03 | 5.88E-09 |
| CFA0538 | 4.73E+05 | 1.85E-03 | 3.91E-09 | 3.05E+05 | 1.66E-03 | 5.44E-09 |
| CFA0599 | 4.74E+05 | 2.81E-03 | 5.93E-09 | 5.42E+05 | 2.35E-03 | 4.33E-09 |
| CFA0666 | 3.65E+05 | 6.26E-04 | 1.71E-09 | 3.14E+05 | 4.93E-04 | 1.57E-09 |
| CFA0672 | 5.23E+05 | 1.83E-04 | 3.51E-10 | 6.41E+05 | 1.85E-03 | 2.88E-09 |
| CFA0675 | 3.83E+05 | 4.12E-04 | 1.08E-09 | 2.94E+05 | 3.78E-04 | 1.29E-09 |

### [EXAMPLE 4]

### Epitope mapping of anti-C5 antibodies

4.1. Binding of anti-C5 MAbs to C5 beta-chain-derived peptides Anti-C5 monoclonal antibodies (MAbs) were tested for binding to C5 beta-chain-derived peptides in Western blot analysis. The C5 peptides: 19-180, 161-340, 321-500, and 481-660, fused to GST-tag (pGEX-4T-1, GE Healthcare Life Sciences, 28-9545-49) were expressed in E. coli (DH5 alpha, TOYOBO, DNA-903). The E. coli samples were harvested after incubation with 1 mM Isopropyl beta-D-1-thiogalactopyranoside (IPTG) for 5 hours at 37 degrees C, and centrifuged at 20000 x g for 1 min to obtain pellets. The pellets were suspended with a sample buffer solution (2ME+) (Wako, 191-13272), and used for Western blot analysis. Expression of each peptide was confirmed with anti-GST antibody (Abcam, ab9085) (Figure 3). The arrow indicates GST-fused C5 peptides (46-49kDa). Anti-C5 MAbs: CFA0305, CFA0307, CFA0366, CFA0501, CFA0538, CFA0599, CFA0666, CFA0672, and CFA0675, bound to 19-180 of C5 (Figure 3).

### 4.2. Expression and purification of MG1-MG2 domain (1-225) of human C5

Recombinant MG1-MG2 domain (SEQ ID NO: 43) of human C5 beta-chain was expressed transiently using FreeStyle293-F cell line (Thermo Fisher, Carlsbad, CA, USA). Conditioned media expressing the MG1-MG2 domain was diluted with 1/2 vol of milliQ water, followed by application to a Q-sepharose FF anion exchange column (GE healthcare, Uppsala, Sweden). The flow through fraction from the anion exchange column was adjusted to pH 5.0 and applied to a SP-sepharose HP cation exchange column (GE healthcare, Uppsala, Sweden) and eluted with a NaCl gradient. Fractions containing the MG1-MG2 domain were collected from the eluent and subsequently subjected to a Superdex 75 gel filtration column (GE healthcare, Uppsala, Sweden) equilibrated with 1x PBS. The fractions containing the MG1-MG2 domain were then pooled and stored at -80 degrees C.

### 4.3. Binding ability to MG1-MG2 domain

The binding ability of anti-C5 antibodies towards the MG1-MG2 domain was measured using the same assay settings as described in Example 3.2, except that measurements were only performed under pH7.4 conditions. The MG1-MG2 domain was injected at concentrations of 20 nM and 40 nM. As shown in Figure 4, all antibodies except eculizumab-F760G4 showed an increase of the binding response, indicating these antibodies are MG1-MG2 binders. Eculizumab-F760G4, which is a known alpha-chain binder, did not show binding to MG1-MG2 domain.

### 4.4. Binding of anti-C5 MAbs to C5 MG1-MG2 domain-derived peptides

The anti-C5 MAbs were tested for binding to MG1-MG2 domain-derived peptides in Western blot analysis. The C5 peptides: 33-124, 45-124, 52-124, 33-111, 33-108, and 45-111 (SEQ ID NO:40), fused to GST-tag, were expressed in E. coli. The E. coli samples were harvested after incubation with 1 mM IPTG for 5 hours at 37 degrees C, and centrifuged at 20000 x g for 1 min to obtain pellets. The pellets were suspended with the sample buffer solution (2ME+), and used for Western blot analysis. Expression of C5-derived peptides was confirmed with anti-GST antibody (Figure 5A). CFA0305 bound to only the peptide of 33-124 (Figure 5B). CFA0305 bound to beta-chain of recombinant human C5 (rhC5) (approx.70kDa), which was used as a control. Figure 5C summarizes the reaction of anti-C5 MAbs to C5-derived peptides.

### 4.5. Binding of anti-C5 MAbs to C5 mutants

Since three amino acid residues in the C5 beta-chain: E48, D51, and K109, were predicted to be involved in the binding between C5 and the anti-C5-MAbs by crystal structure analysis, the anti-C5 MAbs were tested for binding to human C5 point mutants in Western blot analysis. C5 point mutants, in which any one of E48, D51, and K109 was substituted with alanine, were expressed in FS293 cells by lipofection. Culture media was harvested 5 days after lipofection, and thereafter used for Western blot. SDS-PAGE was conducted under reducing conditions. The results are shown in Figure 6. Eculizumab bound to alpha-chain of wild type (WT) C5 and three C5 point mutants, whereas CFA0305 bound to the beta-chain of WT C5 strongly, the E48A C5 mutant weakly, and did not bind to the beta-chain of the D51A and K109A C5 mutants, indicating that these 3 amino acid residues are involved in the antibody/antigen interactions. Table 5 presents a summary of Western blot analysis of the anti-C5 MAbs (CFA0305, CFA0307, CFA0366, CFA0501, CFA0538, CFA0599, CFA0666, CFA0672, and CFA0675). The anti-C5 MAbs are grouped into the same epitope C, but binding patterns are slightly different between the antibodies, suggesting that the binding regions of C5 to the anti-C5 MAbs are close to each other but not identical.

**[Table 5]**

| **Summary of anti-C5 MAbs reaction to C5 mutants** | | | | |
|---|---|---|---|---|
| | WT | E48A | D51A | K109A |
| Eculizumab | + | + | + | + |
| CFA0305 | + | + | - | - |
| CFA0307 | + | - | - | - |
| CFA0366 | + | - | - | - |
| CFA0501 | + | - | - | - |
| CFA0538 | + | - | - | - |
| CFA0599 | + | - | - | + |
| CFA0666 | + | - | - | + |
| CFA0672 | + | - | - | + |
| CFA0675 | + | + | - | + |

### 4.6. BIACORE (registered trademark) binding analysis of anti-C5 antibodies with C5 mutants

To test if residues E48, G51, and K109 are indeed involved in antibody/antigen interactions, BIACORE (registered trademark) binding analysis was performed. Three C5 mutants were prepared: E48A, G51A, and K109A, as described in Example 4.5. Culture supernatant samples containing the mutant C5 over-expressed in FS293 cells were prepared at 40 micro g/ml of the mutant C5. For BIACORE (registered trademark) binding analysis, the sample was diluted 10x with BIACORE (registered trademark) running buffer (ACES pH7.4, 10 mg/ml BSA, 1 mg/ml carboxymethyl dextran) to a final sample concentration of 4 micro g/ml of the mutant C5.

The interactions of the three C5 mutants with anti-C5 antibodies were assessed at 37 degrees C with BIACORE (registered trademark) T200 instrument (GE Healthcare), using the assay condition described in Example 3.2. ACES pH 7.4 buffer containing 10 mg/ml BSA, 1 mg/ml carboxymethyl dextran was used as running buffer. Eculizumab-F760G4 and 305LO5 were captured on different flow cells by monoclonal mouse anti-human IgG, Fc fragment specific antibody (GE Healthcare). Flow cell 1 was used as the reference surface. Wild type and mutant C5 proteins were injected over sensor surface at 4 micro g/ml concentration to interact with the captured antibodies. At the end of each analysis cycle, the sensor surface was regenerated with 3M MgCh. The results were analyzed with Bia Evaluation software, version 2.0 (GE Healthcare). Curves of reference flow cell (flow cell 1) and blank injections of running buffer were subtracted from curves of the flow cell with captured antibodies.

As shown in Figure 7, all three C5 mutants could bind to eculizumab with a similar binding profile compared to wild type C5. For the 305LO5, all three mutants showed lower binding response to 305LO5 compared to wild type C5. The D51A and K109A mutants reduced the binding of C5 by 305LO5 to the baseline level.

### 4.7. Identification of His residues on C5 that contribute to pH dependent interactions between anti-C5 antibody and C5

The crystal structure analysis revealed that 3 histidine residues on human C5 are located at the antibody/antigen interface. A histidine residue with a typical pKa of approximately 6.0 is known to contribute to pH dependent protein-protein interactions (Igawa et al., Biochim Biophys Acta 1844(11):1943-1950 (2014)). To investigate which of the His residues on the antibody/antigen interface contribute to pH dependent interactions between anti-C5 antibody and C5, BIACORE (registered trademark) binding analysis was performed. Three human C5 mutants with a single His mutation (H70Y, H72Y, and H110Y) and a mutant with a double-His mutation (H70Y + H110Y) were prepared as follows: single His mutants in which any one of H70, H72, and H110 is substituted with tyrosine, and a double His mutant in which both H70 and H110 are substituted with tyrosine, were expressed in FS293 cells by lipofection. The antigen binding properties of the C5 His mutants to 305LO5, a pH-dependent anti-C5 antibody, were determined by a modified BIACORE (registered trademark) assay as described in Example 4.6. Briefly, an additional dissociation phase at pH5.8 was integrated into the BIACORE (registered trademark) assay immediately after the dissociation phase at pH7.4 to assess the pH-dependent dissociation between the antibody and the antigen from the complexes formed at pH7.4. The dissociation rate at pH5.8 was determined by processing and fitting data using Scrubber 2.0 (BioLogic Software) curve fitting software.

As shown in Figure 8, the C5 single His mutation at H70 or H110 and the double His mutation (H70 + H110) did not affect the binding of C5 to the 305LO5 at neutral pH. Meanwhile, the single His mutation at H72 exhibited a significant impairment of binding of C5 to 305LO5. The dissociation rates at pH5.8 for the C5 His mutants and the C5-wt protein are shown in Table 6. As shown in Table 6, the C5-wt showed fastest dissociation from 305LO5 at pH5.8 among the C5 antigens tested. The single His mutation at H70 exhibited an almost two-fold slower dissociation rate at pH5.8 and the single His mutation at H110 resulted in a slightly slower dissociation rate at pH5.8 compared to C5-wt. The double His mutation at both H70 and H110 resulted in larger effect on pH-dependent binding with a dissociation rate at pH5.8 almost three fold slower than C5-wt.

**[Table 6]**

| **pH5.8 dissociation rate value for C5 His mutants binding to 305LO5** | |
|---|---|
| **Antigens** | **kd (1/s)** |
| C5-wt | 1.1E-2 |
| C5-H70Y | 5.3E-3 |
| C5-H110Y | 9.3E-3 |
| C5-H70Y, H110Y | 3.9E-3 |

### [EXAMPLE 5]

### Inhibitory activity of anti-C5 antibodies on C5 activation

5.1. Inhibition of complement-activated liposome lysis by anti-C5 MAbs The anti-C5 MAbs were tested for inhibition of complement activity by a liposome lysis assay. Thirty microliters of normal human serum (6.7%) (Biopredic, SER018) was mixed with 20 micro L of the diluted MAb in a 96-well plate and incubated on a shaker for 30 min at 25 degrees C. Liposomes sensitized with the antibodies against dinitrophenyl (Autokit CH50, Wako, 995-40801) were transferred into each well and the plate was placed on a shaker for 2 min at 25 degrees C. Fifty microliters of substrate solution (Autokit CH50) was added to each well and mixed by shaking for 2 min at 25 degrees C. The final mixture was incubated at 37 degrees C for 40 minutes, and thereafter OD at 340 nm of the mixture was measured. The percent of liposome lysis was defined as 100 × [(OD _{MAb} - OD _{serum and liposome background})]/ [(OD _{without MAb} - OD _{serum and liposome background})]. Figure 9A shows that anti-C5 Mabs: CFA0305, 0307, 0366, 0501, 0538, 0599, 0666, 0672, and 0675, inhibited the liposome lysis. Two non-pH-dependent antibodies: CFA0330 and 0341, also inhibited the lysis (Figure 9B).

### 5.2. Inhibition of C5a generation by anti-C5 MAbs

The anti-C5 MAbs were tested for C5a generation during liposome lysis to confirm that the anti-C5 MAbs inhibit cleavage of C5 into C5a and C5b. The C5a level in the supernatants from liposome lysis assay was quantified using a C5a ELISA kit (R&D systems, DY2037). All MAbs inhibited C5a generation in the supernatants dosedependently (Figures 10A and 10B).

### 5.3. Inhibition of complement-activated hemolysis by anti-C5 MAbs

The anti-C5 MAbs were tested for inhibition of the classical complement activity in a hemolytic assay. Chicken red blood cells (cRBCs) (Innovative research, IC05-0810) were washed with gelatin/veronal-buffered saline containing 0.5 mM MgCl₂ and 0.15 mM CaCl₂ (GVB++) (Boston BioProducts, IBB-300X), and thereafter sensitized with anti-chicken RBC antibody (Rockland 103-4139) at 1 micro g/ml for 15 minutes at 4 degrees C. The cells were then washed with GVB++ and suspended in the same buffer at 5×10⁷ cells/ml. In a separate round-bottom 96-well microtest plate, 50 micro l of normal human serum (20%) (Biopredic, SER019) was mixed with 50 micro l of diluted Mab and incubated on a shaker at 37 degrees C for 30 minutes. Sixty microliters of the sensitized cRBCs suspension was then added to the wells containing the serum, and the antibody mixture was incubated at 37 degrees C for 30 minutes. After the incubation, the plate was centrifuged at 1000 × g for 2 minutes at 4 degrees C. Supernatants (100 micro l) were transferred to wells on a flat-bottom 96-well microtest plate for measurement of OD at 415 nm with a reference wavelength at 630 nm. The percent of hemolysis was defined as 100 × [(OD _{MAb} - OD _{serum and CRBCs})]/ [(OD _{without MAb} - OD _{serum and cRBCs background})]. Figure 11 shows that anti-C5 Mabs: CFA0305 and 305LO5, inhibited the hemolysis of cRBCs.

### 5.4. Inhibition of alternative complement pathway by anti-C5 MAbs

A hemolytic assay for the alternative pathway was performed in a similar way to the classical pathway haemolytic assay. Blood collected from a New Zealand White rabbit (InVivos) was mixed with the same volume of Alsever's solution (Sigma, A3551), and the mixture was used as rabbit RBCs (rRBCs). rRBCs were washed with GVB supplemented with 2 mM MgCl₂ and 10 mM EGTA and suspended in the same buffer at 7×10⁸ cells/ml. In a round-bottom 96-well microtest plate, 40 micro l of normal human serum (25%) (Biopredic, SER019) was mixed with 40 micro l of diluted Mab and incubated on a shaker at 37 degrees C for 30 minutes. Twenty microliters of the rRBCs suspension was then added to the wells containing the serum, and the antibody mixture was incubated at 37 degrees C for 60 minutes. After the incubation, the plate was centrifuged at 1000 × g for 2 minutes at 4 degrees C. Supernatants (70 micro l) were transferred to wells on a flat-bottom 96-well microtest plate for measurement of OD at 415 nm with a reference wavelength at 630 nm. Figure 12 shows that anti-C5 Mabs: CFA0305 and CFA0672, inhibited the hemolysis of rRBCs, indicating that these antibodies inhibit the alternative complement pathways.

### [EXAMPLE 6]

### Pharmacokinetic study of anti-C5 monoclonal antibodies with human C5 in mice

### 6.1. In vivo test using C57BL/6 mice

The in vivo kinetics of human C5 (Calbiochem) and anti-human C5 antibody was assessed after administering human C5 alone or human C5 and anti-human C5 antibody to C57BL/6 mice (In Vivos or Biological Resource Centre, Singapore). A human C5 solution (0.01 mg/ml) or a solution of mixture containing human C5 and anti-human C5 antibody (0.01 mg/ml and 2 mg/ml (CFA0305-F760G4, CFA0307-F760G4, CFA0366-F760G4, CFA0501-F760G4, CFA0538-F760G4, CFA0599-F760G4, CFA0666-F760G4, CFA0672-F760G4, and CFA0675-F760G4) or 0.2 mg/ml (CFA0330-F760G4, and CFA0341-F760G4), respectively) was administered once at a dose of 10 ml/kg into the caudal vein. In this case, the anti-human C5 antibody is present in excess over human C5, and therefore almost every human C5 is assumed to be bound to the antibody. Blood was collected 5 minutes, seven hours, one day, two days, three days, and seven days after administration. The collected blood was immediately centrifuged at 14,000 rpm and 4 degrees C for 10 minutes to separate the plasma. The separated plasma was stored in a refrigerator at -80 degrees C before assay. The anti-human C5 antibodies used are: above-described CFA0305-F760G4, CFA0307-F760G4, CFA0330-F760G4, CFA0341-F760G4, CFA0366-F760G4, CFA0501-F760G4, CFA0538-F760G4, CFA0599-F760G4, CFA0666-F760G4, CFA0672-F760G4, and CFA0675-F760G4.

### 6.2. Measurement of total human C5 plasma concentration by electrochemiluminescence (ECL) assay

The concentration of total human C5 in mouse plasma was measured by ECL.

In the presence of CFA0330-F760G4, CFA0341-F760G4, or human C5 alone in the plasma sample, the following method was used. Anti-human C5 antibody (Santa Cruz) was dispensed onto a MULTI-ARRAY 96-well bare plate (Meso Scale Discovery) and allowed to stand overnight at 4 degrees C to prepare anti-human C5-immobilized plates. Calibration curve samples and mouse plasma samples diluted 100-fold or more with 1 micro g/ml injected antibody (CFA0330-F760G4 or CFA0341-F760G4) were prepared and incubated for 30 minutes at 37 degrees C. Subsequently, the samples were dispensed onto the anti-human C5-immobilized plates, and allowed to stand for one hour at room temperature. Then, SULFO-TAG labelled anti-human IgG antibody (Meso Scale Discovery) was added to react for one hour at room temperature, and washing was performed. Immediately thereafter, Read Buffer T (x4) (Meso Scale Discovery) was dispensed and measurement was performed using a Sector Imager 2400 (Meso Scale Discovery).

In the presence of CFA0305-F760G4, CFA0307-F760G4, CFA0366-F760G4, CFA0501-F760G4, CFA0538-F760G4, CFA0599-F760G4, CFA0666-F760G4, CFA0672-F760G4, or CFA0675-F760G4 in the plasma sample, the following method was used. Anti-human C5 antibody (CFA0329-F939G4; VH, SEQ ID NO: 23 and VL, SEQ ID NO: 27) was dispensed onto a MULTI-ARRAY 96-well bare plate (Meso Scale Discovery) and allowed to stand overnight at 4 degrees C to prepare anti-human C5-immobilized plates. Calibration curve samples and mouse plasma samples diluted 100-fold or more with acidic solution (pH 5.5) were prepared and incubated for 30 minutes at 37 degrees C. Subsequently, the samples were dispensed onto the anti-human C5-immobilized plates, and allowed to stand for one hour at room temperature. Then, SULFO-TAG labelled anti-human C5 antibody (CFA0300-F939G4; VH, SEQ ID NO: 24 and VL, SEQ ID NO: 28) was added to react for one hour at room temperature, and washing was performed. Immediately thereafter, Read Buffer T (x4) (Meso Scale Discovery) was dispensed and measurement was performed using a Sector Imager 2400 (Meso Scale Discovery).

The human C5 concentration was calculated based on the response of the calibration curve using the analytical software SOFTmax PRO (Molecular Devices). The time course of plasma human C5 concentration after intravenous administration as measured by this method is shown in Figure 13. Data are plotted as the percentage remaining compared to the plasma human C5 concentration at 5 minutes.

### 6.3. Measurement of anti-human C5 antibody plasma concentration by ECL assay

The concentration of anti-human C5 antibody in mouse plasma was measured by ECL. Anti-human IgG (gamma-chain specific) F(ab')2 antibody fragment (Sigma) or anti-human IgG kappa chain antibody (Antibody Solutions) was dispensed onto a MULTI-ARRAY 96-well bare plate (Meso Scale Discovery) and allowed to stand overnight at 4 degrees C to prepare anti-human IgG-immobilized plates. Calibration curve samples and mouse plasma samples diluted 100-fold or more were prepared. Subsequently, the samples were dispensed onto the anti-human IgG-immobilized plates, and allowed to stand for one hour at room temperature. Then, biotinylated Anti-Human IgG Antibody (Southernbiotech) or SULFO-TAG labelled anti-human IgG Fc antibody (Southernbiotech) was added to react for one hour at room temperature, and washing was performed. Subsequently, only when biotinylated Anti-Human IgG Antibody was used, SULFO-TAG labelled streptavidin (Meso Scale Discovery) was added to react for one hour at room temperature, and washing was performed. Immediately therafter, Read Buffer T (x4) (Meso Scale Discovery) was dispensed and measurement was performed using a Sector Imager 2400 (Meso Scale Discovery). The anti-human C5 antibody concentration was calculated based on the response of the calibration curve using the analytical software SOFTmax PRO (Molecular Devices). The time course of plasma anti-human C5 antibody concentration after intravenous administration as measured by this method is shown in Figure 14. Data are plotted as the percentage remaining compared to the plasma anti-human C5 antibody concentration at 5 minutes.

### 6.4. Effect of pH dependent anti-human C5 antibody binding upon clearance of human C5 in vivo

The pH dependent anti-human C5 antibodies (CFA0305-F760G4, CFA0307-F760G4, CFA0366-F760G4, CFA0501-F760G4, CFA0538-F760G4, CFA0599-F760G4, CFA0666-F760G4, CFA0672-F760G4, and CFA0675-F760G4) and non pH dependent anti-human C5 antibodies (CFA0330-F760G4, and CFA0341-F760G4) were tested in vivo and the resulting plasma anti-human C5 antibody concentration and plasma human C5 concentration were compared. As shown in Figure 14, the antibody exposure was comparable. Meanwhile, the clearance of human C5 simultaneously administered with the pH dependent anti-human C5 antibodies was accelerated compared to that with the non pH dependent anti-human C5 antibodies (Figure 13).

### [EXAMPLE 7]

### Optimization of anti-C5 monoclonal antibodies (305 variants)

A number of mutations were introduced to the optimized variable region of the anti-C5 antibody 305LO5 to further improve its properties, and the optimized variable regions 305LO15, 305LO16, 305LO18, 305LO19, 305LO20, 305LO22, and 305LO23 were generated. Amino acid sequences of VH and VL of the 305 variants are listed in Tables 7 and 8, respectively. The genes encoding the humanized VH were combined with a modified human IgG1 CH variant SG115 (SEQ ID NO: 114), and modified human IgG4 CH variants SG422 (SEQ ID NO: 115) or SG429 (SEQ ID NO: 116). The genes encoding the humanized VL were combined with a human CL (SK1, SEQ ID NO: 38). Separately, heavy and light chain genes encoding a humanized anti-C5 antibody, BNJ441 (BNJ441H, SEQ ID NO: 149; BNJ441L, SEQ ID NO: 150), were synthesized and each was cloned into an expression vector.

Antibodies were expressed in HEK293 cells co-transfected with a combination of heavy and light chain expression vectors, and were purified by protein A.

**Table 7**

| **VH amino acid sequences of the 305 variants** | | | | |
|---|---|---|---|---|
| **Antibody** | **VH** | **HVR-H1** | **HVR-H2** | **HVR-H3** |
| 305LO5 | SEQ ID NO: 10 | SEQ ID NO: 54 SSYYVA | SEQ ID NO: 64 AIYTGSGATYKASWAKG | SEQ ID NO: 74 DGGYDYPTHAMHY |
| 305LO15 | SEQ ID NO: 106 | SEQ ID NO: 117 SSYYMA | SEQ ID NO: 118 AIFTGSGAEYKAEWAKG | SEQ ID NO: 121 DAGYDYPTHAMHY |
| 305LO16 | SEQ ID NO: 107 | SEQ ID NO: 117 SSYYMA | SEQ ID NO: 119 AIFTGSGAEYKAEWVKG | SEQ ID NO: 121 DAGYDYPTHAMHY |
| 305LO18 | SEQ ID NO: 108 | SEQ ID NO: 117 SSYYMA | SEQ ID NO: 118 AIFTGSGAEYKAEWAKG | SEQ ID NO: 121 DAGYDYPTHAMHY |
| 305LO19 | SEQ ID NO: 109 | SEQ ID NO: 117 SSYYMA | SEQ ID NO: 118 AIFTGSGAEYKAEWAI<G | SEQ ID NO: 121 DAGYDYPTHAMHY |
| 305LO20 | SEQ ID NO: 109 | SEQ ID NO: 117 SSYYMA | SEQ ID NO: 118 AIFTGSGAEYKAEWAKG | SEQ ID NO: 121 DAGYDYPTHAMHY |
| 305LO22 | SEQ ID NO: 109 | SEQ ID NO: 117 SSYYMA | SEQ ID NO: 118 AIFTGSGAEYKAEWAKG | SEQ ID NO: 121 DAGYDYPTHAMHY |
| 305LO23 | SEQ ID NO: 110 | SEQ ID NO: 117 SSYYMA | SEQ ID NO: 120 GIFTGSGATYIKAEWAKG | SEQ ID NO: 121 DAGYDYPTHAMHY |

**[Table 8]**

| **VL amino acid sequences of the 305 variants** | | | | |
|---|---|---|---|---|
| Antibody | VL | HVR-L1 | HVR-L2 | HVR-L3 |
| 30SLO5 | SEQ ID NO: 20 | SEQ ID NO: 84 QASQNIGSSLA | SEQ ID NO: 94 GASKTHS | SEQ ID NO: 104 QSTKVGSSYGNH |
| 305LO15 | SEQ ID NO: 111 | SEQ ID NO: 122 RASQGISSSLA | SEQ ID NO: 123 GASETES | SEQ ID NO: 125 QNTKVGSSYGNT |
| 305LO16 | SEQ ID NO: 111 | SEQ ID NO: 122 RASQGISSSLA | SEQ ID NO: 123 GASETES | SEQ ID NO: 125 QNTKVGSSYGNT |
| 305LO18 | SEQ ID NO: 111 | SEQ ID NO: 122 RASQGISSSLA | SEQ ID NO: 123 GASETES | SEQ ID NO: 125 QNTKVGSSYGNT |
| 305LO19 | SEQ ID NO: 111 | SEQ ID NO: 122 RASQGISSSLA | SEQ ID NO: 123 GASETES | SEQ ID NO: 125 QNTKVGSSYGNT |
| 305L020 | SEQ ID NO: 112 | SEQ ID NO: 122 RASQGISSSLA | SEQ ID NO: 123 GASETES | SEQ ID NO: 125 QNTKVGSSYGNT |
| 305LO22 | SEQ ID NO: 113 | SEQ ID NO: 122 RASQGISSSLA | SEQ ID NO: 124 GASTTQS | SEQ ID NO: 125 QNTKVGSSYGNT |
| 305L023 | SEQ ID NO: 113 | SEQ ID NO: 122 RASQGISSSLA | SEQ ID NO: 124 GASTTQS | SEQ ID NO: 125 QNTKVGSSYGNT |

### [EXAMPLE 8]

### Binding characterization of anti-C5 antibodies (305 variants)

The kinetic parameters of anti-C5 antibodies against recombinant human C5 were assessed at 37 degrees C using BIACORE (registered trademark) T200 instrument (GE Healthcare) at three different conditions; (1) both association and dissociation were at pH7.4, (2) both association and dissociation were at pH5.8, and (3) association was at pH7.4 but dissociation was at pH5.8. ProA/G (Pierce) was immobilized onto a CM1 sensorchip using amine coupling kit (GE Healthcare) according to the recommended settings by GE Healthcare. Antibodies and analytes for condition (1) and (3) were diluted in ACES pH7.4 buffer (20 mM ACES, 150 mM NaCl, 1.2 mM CaCl₂, 0.05% Tween 20, 0.005% NaN₃) and for condition (2) they were diluted in ACES pH5.8 buffer (20 mM ACES, 150 mM NaCl, 1.2 mM CaCl₂, 0.05% Tween 20, 0.005% NaN₃). Each antibody was captured onto the sensor surface by ProA/G. Antibody capture levels were typically 60-90 resonance units (RU). Then, recombinant human C5 was injected at 3 to 27 nM or 13.3 to 120 nM prepared by three-fold serial dilution, followed by dissociation. The surface was regenerated using 25 mM NaOH. Kinetic parameters at condition (1) and (2) were determined by fitting the sensorgrams with a 1:1 binding model and the dissociation rate at condition (3) was determined by fitting the sensorgrams with a 1:1 dissociation for MCK model using BIACORE (registered trademark) T200 Evaluation software, version 2.0 (GE Healthcare). pH dependency of all antibodies were shown as ratio of dissociation rate of condition (2) and (1).

Association rate (ka), dissociation rate (kd), binding affinity (KD), and pH dependency are listed in Table 9. All antibodies showed a faster dissociation rate at pH 5.8 than pH7.4 and their pH dependency was around 20 fold.

**[Table 9]**

| **Kinetics parameters of anti-C5 antibody variants under pH7.4 and pH5.8 conditions** | | | | | | | | |
|---|---|---|---|---|---|---|---|---|
| | 7.4_7.4 | | | 5.8_5.8 | | | 7.4_5.8 kd (1/s) | pH dependency |
| | ka (1/Ms) | kd (1/s) | KD (M) | ka (1/Ms) | kd (1/s) | KD (M) | | |
| LO15-SG422 | 1.40E+06 | 4.19E-04 | 3.00E-10 | 1.34E+05 | 8.79E-03 | 6.57E-08 | 1.61E-02 | 21.0 |
| LO15-SG115 | 1.31E+06 | 3.54E-04 | 2.70E-10 | 9.49E+04 | 8.27E-03 | 8.72E-08 | 1.67E-02 | 23.4 |
| LO16-SG422 | 1.28E+06 | 4.12E-04 | 3.21E-10 | 1.09E+05 | 8.69E-03 | 7.95E-08 | 1.61E-02 | 21.1 |
| LO18-SG422 | 1.36E+06 | 4.26E-04 | 3.14E-10 | 1.39E+05 | 8.65E-03 | 6.24E-08 | 1.69E-02 | 20.3 |
| LO19-SG422 | 1.37E+06 | 4.76E-04 | 3.46E-10 | 1.38E+05 | 8.30E-03 | 6.00E-08 | 1.61E-02 | 17.4 |
| LO20-SG115 | 1.44E+06 | 4.67E-04 | 3.24E-10 | 1.41E+05 | 8.18E-03 | 5.81E-08 | 1.61E-02 | 17.5 |
| LO20-SG422 | 1.35E+06 | 4.70E-04 | 3.49E-10 | 1.36E+05 | 8.15E-03 | 5.99E-08 | 1.55E-02 | 17.3 |
| LO22-SG115 | 1.46E+06 | 3.82E-04 | 2.62E-10 | 1.71E+05 | 9.30E-03 | 5.45E-08 | 1.46E-02 | 24.3 |
| LO23-SG115 | 1.53E+06 | 4.23E-04 | 2.77E-10 | 1.33E+05 | 8.55E-03 | 6.41E-08 | 1.73E-02 | 20.2 |

The binding affinity of anti-C5 antibodies (BNJ441, eculizumab, and a 305 variant) to recombinant human C5 at pH7.4 and pH5.8 were determined at 37 degrees C using a BIACORE (registered trademark) T200 instrument (GE Healthcare) to assess the effect of pH upon antigen binding. Goat anti-human IgG (Fc) polyclonal antibody (KPL #01-10-20) was immobilized onto a CM4 sensorchip using an amine coupling kit (GE Healthcare) according to the recommended settings by manufacturer. Antibodies and analytes were diluted either in ACES pH7.4 buffer or ACES pH5.8 buffer containing 20 mM ACES, 150 mM NaCl, 1.2 mM CaCl₂, 0.05% Tween 20, and 0.005% NaN₃. Antibodies were captured on the sensor surface using the anti-Fc method, capture levels were typically 50-80 resonance units (RU). Recombinant human C5 was prepared by three-fold serial dilution starting from 27 nM for pH7.4 assay conditions, or 135 nM for pH 5.8 assay conditions. The surface was regenerated using 20 mM HCl, 0.01% Tween 20. The data were processed and fit with a 1:1 binding model using BiaEvaluation 2.0 software (GE Healthcare).

The binding affinity (KD) of BNJ441, eculizumab, and the 305 variant to recombinant human C5 at pH7.4 and pH5.8 is shown in Table 10. The 305 variant showed a ratio of (KD at pH 5.8)/(KD at pH 7.4) of almost 800, 8 fold higher than BNJ441 which only showed a ratio of (KD at pH 5.8)/(KD at pH 7.4) of 93.

**[Table 10]**

| **Antibody** | **KD (M)** | | **ratio of KD at pH 5.8/pH 7.4** |
|---|---|---|---|
| | **pH 7.4** | **pH 5.8** | |
| 305LO5 variant | 1.66E-10 | 1.32E-07 | 795 |
| Eculizumab | 1.42E-10 | 2.64E-09 | 19 |
| BNJ441 | 1.38E-09 | 1.28E-07 | 93 |

### [EXAMPLE 9]

### Inhibitory activity of anti-C5 antibodies (305 variants) on C5 activation

9.1. Inhibition of complement-activated liposome lysis by anti-C5 MAbs The anti-C5 MAbs were tested for inhibition of complement activity by a liposome lysis assay. Thirty microliters of normal human serum (6.7%) (Biopredic, SER019) was mixed with 20 micro L of diluted MAb in a 96-well plate and incubated on a shaker for 30 min at room temperature. Liposome solution sensitized with antibodies against dinitrophenyl (Autokit CH50, Wako, 995-40801) was transferred into each well and placed on a shaker for 2 min at 37 degrees C. Fifty microliters of substrate solution (Autokit CH50) was added to each well and mixed by shaking for 2 min at 37 degrees C. The final mixture was incubated at 37 degrees C for 40 minutes, and thereafter OD at 340 nm was measured. The percent of liposome lysis was defined as 100 × [(OD _{MAb} - OD _{serum and liposome background})]/ [(OD _{without} MAb - OD _{serum and liposome background})]. Figure 15 shows that the anti-C5 Mabs: 305LO15-SG422, 305LO16-SG422, 305LO18-SG422, 305LO19-SG422, 305LO20-SG422, and 305LO20-SG115, inhibited liposome lysis. Two antibodies with Fc variants: 305LO15-SG115 and 305LO23-SG429, also inhibited lipisome lysis (Figure 16).

The anti-C5 MAbs were tested for inhibition of recombinant human C5 (SEQ ID NO: 39). Ten microliters of CS-deficient human serum (Sigma, C1163) was mixed with 20 micro L of diluted MAb and 20 micro L of recombinant C5 (0.1 micro g/mL) in a 96-well plate and incubated on a shaker for 1 hour at 37 degrees C. Liposomes (Autokit CH50) were transferred into each well and placed on a shaker for 2 min at 37 degrees C. Fifty microliters of substrate solution (Autokit CH50) was added to each well and mixed by shaking for 2 min at 37 degrees C. The final mixture was incubated at 37 degrees C for 180 minutes, and thereafter OD at 340 nm was measured. The percent of liposome lysis was defined as above. Figure 17 shows that anti-C5 Mabs: 305LO22-SG115, 305LO22-SG422, 305LO23-SG115, and 305LO23-SG422, inhibited liposome lysis.

### 9.2. Inhibition of C5a generation by anti-C5 MAbs

Anti-C5 MAbs were tested for C5a generation during liposome lysis to confirm that anti-C5 MAbs inhibit cleavage of C5 into C5a and C5b. C5a levels in the supernatants from a liposome lysis assay were quantified using a C5a ELISA kit (R&D systems, DY2037). All MAbs inhibited C5a generation in the supernatants in a dose-dependent manner (Figures 18 and 19).

### 9.3. Measurement of complement activity in cynomolgus monkey plasma

Anti-C5 MAbs were tested for inhibition of complement activity in cynomolgus monkey plasma. The anti-C5 Mabs were intra administered to the monkeys (20mg/kg), and plasma samples were collected periodically until day 56. Chicken red blood cells (cRBCs) (Innovative research, IC05-0810) were washed with gelatin/veronal-buffered saline containing 0.5 mM MgCl₂ and 0.15 mM CaCl₂ (GVB++) (Boston BioProducts, IBB-300X), and thereafter sensitized with anti-chicken RBC antibody (Rockland 103-4139) at 1 micro g/ml for 15 minutes at 4 degrees C. The cells were then washed with GVB++ and suspended in the same buffer at 1×10⁸ cells/ml. In a separate round-bottom 96-well microtest plate, monkey plasma was incubated with the sensitized cRBCs at 37 degrees C for 20 minutes. After the incubation, the plate was centrifuged at 1000 × g for 2 minutes at 4 degrees C. Supernatants were transferred to wells on a flat-bottom 96-well microtest plates for measurement of OD at 415 nm with a reference wavelength at 630 nm. The percent of hemolysis was defined as 100 × [(OD _{Post administration} - OD _{plasma and cRBCs background})]/ [(OD _{Pre administration} - OD _{plasma and cRBCs background})]. Figure 20 shows that anti-C5 MAbs: 305LO15-SG422, 305LO15-SG115, 305LO16-SG422, 305LO18-SG422, 305LO19-SG422, 305LO20-SG422, 305LO20-SG115, and 305LO23-SG115, inhibited the complement activity in the plasma.

### 9.4. Inhibition of biological activity of C5 variants by anti-C5 MAbs

Anti-CS MAbs were tested for the inhibition of recombinant human C5 variants: V145I, R449G, V802I, R885H, R928Q, D966Y, S1310N, and E1437D. It has been reported that PNH patients who have a R885H mutation in C5 show poor response to eculizumab (see, e.g., Nishimura et al., New Engl. J. Med. 370:632-639 (2014)). Each of the human C5 variants was expressed in FS293 cells, and the supernatants were used for the following study. Ten microliters of CS-deficient human serum (Sigma, C1163) was mixed with 20 micro L of diluted MAb and 20 micro L of cell culture media containing a recombinant C5 variant (2-3 micro g/mL) in a 96-well plate and incubated on a shaker for 0.5 hour at 37 degrees C. Liposomes (Autokit CH50) were transferred into each well and placed on a shaker for 2 min at 37 degrees C. Fifty microliters of substrate solution (Autokit CH50) was added to each well and mixed by shaking for 2 min at 37 degrees C. The final mixture was incubated at 37 degrees C for 90 minutes, and thereafter OD at 340 nm was measured. The percent of liposome lysis was defined as above. Figure 21 shows that an anti-C5 MAb (eculizumab) did not inhibit the R885H C5 variant, but inhibited the other variants tested. Figure 22 shows that the anti-C5 MAb (a 305 variant) inhibited all variants of C5 tested.

### 9.5. Inhibition of complement-activated liposome lysis by anti-C5 MAbs

Anti-C5 MAbs were tested for inhibition of complement activity by a liposome lysis assay. Thirty microliters of normal human serum (6.7%) (Biopredic, SER019) was mixed with 20 micro L of diluted MAb in a 96-well plate and incubated on a shaker for 30 min at room temperature. Liposome solution sensitized with antibodies against dinitrophenyl (Autokit CH50, Wako, 995-40801) was transferred into each well and placed on a shaker for 2 min at 25 degrees C. Fifty microliters of substrate solution (Autokit CH50) was added to each well and mixed by shaking for 2 min at 25 degrees C. The final mixture was incubated at 37 degrees C for 45 minutes, and thereafter OD at 340 nm was measured. The percent inhibition of liposome lysis was defined as 100 × [(OD _{MAb} - OD _{serum and liposome background})]/ [(OD _{without MAb} - OD _{serum and liposome background})]. Figure 23 shows that the anti-C5 MAbs, BNJ441 and the 305 variant inhibited liposome lysis, and that the 305 variant has stronger inhibitory activity than BNJ441.

### [EXAMPLE 10]

### Pharmacokinetic study of anti-C5 monoclonal antibodies (305 variants) in cynomolgus monkey

### 10.1. In vivo test using cynomolgus monkey

The in vivo kinetics of anti-human C5 antibody was assessed after administering anti-human C5 antibody in cynomolgus monkey (Shin Nippon Biomedical Laboratories, Ltd., Japan). A solution of anti-human C5 antibody (2.5 mg/ml) was administered once at a dose of approximately 8 ml/kg into the cephalic vein of the forearm by 30 minutes infusion. Blood was collected at pre-administration and 5 minutes, seven hours, one day, two days, three days, seven days, fourteen days, twenty one days, twenty eight days, thirty five days, forty two days, forty nine days, and fifty six days after administration. The collected blood was immediately centrifuged at 1,700 × g and 4 degrees C for 10 minutes to separate the plasma. The separated plasma was stored in a refrigerator at -70 degrees C or below before assay. The anti-human C5 antibodies were prepared as described in Example 7.

### 10.2. Measurement of total cynomolgus monkey C5 plasma concentration by ELISA assay

The concentration of total cynomolgus monkey C5 in cynomolgus monkey plasma was measured by ELISA. Anti-human C5 antibody (in-house antibody generated using the method described in Example 2) was dispensed onto Nunc-ImmunoPlate MaxiSorp (Nalge Nunc International) and allowed to stand overnight at 4 degrees C to prepare anti-cynomolgus monkey C5-immobilized plates. Calibration curve samples and cynomolgus monkey plasma samples diluted 20000-fold with 0.4 micro g/ml injected antibody were prepared and incubated for 60 minutes at 37 degrees C. Subsequently, the samples were dispensed onto the anti-cynomolgus monkey C5-immobilized plates, and allowed to stand for one hour at room temperature. Then, HRP-labelled anti-human IgG Antibody (SouthernBiotech) was added to react for thirty minutes at room temperature, and washing was performed. Subsequently, ABTS ELISA HRP Substrate (KPL) was added. The signal was measured by a plate reader at a wavelength of 405 nm. The cynomolgus monkey C5 concentration was calculated based on the response of the calibration curve using the analytical software SOFTmax PRO (Molecular Devices). The time course of plasma cynomolgus monkey C5 concentration after intravenous administration as measured by this method is shown in Figure 24. Data are plotted as the percentage remaining compared to plasma cynomolgus monkey C5 concentration at pre-administration. The pH dependent anti-human C5 antibodies (305LO15-SG422, 305LO15-SG115, 305LO16-SG422, 305LO18-SG422, 305LO19-SG422, 305LO20-SG422, 305LO20-SG115, 305LO22-SG422, 305LO23-SG422, and 305LO23-SG115) showed lower accumulation of plasma C5 compared to non pH dependent anti-human C5 antibodies.

### 10.3. Measurement of anti-human C5 antibody plasma concentration by ELISA assay

The concentration of anti-human C5 antibody in cynomolgus monkey plasma was measured by ELISA. Anti-human IgG kappa chain antibody (Antibody Solutions) was dispensed onto Nunc-ImmunoPlate MaxiSorp (Nalge Nunc International) and allowed to stand overnight at 4 degrees C to prepare anti-human IgG-immobilized plates. Calibration curve samples and cynomolgus monkey plasma samples diluted 100-fold or more were prepared. Subsequently, the samples were dispensed onto the anti-human IgG-immobilized plates, and allowed to stand for one hour at room temperature. Then, HRP-labelled anti-human IgG antibody (SouthernBiotech) was added to react for thirty minutes at room temperature, and washing was performed. Subsequently, ABTS ELISA HRP Substrate (KPL) was added. The signal was measured by a plate reader at a wavelength of 405 nm. The anti-human C5 antibody concentration was calculated based on the response of the calibration curve using the analytical software SOFTmax PRO (Molecular Devices). The time course of plasma anti-human C5 antibody concentration after intravenous administration as measured by this method is shown in Figure 25. The pH dependent anti-human C5 antibodies (305LO15-SG422, 305LO15-SG115, 305LO16-SG422, 305LO18-SG422, 305LO19-SG422, 305LO20-SG422, 305LO20-SG115, 305LO22-SG422, 305LO23-SG422, and 305LO23-SG115) displayed longer half-life compared to non pH dependent anti-human C5 antibodies.

### [EXAMPLE 11]

X-ray crystal structure analysis of a 305 variant Fab and human C5-MG1 domain complex

### 11.1. Expression and purification of the MG1 domain (20-124) of human C5

The MG1 domain (amino acid residues 20-124 of SEQ ID NO:39) fused to a GST-tag via thrombin cleavable linker (GST-MG1) was expressed in the E. coli strain BL21 DE3 pLysS (Promega) using a pGEX-4T-1 vector (GE healthcare). Protein expression was induced with 0.1 mM Isopropyl beta-D-1-thiogalactopyranoside (IPTG) at 25 degrees C for 5 hours. The bacterial cell pellet was lysed with Bugbuster (Merck) supplemented with lysonase (Merck) and complete protease inhibitor cocktail (Roche), followed by the purification of GST-MG1 from the soluble fraction using a GSTrap column (GE healthcare) according to the manufacturer's instruction. The GST tag was cleaved with thrombin (Sigma), and the resulting MG1 domain was further purified with a Superdex 75 gel filtration column (GE healthcare). Fractions containing MG1 domain were pooled and stored at -80 degrees C.

### 11.2. Preparation of Fab fragment of a 305 variant

Fab fragments of one of the optimized variants from 305 were prepared by the conventional method using limited digestion with papain (Roche Diagnostics, Cat No.1047825), followed by loading onto a protein A column (MabSlect SuRe, GE Healthcare) to remove Fc fragments, a cation exchange column (HiTrap SP HP, GE Healthcare), and a gel filtration column (Superdex200 16/60, GE Healthcare). Fractions containing Fab fragment were pooled and stored at -80 degrees C.

### 11.3. Preparation of a 305 variant Fab and human C5-MG1 domain complex

Purified recombinant human C5-MG1 domain was mixed with a purified 305 variant Fab fragment in a 1:1 molar ratio. The complex was purified by gel filtration chromatography (Superdex200 10/300 increase, GE Healthcare) using a column equilibrated with 25 mM HEPES pH 7.5, 100 mM NaCl.

### 11.4. Crystallization

The purified complexes were concentrated to about 10 mg/mL, and crystallization was carried out by the sitting drop vapor diffusion method in combination with the seeding method at 4 degrees C. The reservoir solution consisted of 0.2 M magnesium formate dehydrate, 15.0% w/v polyethylene glycol 3350. This succeeded in yielding plate-like crystals in a few days. The crystal was soaked in a solution of 0.2 M magnesium formate dehydrate, 25.0% w/v polyethylene glycol 3350, and 20% glycerol.

### 11.5. Data collection and structure determination

X-ray diffraction data were measured by BL32XU at SPring-8. During the measurement, the crystal was constantly placed in a nitrogen stream at -178 degrees C to maintain a frozen state, and a total of 180 X-ray diffraction images were collected using an MX-225HS CCD detector (RAYONIX) attached to a beam line, while rotating the crystal 1.0 degree at a time. Determination of the cell parameters, indexing the diffraction spots, and processing the diffraction data obtained from the diffraction images were performed using the Xia2 program (J. Appl. Cryst. 43:186-190 (2010), XDS Package (Acta. Cryst. D66:125-132 (2010)), and Scala (Acta. Cryst. D62:72-82 (2006)), and finally the diffraction intensity data up to 2.11 Angstrom(s) resolution was obtained. The crystallography data statistics are shown in Table 11.

**[Table 11]**

| | **X-ray data collection and refinement statistics** | |
|---|---|---|
| **Data collection** | | |
| Space group | | *P1* |
| Unit Cell | | |
| | *a,b,c* (Å) | 39.79, 55.10, 127.76 |
| | α,β,γ (°) | 89.18, 86.24, 78.20 |
| Resolution (Å) | | 49.49-2.11 |
| Total reflections | | 112,102 |
| Unique reflections | | 56,154 |
| Completeness (highest resolution shell) (%) | | 92.1 (95.8) |
| *R*_{merge} ^{a} (highest resolution shell) (%) | | 7.2 (31.7) |

| **Refinement** | | |
|---|---|---|
| Resolution (A) | | 25.00-2.11 |
| Reflections | | 53,398 |
| *R* factor ^{b} (*R*_{free} ^{c}) (%) | | 20.42 (26.44) |
| rms deviation from ideal | | |
| | Bond lengths (Å) | 0.0088 |
| | Bond angles (°) | 1.3441 |

| | | |
|---|---|---|
| a; *R*_{merge} = *ΣhklΣj*\|*Ij* (*hkl*)- 〈*I*(*hkl*)〉 \|/Σ*hkl*Σ*j*\|*Ij* (*hkl*)\|, where *Ij* (*hkl*) and 〈*I*(*hkl*)〉 are the intensity of measurement j and the mean intensity for the reflection with indices *hkl,* respectively. b; *R* factor = Σ*hkl*\|*F*_{calc}(*hkl*)\| - \|*F*_{obs} (*hkl*)\|/Σ*hkl*\|*F*_{obs} (*hkl*)\|, where *F*_{obs} and *F*_{calc} are the observed and calculated structure factor amplitudes, respectively. c; *R*_{free} is calculated with 5% of the reflection randomly set aside. | | |

The structure was determined by molecular replacement with the program Phaser (J. Appl. Cryst. 40:658-674 (2007)). The search model of the Fab domain was derived from the published human IgG4 Fab crystal structure (PDB code:1BBJ), and the search model of the MG1 domain was from the published human C5 crystal structure (PDB code: 3CU7, Nat.Immunol. 9:753-760 (2008)). A model was built with the Coot program (Acta Cryst. D66:486-501 (2010)) and refined with the program Refmac5 (Acta Cryst. D67:355-367 (2011)). The crystallographic reliability factor (R) for the diffraction intensity data from 25-2.11 Angstrom(s) was 20.42%, with a Free R value of 26.44%. The structure refinement statistics are shown in Table 11.

### 11.6. Overall structure of a 305 variant Fab and C5-MG1 domain complex

The Fab fragment of an optimized variant from 305 ("305 Fab") bound to the human C5-MG1 domain ("MG1 ") in a 1:1 ratio, and the asymmetric unit of the crystal structure contained two complexes, Molecules 1 and 2, as depicted in Figure 26A. Molecules 1 and 2 can be aligned well at 0.717 Angstrom(s) RMSD with the C alpha atom position in all the residues, as shown in Figure 26B. The figures discussed below were prepared using Molecule 1.

In Figures 27A and 27B, the epitope of the 305 Fab contact region is mapped in the MG1 amino acid sequence and in the crystal structure, respectively. The epitope includes the amino acid residues of MG1 that contain one or more atoms located within 4.5 Angstrom(s) distance from any part of the 305 Fab in the crystal structure. In addition, the epitope within 3.0 Angstrom(s) is highlighted in Figure 27A.

### 11.7. Interactions of E48, D51, and K109

As described in Examples 4.5 and 4.6, the anti-C5 Mabs that include the 305 antibody series were tested for binding to three human C5 point mutants, E48A, D51A, and K109A, by Western blot and BIACORE (registered trademark) binding analyses. While the 305 variants bound WT C5 strongly, they bound the E48A C5 mutant only weakly and did not bind to the D51A and K109A mutants. The crystal structure of the 305 Fab and MG1 complex revealed that the three amino acids E48, D51, and K109, are all within 3.0 Angstrom(s) distance from the 305 Fab, forming a number of hydrogen bonds with the Fab, as shown in Figure 28A. On more detailed examination, the K109 residue of MG1 is buried in a groove formed at the interface of the heavy chain of the Fab and tightly interacts with the Fab by three hydrogen bonds with H-CDR3_G97, H-CDR3_Y100, and H-CDR3_T100b, and by a salt bridge with H-CDR3 _D95 (Figure 28D). D51 is located between MG1 and the heavy chain of the 305 Fab and makes two hydrogen bonds with H-CDR1_Ser32 and H-CDR2_Ser54 to fill the space (Figure 28C). These indicate that K109 and D51 of C5 are both critical residues for binding of the 305 antibody series. On the other hand, E48 is located closer to the surface and forms only one hydrogen bond with the Fab, suggesting that its contribution to the antibody binding would be less than those of K109 and E51 (Figure 28B). These relationships are consistent with the results of the Western blot and BIACORE (registered trademark) binding analyses of human C5 mutants (Examples 4.5 and 4.6). Supplementary note: residue numbering for the Fab amino acids is based on the Kabat numbering scheme. (Kabat et al., Sequences of Proteins of Immunological Interest, 5th Ed. Public Health Service, National Institutes of Health, Bethesda, Md., 1991)

### 11.8. Interactions of H70, H72, and H110 of human C5 and 305 antibody series

The crystal structure analysis revealed that three histidine residues on human C5, namely, H70, H72, and H110, are included in the epitope of the 305 variant Fab, as shown in Figure 27A and Figure 29A. A BIACORE (registered trademark) binding analysis was performed to investigate the contribution of these histidine residues to the pH-dependent protein-protein interaction between human C5 and the 305 variant Fab using the human C5 mutants H70Y, H72Y, H110Y, and H70Y+H110Y (Example 4.7). H72Y resulted in the complete loss of the binding of the 305 variant Fab to C5. This residue of C5 is located in the pocket formed by the CDR2 loop of the heavy chain of the 305 Fab and the loop of MG1 (L73, S74, and E76) and fills this space tightly, as shown in Figure 29C. In addition, the H72 residue of C5 makes a hydrogen bond with H-CDR2_Y58. The H72Y mutation would not be expected to be tolerated as there is not enough space to accommodate the bulkier side chain of tyrosine. Also a hydrogen bond with H-CDR2_Y58 cannot be maintained. With regards to the contribution of H70 and H110 to pH dependency, H70Y and H110Y mutations resulted in slower dissociation of the 305 variant Fab from C5 at pH 5.8. H70 forms an intra-molecular hydrogen bond with T53 of MG1, which is believed to be disrupted at pH 5.8 when protonation of H70 of C5 causes a conformational change in the corresponding part of the interaction interface of MG1 (Figure 29B). For H110, protonation of this C5 residue would be expected to cause a charge repulsion to the 305 Fab, which may be augmented by the protonation of the neighboring histidine residue, H-CDR3 _H100c (Figure 29D).

## Claims

1. An anti-C5 antibody comprising a VH sequence of SEQ ID NO: 106 and a VL sequence of SEQ ID NO: 111.

2. The antibody of claim 1, which comprises a heavy chain constant region comprising the amino acid sequence of SEQ ID NO: 114 and a light chain constant region comprising the amino acid sequence of SEQ ID NO: 38.

3. The antibody of claim 1 or 2, which is a full length IgG1 antibody.

4. An isolated nucleic acid encoding the antibody of any one of claims 1 to 3.

5. A host cell comprising the nucleic acid of claim 4.

6. A method of producing an antibody comprising culturing the host cell of claim 5 so that the antibody is produced.

7. An anti-C5 antibody as obtainable by the method of claim 6.

8. A pharmaceutical formulation comprising the antibody of any one of claims 1 to 3 or 7 and a pharmaceutically acceptable carrier.

## Patentansprüche

1. Anti-C5-Antikörper, umfassend eine VH-Sequenz von SEQ ID NO:106 und eine VL-Sequenz von SEQ ID NO:111.

2. Antikörper nach Anspruch 1, der eine konstante Region der schweren Kette umfasst, die die Aminosäuresequenz von SEQ ID NO:114 umfasst, und eine konstante Region der leichten Kette umfasst, die die Aminosäuresequenz von SEQ ID NO:38 umfasst.

3. Antikörper nach Anspruch 1 oder 2, der ein IgG1-Antikörper voller Länge ist.

4. Isolierte Nucleinsäure, die den Antikörper nach einem der Ansprüche 1 bis 3 codiert.

5. Wirtszelle, umfassend die Nucleinsäure nach Anspruch 4.

6. Verfahren zur Herstellung eines Antikörpers, das das Züchten der Wirtszelle nach Anspruch 5 umfasst, so dass der Antikörper hergestellt wird.

7. Anti-C5-Antikörper wie mit dem Verfahren nach Anspruch 6 erhältlich.

8. Pharmazeutische Formulierung, die den Antikörper nach einem der Ansprüche 1 bis 3 oder 7 und einen pharmazeutisch verträglichen Träger umfasst.

## Revendications

1. Anticorps anti-C5 comprenant une séquence VH de SEQ ID NO : 106 et une séquence VL de SEQ ID NO : 111.

2. Anticorps selon la revendication 1, qui comprend une région constante de chaîne lourde comprenant la séquence d'acides aminés de SEQ ID NO : 114 et une région constante de chaîne légère comprenant la séquence d'acides aminés de SEQ ID NO : 38.

3. Anticorps selon la revendication 1 ou 2, qui est un anticorps IgG1 de pleine longueur.

4. Acide nucléique isolé codant l'anticorps de l'une quelconque des revendications 1 à 3.

5. Cellule hôte comprenant l'acide nucléique de la revendication 4.

6. Méthode de production d'un anticorps, comprenant la culture de la cellule hôte de la revendication 5 de façon que l'anticorps soit produit.

7. Anticorps anti-C5 susceptible d'être obtenu par la méthode de la revendication 6.

8. Formulation pharmaceutique comprenant l'anticorps de l'une quelconque des revendications 1 à 3 et 7 et un support pharmaceutiquement acceptable.
